(19) Europäisches Patentamt
European Patent Office
Office européen des brevets

(11) Publication number: **0 186 057**
**A1**

(12) # EUROPEAN PATENT APPLICATION

(21) Application number: 85115847.7

(22) Date of filing: 12.12.85

(51) Int. Cl.[4]: C 07 D 487/04
A 61 K 31/40
//C07F9/65, C07F7/18,
(C07D487/04, 209:00, 205:00)

(30) Priority: 13.12.84 US 681408

(43) Date of publication of application:
02.07.86 Bulletin 86/27

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Salzmann, Thomas N.
387 Brook Avenue
North Plainfield New Jersey 07060(US)

(72) Inventor: Christensen, Burton G.
770 Anderson Avenue Apt. 19 H
Cliffside New Jersey 07010(US)

(72) Inventor: Heck, James V.
79 Hunter Avenue
Fanwood New Jersey 07023(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **Carbapenems having a 2-position substituent joined through an alkyleneheteroatom bridge.**

(57) Carbapenems having the formula:

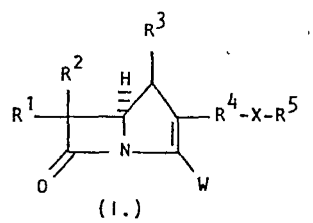

(I.)

wherein

$R^3$ is H;

$R^4$ is a bridging group comprising substituted or unsubstituted $C_2$-$C_4$ straight, $C_2$-$C_6$ branched or $C_3$-$C_7$ cycloalkyl groups wherein the substituents are selected from $C_1$-$C_6$ alkyl, O-$C_1$-$C_6$ alkyl, S-$C_1$-$C_6$ alkyl, $CF_3$, $N(C_1$-$C_6$ alkyl$)_2$;

$R^1$ and $R^2$ are independently H, $CH_3$-, $CH_3CH_2$-, $(CH_3)_2CH$-HOCH$_2$-, $CH_3CH(OH)$-, $(CH_3)_2C(OH)$-, $FCH_2$-, $F_2CH-$, $F_3C-$, $CH_3CH(F)$-, $(CH_3)_2C(F)$-, or $CH_3CF_2$-;

$R^5$ is unsubstituted or substituted aliphatic radical which can include heterocyclic and heteroarylium substituents; X is $-S-$, $-SO-$, $-SO_2-$, $-O-$, or $-NH$; and W is a carboxy-containing group; their preparation and antibiotic use are disclosed.

EP 0 186 057 A1

TITLE OF THE INVENTION

CARBAPENEMS HAVING A 2-POSITION SUBSTITUENT JOINED THROUGH AN ALKYLENEHETEROATOM BRIDGE

BACKGROUND OF THE INVENTION

The present invention is concerned with carbapenem antibiotics having a 2-position substituent joined through an alkyleneheteroatom bridge.

Thienamycin is a known carbapenem, broad spectrum antibiotic of the formula:

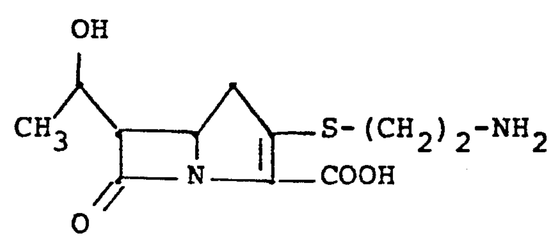

A

Other derivatives of A are also known.

The present 2-substituted carbapenems have an antibiotic spectrum comparable to A.

## BRIEF DESCRIPTION OF DISCLOSURES IN THE PRIOR ART

Sankyo U.S. Pat. No. 4,377,591 and Japanese patent publications 56-199682 and 56-60852; Shionogi Japanese patent publications 57-145086 and 57-145087; and Roche U.K. patent publication 2 092 147A, all describe azabicycloheptene antibiotics having a 2-position substituent joined through a thioalkylene bridge. U.S. Patent 4,189,493 to Bristol Myers discloses externally alkylated heteroarylium alkylthio azabicyclo heptene antibiotics. U.S. Patent 4,465,632, U.S. Patent 4,260,627 and U.S. Patent 4,267,188, all assigned to Merck & Co., Inc. disclose 2,6-substituted-1-carba-2-penem-3-carboxylic acids where the 2-substituent can be substituted or unsubstituted alkyl or aryl. However, none of these references specifically describe the carbapenem compounds of the present invention.

## SUMMARY OF THE INVENTION

Carbapenems having the formula:

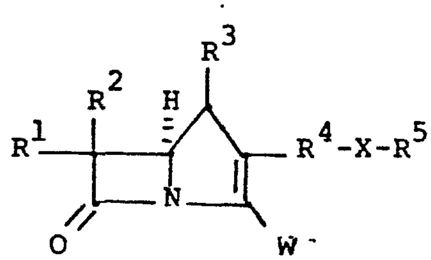

(I.)

wherein $R^3$ is H;

$R^4$ is a bridging group comprising substituted or unsubstituted $C_2$-$C_4$ straight, $C_2$-$C_6$ branched or $C_3$-$C_7$ cycloalkyl groups wherein the substituents are selected from $C_1$-$C_6$ alkyl, O-$C_1$-$C_6$ alkyl, S-$C_1$-$C_6$ alkyl, $CF_3$, N($C_1$-$C_6$ alkyl)$_2$;

$R^1$ and $R^2$ are independently H, $CH_3$-, $CH_3CH_2$-, $(CH_3)_2CH$-, $HOCH_2$-, $CH_3CH(OH)$-, $(CH_3)_2C(OH)$-, $FCH_2$-, $F_2CH$-, $F_3C$-, $CH_3CH(F)$-, $(CH_3)_2C(F)$-, or $CH_3CF_2$-;

$R^5$ is an unsubstituted or substituted aliphatic radical which can include heterocyclic and heteroarylium substituents;

X is -S-, -SO-, -$SO_2$-, -O-, or -NH; and W is a carboxy-containing group.

DETAILED DESCRIPTION OF THE INVENTION

The invention is embodied in a compound having the formula:

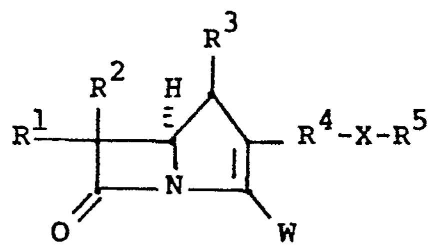

(I.)

wherein:

$R^1$ and $R^2$ are independently H, $CH_3$-, $CH_3CH_2$-, $(CH_3)_2CH$-, $HOCH_2$-, $CH_3CH(OH)$-, $(CH_3)_2C(OH)$-, $FCH_2$-, $F_2CH$-, $F_3C$-, $CH_3CH(F)$-, $(CH_3)_2C(F)$-, or $CH_3CF_2$-;

$R^3$ is H;

$R^4$ is a bridging group comprising substituted or unsubstituted $C_2$-$C_4$ straight, $C_2$-$C_6$ branched or $C_3$-$C_7$ cycloalkyl groups wherein the substituents are selected from $C_1$-$C_6$ alkyl, O-$C_1$-$C_6$ alkyl, S-$C_1$-$C_6$ alkyl, $CF_3$, N($C_1$-$C_6$ alkyl)$_2$;

X is -S-, $-\overset{O}{\overset{\|}{S}}-$, $-SO_2-$, -O-, or -NH-;

$R^5$ is selected from the group consisting of:

1. Aliphatic

$-C_1$-$C_{10}$ straight or branched alkyl; $C_3$-$C_8$ cycloalkyl; $C_2$-$C_{10}$ straight or branched alkenyl; $C_3$-$C_8$ cycloalkenyl; or $C_2$-$C_{10}$ straight or branched alkynyl;

2. Substituted Aliphatic

$-C_1$-$C_{10}$ branched or unbranched alkyl; $C_2$-$C_{10}$ branched or unbranched alkenyl, or akynyl; or $C_3$-$C_8$ cycloalkyl or cycloalkenyl; wherein all of the above groups are substituted by one or more halo, $OR_a$, $O\overset{O}{\overset{\|}{C}}R_a$, $O\overset{O}{\overset{\|}{C}}NR_aR_b$, $NHR_a$, $NR_aR_b$, $\overset{O}{\overset{\|}{C}}R_a$, $CO_2R_a$, $CONHR_a$, $CONR_aR_b$, CN, $SR_a$, $\overset{O}{S}R_a$, $SO_2R_a$, $SO_2NHR_a$, $SO_2NR_aR_b$, $NH\overset{O}{\overset{\|}{C}}R_a$, $NH\overset{O}{\overset{\|}{C}}NHR_a$, $NH\overset{O}{\overset{\|}{C}}NR_aR_b$, or $NH\overset{O}{\overset{\|}{C}}OR_a$, wherein $R_a$ and Rb are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl

moieties; phenyl; aralkyl, aralkenyl, and aralkynyl wherein the aryl moiety is phenyl and the aliphatic portion has 1-6 carbon atoms; heteroalkyl, heteroaralkyl, heterocyclyo and heterocyclyoalkyl and wherein the heteroatom or atoms in the above-named heterocyclic moieties are selected from the group consisting of 1-4 oxygen, nitrogen or sulfur atoms and wherein the alkyl moieties associated with said heterocyclic moieties have 1-6 carbon atoms;

3. Aryl
-phenyl; or phenyl substituted with 1-3 substituents selected from the group consisting of those substituents set forth above under "Substituted Aliphatic";

4. Heterocyclic
-mono- or bicyclic heterocyclic group containing from 5 to 11 total atoms of which 1 to 5 are heteroatoms selected from nitrogen, oxygen, and sulfur; or substituted heterocyclic where heterocyclic is as defined immediately above and the substituents are 1 to 3 in number and are selected from the group consisting of those substituents set forth above under "Substituted Aliphatic";

5. Arylaliphatic
-the group: -Y-Aryl, where "Aryl" is as defined above, including substituted aryl; and Y is $C_1$-$C_6$ straight or branched alkyl, $C_3$-$C_8$ cycloalkyl, or $C_2$-$C_6$ alkenyl or alkynyl;

6. <u>Heterocycloaliphatic</u>

-the group: -Y-Heterocyclic, where "Heterocyclic" is as defined above, including substituted heterocyclic; and Y is as defined above;

7. <u>Alkyl-Heteroatom-Alkyl</u>

-the group: $-(CH_2)_n-X-(CH_2)_{n'}-R_c$, where n and n' are independently 2-4; X is NR, O, or S where R is H, $CH_3$, $CH_2CH_3$, $CH_2CH_2OH$, or $CH_2CH_2NH_2$; and $R_c$ is OH, $NH_2$, $NHCH_3$, $N(CH_3)_2$, $O\underset{O}{C}CH_3$, or $NH\underset{O}{C}CH_3$; provided that the methylene carbons of the group formula may additionally be substituted with $C_1$-$C_3$ alkyl to form branched alkyl groups;

8. <u>Heteroarylium</u>

-the group: 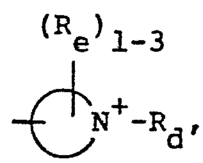, which is a mono- or bicyclic heteroarylium group containing from 5-11 ring atoms of which up to 5 are heteroatoms wherein $R_d$ is:

1) an unsubstituted or substituted $C_1$-$C_6$ alkyl radical;
2) an unsubstituted or substituted $C_2$-$C_6$ alkenyl radical;
3) an unsubstituted or substituted $C_2$-$C_6$ alkynyl radical;
4) a $C_3$-$C_7$ cycloalkyl radical in which the ring is substituted or unsubstituted and one or more atoms may be replaced by a heteroatom;

5) a $C_3$-$C_7$ cycloalkyl methyl radical in which the ring may be substituted and one or more atoms may be replaced by a heteroatom;

6) an unsubstituted or substituted $C_5$-$C_7$ cycloalkenyl radical;

7) an unsubstituted or substituted bivalent $C_2$-$C_6$ alkylidene radical, optionally interrupted by a heteroatom, and joined to the heteroarylium group to form a ring which is carbocyclic or in which one or more atoms is replaced by a heteroatom. The new ring may contain one or more double bonds;

8) an unsubstituted or substituted phenyl or heteroaryl radical;

9) an unsubstituted or substituted phenyl ($C_1$-$C_4$ alkyl) or heteroaryl ($C_1$-$C_4$ alkyl) radical;

10) a cyano ($C_1$-$C_4$ alkyl) radical;

11) a carboxy ($C_1$-$C_4$ alkyl) radical;

12) a sulfo ($C_1$-$C_4$ alkyl) radical;

13) a carbamoyl ($C_1$-$C_4$ alkyl) radical;

14) a phosphonyl ($C_1$-$C_4$ alkyl) radical;

15) a hydroxy ($C_1$-$C_4$ alkyl) radical;

16) an amino ($C_1$-$C_4$ alkyl) radical in which the nitrogen atom is unsubstituted or substituted with one to three $C_1$-$C_4$ alkyl groups;

wherein the substituents in the above definitions of $R_d$ are independently selected from the group consisting of the definitions of $R_e$ set out below;

$R_e$ is independently selected from:

a) a trifluoromethyl group;

b) a halogen atom;

c) an unsubstituted or substituted $C_1$-$C_4$ alkoxyl radical;

d) a hydroxy group;

e) an unsubstituted or substituted ($C_1$-$C_6$ alkyl) carbonyloxy radical;

f) a carbamoyloxy radical which is unsubstituted or substituted on nitrogen with one or two $C_1$-$C_4$ alkyl groups;

g) a $C_1$-$C_6$ alkylthio radical, $C_1$-$C_6$ alkylsulfinyl radical or $C_1$-$C_6$ alkylsulfonyl radical, each of which is unsubstituted or substituted on the alkyl group;

h) a sulfo group;

i) a sulfamoyl group which is unsubstituted or substituted on nitrogen by one or two $C_1$-$C_4$ alkyl groups;

j) an amino group;

k) a mono ($C_1$-$C_4$ alkyl) amino or di($C_1$-$C_4$ alkyl)amino group, each of which is unsubstituted or substituted on the alkyl group;

l) a formylamino group;

m) an unsubstituted or substituted ($C_1$-$C_6$ alkyl)carbonylamino radical;

n) a ($C_1$-$C_4$ alkoxyl) carbonylamino radical;

o) a ureido group in which the terminal nitrogen is unsubstituted or substituted with one or two $C_1$-$C_4$ alkyl groups;

p) an arylsulfonamido or a ($C_1$-$C_6$ alkyl)sulfonamido group;

q) a cyano group;

r) a formyl or acetalized formyl radical;

s) an unsubstituted or substituted ($C_1$-$C_6$ alkyl)carbonyl radical wherein the carbonyl is free or acetalized;

t) an unsubstituted or substituted phenylcarbonyl or heteroarylcarbonyl radical;

u) a hydroximinomethyl radical in which the oxygen or carbon atom is optionally substituted by a $C_1$-$C_4$ alkyl group;

v) a carboxyl group;

w) a ($C_1$-$C_6$ alkoxy)carbonyl radical;

x) a carbamoyl radical which is unsubstituted or substituted on nitrogen by one or two $C_1$-$C_4$ alkyl groups;

y) an N-hydroxycarbamoyl or N($C_1$-$C_4$ alkoxy)carbamoyl radical in which the nitrogen atom may be additionally substituted by a $C_1$-$C_4$ alkyl group;

z) a thiocarbamoyl group;

aa) a 5-(1H)-tetrazolyl group;

ab) an amidino group 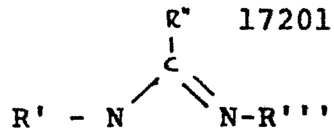

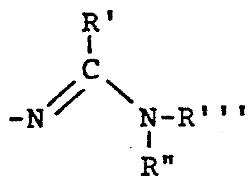

where R', R" and R''' are independently hydrogen, $C_1$-$C_4$alkyl or wherein two of the alkyl groups together form a $C_2$-$C_6$alkylidene radical optionally interrupted by a heteroatom and joined together to form a ring;

ac) a carboxamidino group 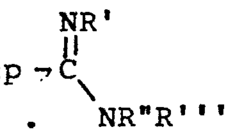 , where R', R" and R''' are as defined above;

ad) a guanidinyl group where R" in ab) above is $NR^{iv}R^{v}$ and $R^{iv}$ and $R^{v}$ are as defined for R' through R''' above;

ae) a phosphonate group $-P(O)(O^-)OR^{vi}$ where $R^{vi}$ is $C_1$-$C_3$alkyl;

af) an alkyl phosphonate group $-(CH_2)_nP(O)(O^-)(OR^{vi})$ where n = 1 to 3 and $R^{vi}$ is $C_1$-$C_3$alkyl;

ag) hydrogen;

ah) an unsubstituted or substituted $C_1$-$C_6$ alkyl radical;

ai) an unsubstituted or substituted $C_2$-$C_6$ alkenyl radical;

aj) an unsubstituted or substituted $C_2$-$C_6$ alkynyl radical;

ak) a $C_3$-$C_7$ cycloalkyl radical in which the ring is substituted or unsubstituted and one or more atoms may be replaced by a heteroatom;

al) a $C_3$-$C_7$ cycloalkyl methyl radical in which the ring may be substituted and one or more atoms may be replaced by a heteroatom;

am) an unsubstituted or substituted $C_5$-$C_7$ cycloalkenyl radical;

an) an unsubstituted or substituted phenyl or heteroaryl radical;

ao) an unsubstituted or substituted phenyl ($C_1$-$C_4$ alkyl) or heteroaryl ($C_1$-$C_4$ alkyl) radical; and wherein a preferred group is

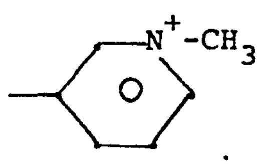 ;

9. Heteroaryliumaliphatic

-the group: -Y-Heteroarylium, where Y is as defined above; and "Heteroarylium" is as defined above and additionally includes the group:

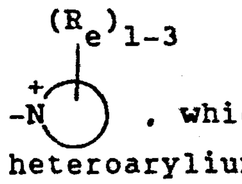

, which is a mono- or bicyclic heteroarylium group containing from 5-11 ring atoms of which up to 5 are heteroatoms, where

$R_e$ is as defined above; preferred are the monocyclic heteroarylium rings and particularly preferred is pyridinium, i.e.,

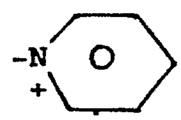 ;

10. Amidino and Amidinium

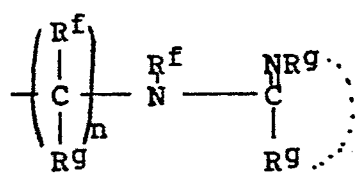

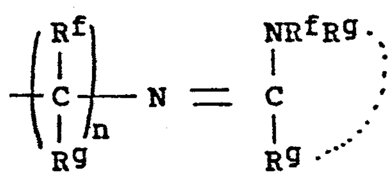

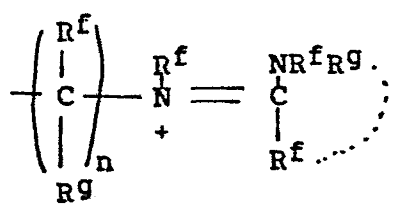

and wherein n = 2-6; $R^f$ and $R^g$ are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heterocyclic groups as defined above, including heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms; and the dotted line indicates provision for the ring formed by the joinder of substituents carried by the imino carbon atoms;

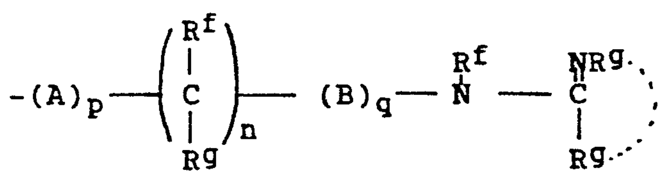

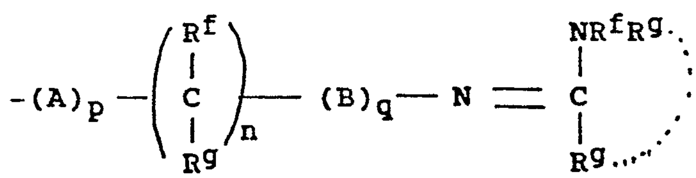

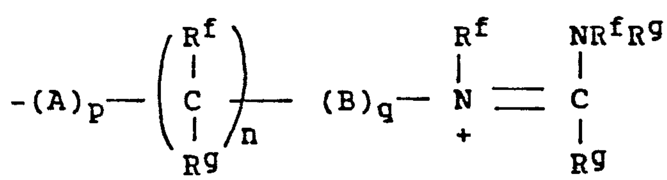

wherein $R^f$, $R^g$ and n are as defined immediately above; p and q are 0 or 1; A and B are in bivalent form and are selected from the following definitions above of $R^5$: cycloalkyl, alkenyl, cycloalkenyl, alkynyl; substituted: cycloalkyl, alkenyl, cycloalkenyl, alkynyl; phenyl and substituted phenyl; substituted and unsubstituted heteroaryl; arylaliphatic; heteroarylaliphatic, heterocycloaliphatic, and heterocyclic; and alkyl-heteroatom-alkyl; and B can also be selected from -O- and $-NR^f-$;

11. Guanidino and Guanidinium

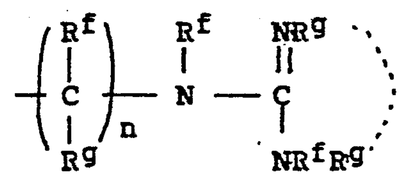

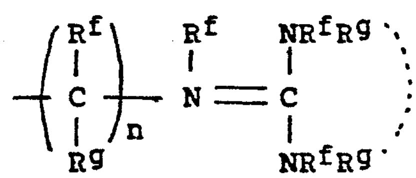

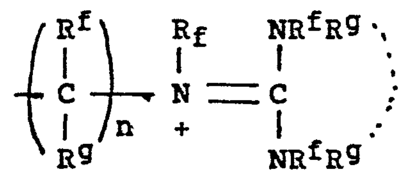

and wherein n = 2-6; $R^f$ and $R^g$ are as initially defined above under "Amidino and Amidinium"; and the dotted line indicates provision for the ring formed by the joinder of substituents carried by the imino carbon atoms;

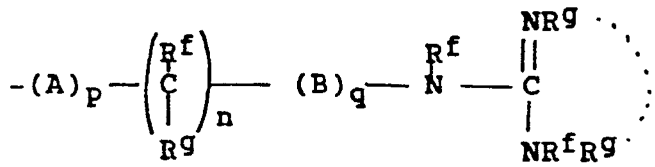

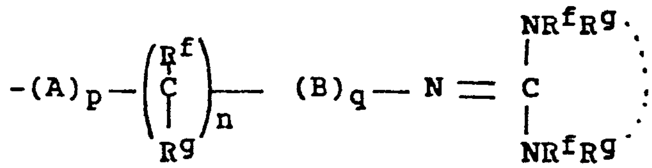

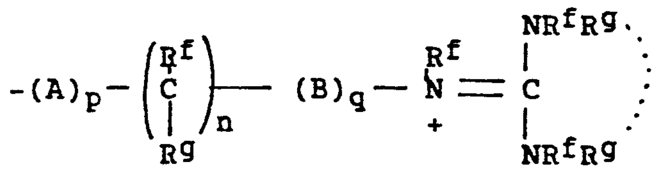

wherein $R^f$ and $R^g$ and n are as defined immediately above; p and q are 0 or 1; A and B are as defined above under "Amidino and Amidinium";

12. <u>Carbamimidoyl and Carbamimidinium</u>

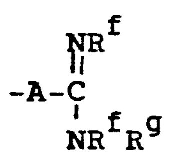

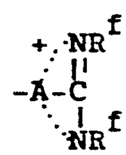

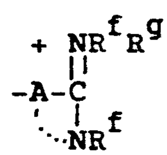

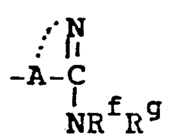

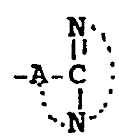

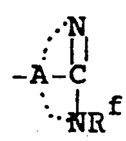

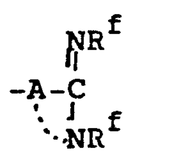

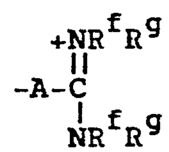

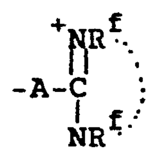

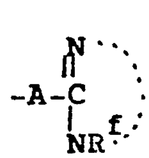

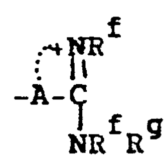

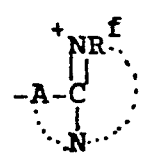

and wherein $R^f$ and $R^g$ are as defined above under "Amidino and Amidinium"; the two nitrogen atoms demonstrated in the above structure may participate in cyclic structures which are indicated by the dotted lines; and A is a connecting group between the sulfur atom and carbamimidoyl function which is bivalent, cyclic or acyclic, is to be read both left to right and right to left, and is selected from: substituted and unsubstituted: loweralkyl having from 1-6 carbon atoms; cycloalkyl having from 3-10 atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; loweralkenyl having from 2-10 carbon atoms, cycloalkenyl having from 3 to 10 carbon atoms, cycloalkenylalkyl wherein the cycloalkenyl moiety comprises 3 to 10 carbon atoms, and the alkyl moiety comprises 1 to 6 carbon atoms; alkynyl having from 2 to 10 carbon atoms; aryl, which is defined as phenyl and naphthyl; arylalkyl and alkylaryl; heteroalkyl, alkylheteroalkyl, arylheteroalkyl and alkylheteroaryl wherein the heteroatoms are selected from the group of sulfur, oxygen and nitrogen, and the alkyl moiety has 1 to 6 carbon atoms, and the aryl moiety is phenyl; heterocyclyo comprising mono- and bicyclic structures having 5 to 10 ring atoms wherein one or more of the heteroatoms is selected from oxygen, nitrogen, or sulphur; heterocycloalkyl wherein the heterocyclyo moiety comprises from 3 to 10 atoms and the alkyl moiety comprises from 1 to 6 atoms; the substituent (or substituents) relative to the

above-named radicals are selected from the group consisting of amino, hydroxyl, cyano, carboxyl, nitro, chloro, bromo, fluoro, alkoxy having from 1 to 6 carbon atoms, mercapto, haloloweralkyl, and alkylthio having from 1-6 carbon atoms;

W is selected from:
i) COOH or a pharmaceutically acceptable ester or salt thereof,
ii) COOR wherein R is a removable carboxy protecting group,
iii) COOM wherein M is an alkali metal, or
iv) $COO^-$; provided that when W is other than iv) a counterion $Z^-$ is provided.

A preferred group of compounds of the present invention has the configurational structure below:

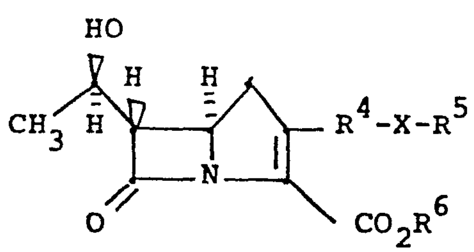

where $R^4$, $R^5$, $R^6$ are as defined above.

As used herein, the term "heteroatom" means nitrogen, oxygen, or sulfur, independently selected where more than one heteroatom is involved.

Representative $R^4$ groups are $-CH(CH_3)-$, $-CH(C_2H_5)-$, $-(CH_2)_{2-4}$, $-CH(CH_3)-CH_2-$, $CH_2-CH(OCH_3)-$, $-CH(CH_3)-(CH_2)_2-$, $-CH(CH_2OH)-CH_2-$, $-CH(CF_3)-CH_2-$, and the like.

A preferred $R^4$ group is $-CH(CH_3)-$, $-(CH_2)_2-$.

A preferred $R^3$ group is H.

Representative $R^5$ groups are as follows:

1. <u>Aliphatic</u>

$CH_3$

$CH_2CH_3$

$CH_2CH_2CH_3$

$CH(CH_3)_2$

$(CH_2)_3CH_3$

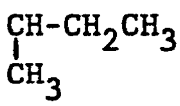

$CH_2CH(CH_3)_2$

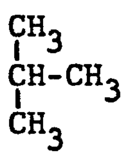

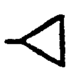

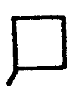

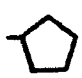

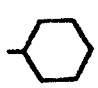

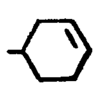

$-CH_2-$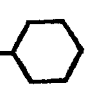

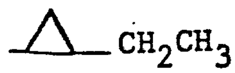

$-CH_2-CH=CH_2$

$-CH_2-CH=C(CH_3)_2$

$-CH_2-C\equiv CH$

$-CH_2-C\equiv C-CH_3$

2. <u>Substituted Aliphatic</u>

$(CH_2)_nOR^k$ $n = 2-4$, $R^k = H$, $\overset{O}{\overset{\|}{C}}CH_3$, $CH_3$

$(CH_2)_n\overset{O}{\overset{\|}{C}}XR^1$ $n=1-3$, $X=O$, NH, $NR^1$; $R^1=H$, $CH_3$

$(CH_2)_nNH_2$ $n=2-4$

$(CH_2)_nNHR^m$ $n=2-4$, $R^m=CH_3$, $CH_2CH_3$, $CH_2CH_2CH_3$, $\overset{O}{\overset{\|}{C}}CH_3$

$(CH_2)_nNR^nR^o$ $n=2-4$, $R^n/R^o=CH_3$, $CH_2CH_3$

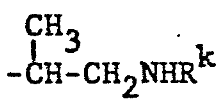 $R^k=H$, $CH_3$, $\overset{O}{\overset{\|}{C}}CH_3$

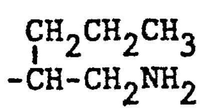

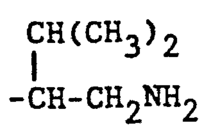

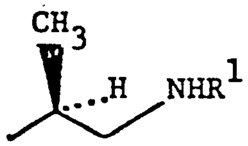 $R^1$ = H, $CH_3$

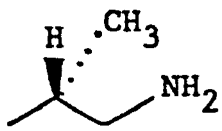

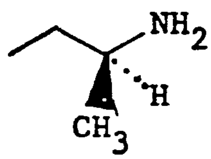

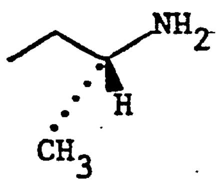

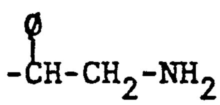

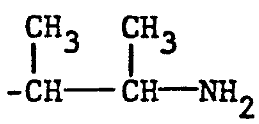

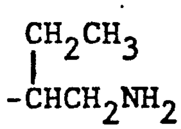

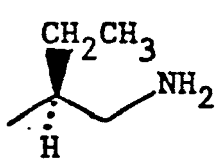

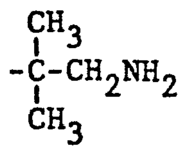

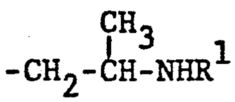 $R^1$ = H, $CH_3$

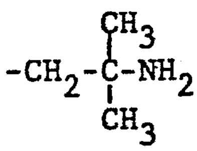

$-CH_2CH_2SCH_3$

$-CH_2CH_2NHC(CH_3)_3$

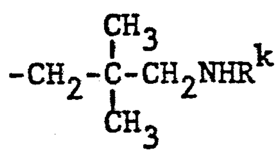 $R^k$ = H, $CH_3$, $\overset{O}{\overset{\|}{C}}CH_3$

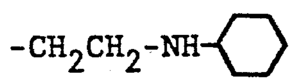

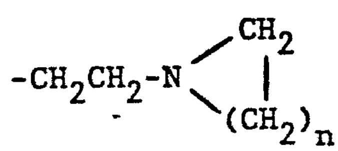 n = 3-5

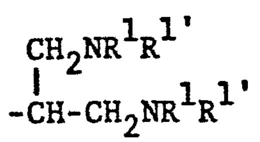 $R^1$ = H, $CH_3$; $R^{1'}$ = H, $CH_3$

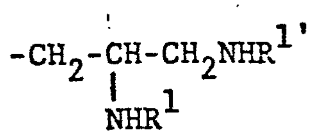 $R^1$=H, $CH_3$; $R^{1'}$ = H, $CH_3$

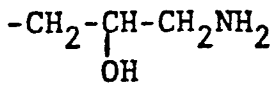

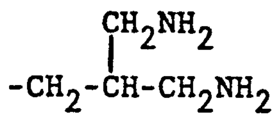

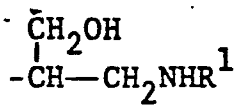 $R^1$ = H, $CH_3$

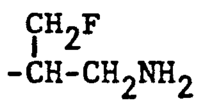

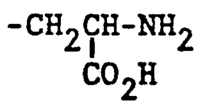

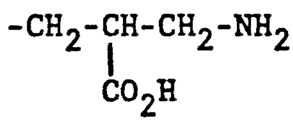

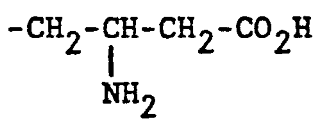

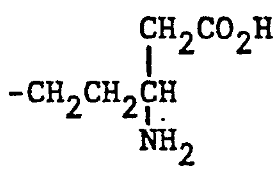

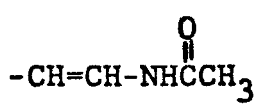

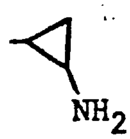

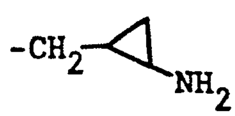

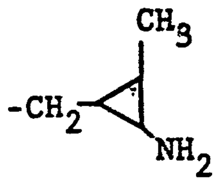

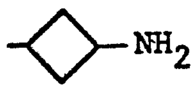

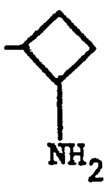

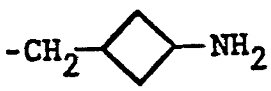

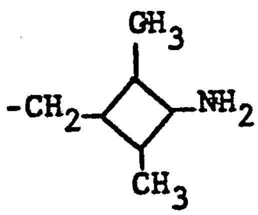

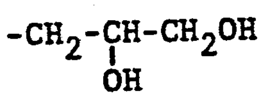

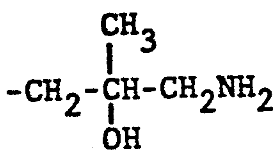

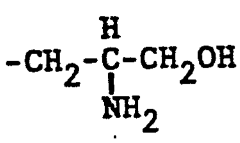

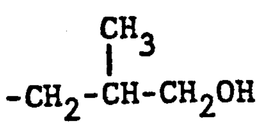

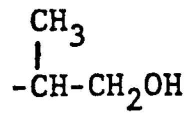

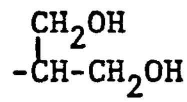

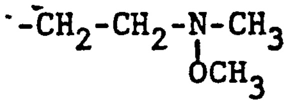

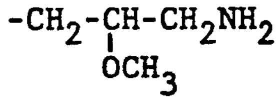

$-CH_2-CH=CH-CH_2NH_2$

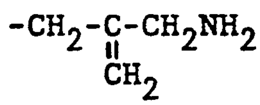

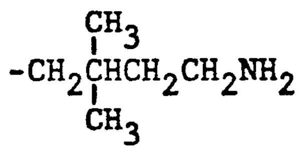

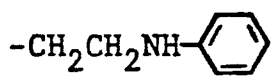

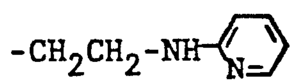

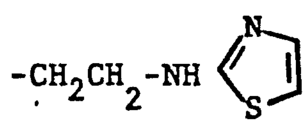

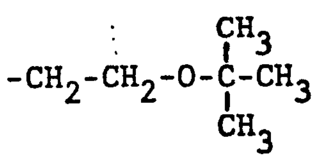

3. Aryl

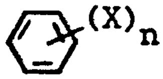

n = 1, 2 or 3,

X = F, Cl, Br, OH, $OR^a$, $O\overset{\overset{O}{\|}}{C}R^a$, $NH_2$, $NHR^a$, $NR^aR^b$, $CH_2NH_2$, $CH_2NR^aR^b$, $CO_2H$, $CO_2R^a$, $COR^a$, $CONH_2$, $CONR^aR^b$, $R^aCONH$, $R^aNHCONH$, $SR^a$, $\overset{\overset{O}{\|}}{S}R^a$, $SO_2R^a$, $CH_3$, $CF_3$;

$R^a$ and $R^b$ are as previously defined under "Substituted Aliphatic";

preferred aryl groups are:

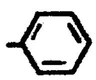
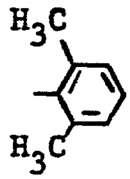
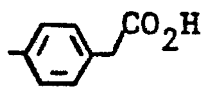

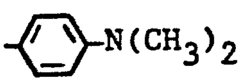
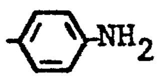
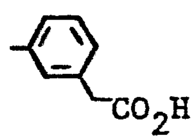

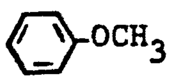
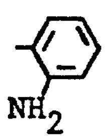
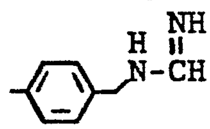

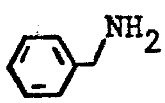
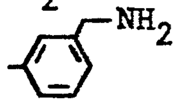
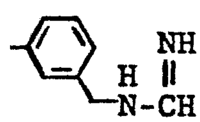

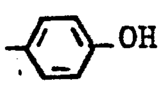
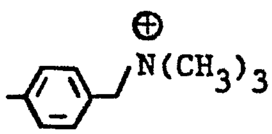
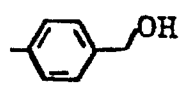

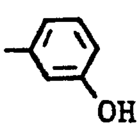
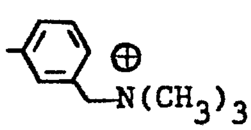
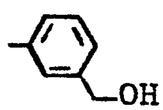

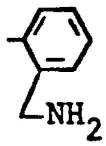

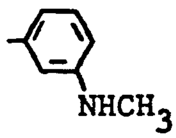

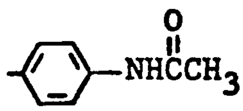

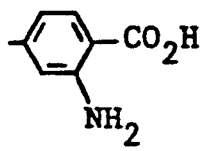

4. Heterocyclic

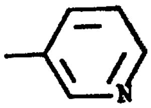

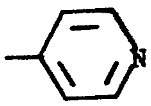

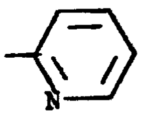

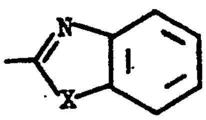

X = N, O, S

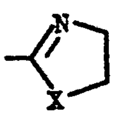

X = N, O, S

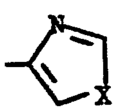

X = N, O, S

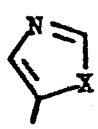

X = N, O, S

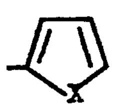

X = NH, O, S

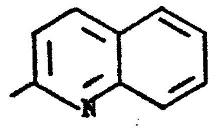

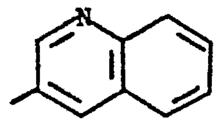

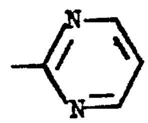

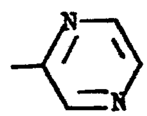

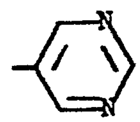

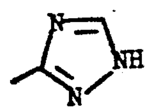

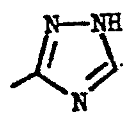

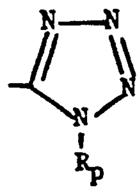

$R^p$ = $-CH_3$
$-H$
$-CH_2SO_3H$
$-CH_2CO_2H$
$-CH_2CH_2SO_3H$
$-CH_2CH_2CO_2H$
$-CH_2CH_2N(CH_3)_2$
$-CH_2CONH_2$
$-CH_2CH_2CONH_2$
$-CH_2CN$
$-CH_2CH_2CN$
$-CH_2CH_3$
$-CH_2CH_2OH$
$-CH_2CH_2NH_2$

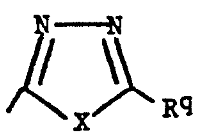

X = NH, S, O

$R^q$ = $-H$
$-CH_3$
$-NH_2$

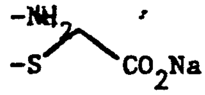

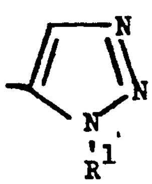

$R^1$ = H
$-CH_3$

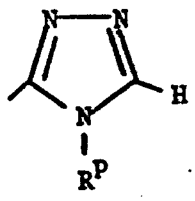

$R^P =$ $-CH_3$
$-CH_2CH_3$
$-H$
$-CH_2CO_2H$
$-CH_2SO_3H$
$-CH_2CH_2CO_2H$
$-CH_2CH_2SO_3H$
$-CH_2CH_2OH$
$-CH_2CH_2NH_2$
$-CH_2CH_2N(CH_3)_2$
$-CH_2CONH_2$
$-CH_2CH_2CONH_2$
$-CH_2CN$
$-CH_2CH_2CN$

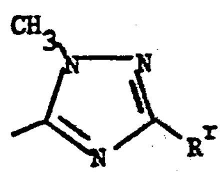

$R^r = -CO_2^{\theta}$
$-CH_2OH$
$-CH_2NH_2$
$-CH_2CH_2OH$
$-CH_2CH_2NH_2$
$-CH_2CO_2^{\theta}$
$-CH_2CH_2CO_2^{\theta}$
$-CH_2CH_2N(CH_3)_2$
$-CH_2CONH_2$
$-CH_2CN$
$-CH_2CH_2CONH_2$

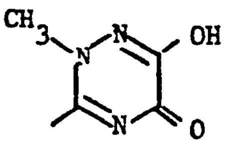

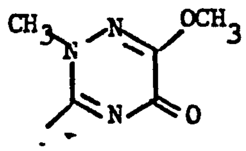

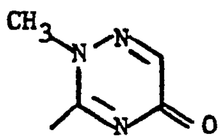

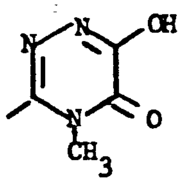

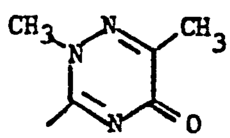

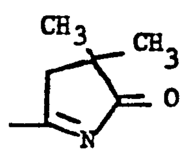

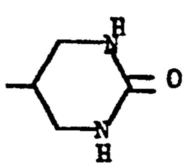

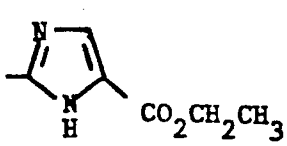

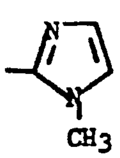

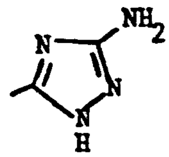

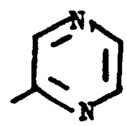

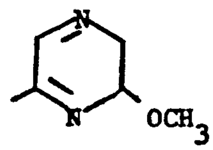

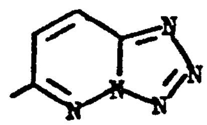

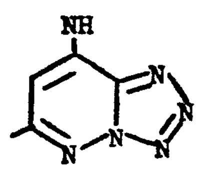

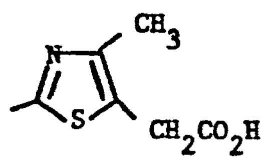

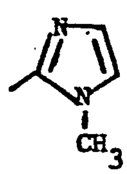

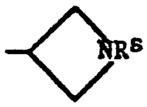

$R^{s} = H, -\overset{\overset{NH}{\|}}{C}H, -\overset{\overset{NH}{\|}}{C}-CH_3, CH_3$

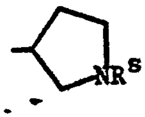

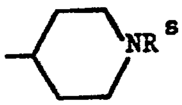

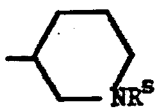

5. <u>Arylaliphatic</u>

Y- Aryl, where the preferred aryl groups are as illustrated above, and -Y- is as originally defined, and is preferably $(CH_2)_n$ where n = 1-3.

6. Heterocycloaliphatic

Y - Heterocyclic, where the preferred heterocyclic groups are as illustrated above, and -Y- is as originally defined, and is preferably $(CH_2)_n$ where n - 1-3; preferred heterocycloaliphatic groups are:

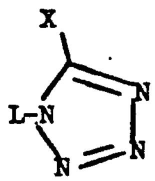

X = H, $CH_3$, $NH_2$

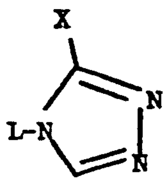

X = H, $CH_3$, $NH_2$

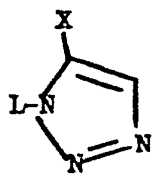

X = H, $CH_3$, $NH_2$

7. <u>Alkyl-Heteroatom-Alkyl</u>

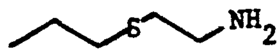

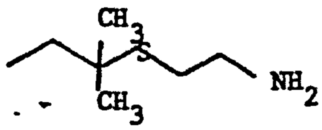

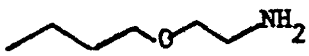

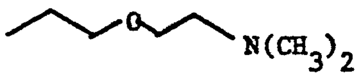

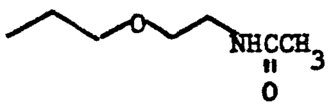

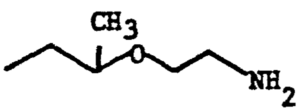

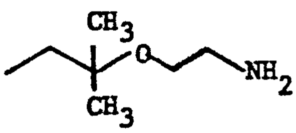

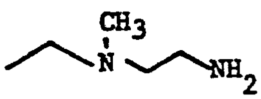

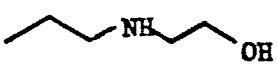

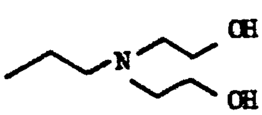

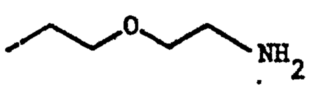

8. Heteroarylium

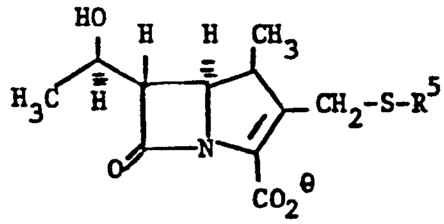

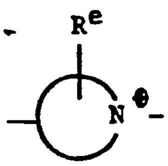

$R^5$ = 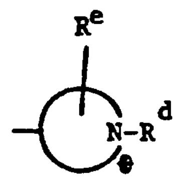

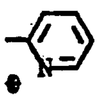

**$R^d$**

$CH_3$
$CH_2CH_3$
$CH_2CO_2H$
$CH_2CONH_2$
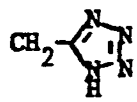
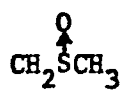
$CH_2SO_3H$

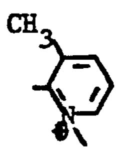 $CH_3$

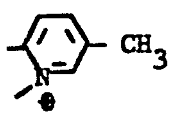 $CH_3$

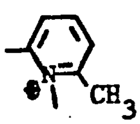 $CH_3$

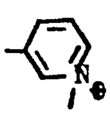

$CH_3$
$CH_2CH_3$
$CH_2CO_2H$
$CH_2CONH_2$
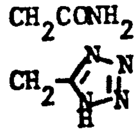
$CH_2SOCH_3$
$CH_2SO_3H$

| 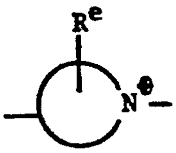 | $R^d$ |
|---|---|
| 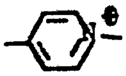 | $CH_3$<br>$CH_2CH_3$<br>$CH_2CO_2H$<br>$CH_2SO_3H$<br>$CH_2CONH_2$<br>$CH_2$- 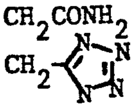<br>$CH_2SOCH_3$ |
| 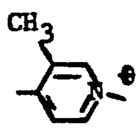 | $CH_3$ |
| 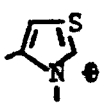 | $CH_3$<br>$CH_2CH_3$ |
| 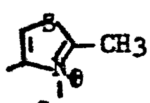 | $CH_3$ |
| 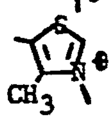 | $CH_3$ |
| 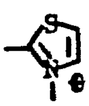 | |
| 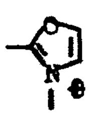 | " |
| 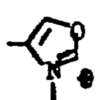 | " |
| 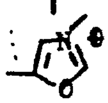 | " |
| 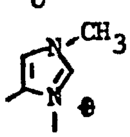 | " |

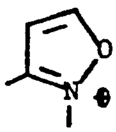 $CH_3$

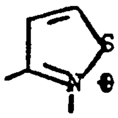 "

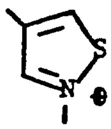 "

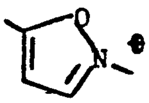 "

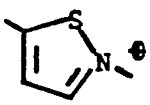 "

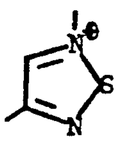 "

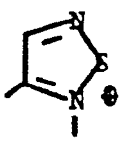 "

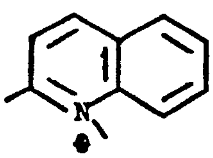 "

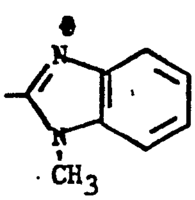 "

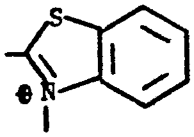

$CH_3$

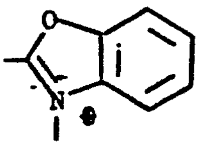

"

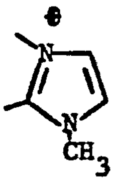

"

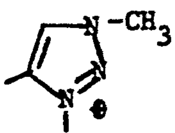

"

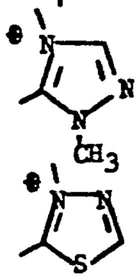

"

"

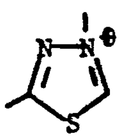

"

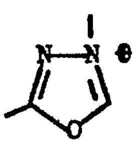

"

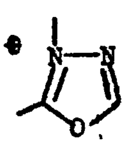

"

| | |
|---|---|
| 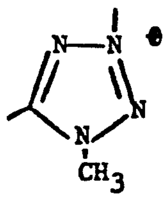 | $CH_3$ |
| 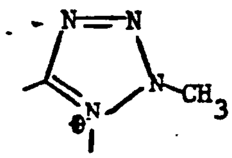 | " |
| 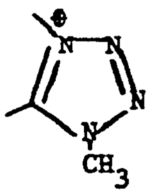 | " |
| 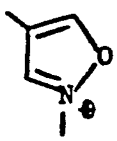 | " |
| 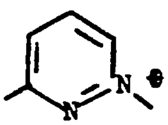 | " |
| 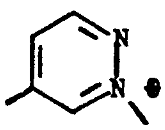 | " |
| 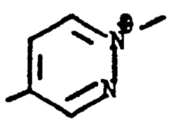 | " |
| 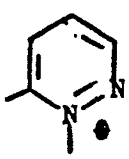 | " |

| | |
|---|---|
| 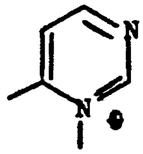 | $CH_3$ |
| 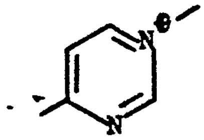 | " |
| 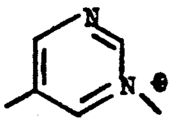 | " |
| 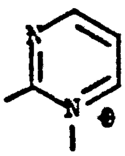 | " |
| 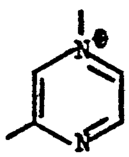 | " |
| 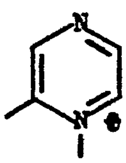 | " |
| 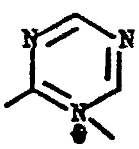 | " |
| 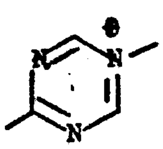 | " |

9. Heteroaryliumaliphatic

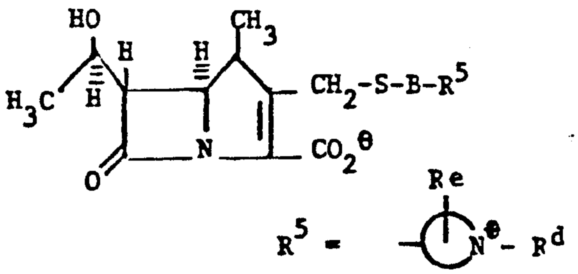

$R^5$ =

| B | 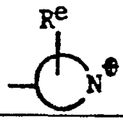 | $R^d$ |
|---|---|---|
| $-CH_2-$ | 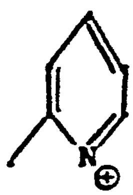 | $CH_2CH_2CH_3$ |
| " | " | $CH_2CH_3$ |
| " | 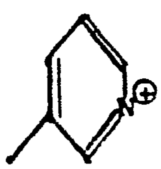 | $CH_2CH_2CH_3$ |
| " | " | $CH_2CH_3$ |
| " | 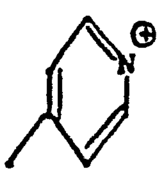 | $CH_2CH_2CH_3$ |

| | | |
|---|---|---|
| -$CH_2$- | " | $CH_2CH_3$ |
| " | 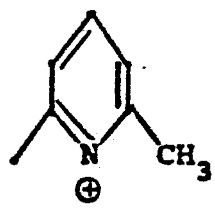 | $CH_3$ |
| " | 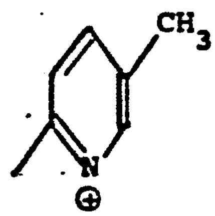 | " |
| " | 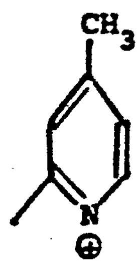 | " |
| " | 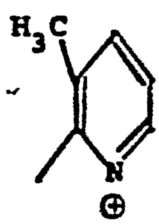 | " |
| " | 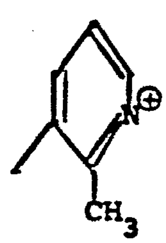 | " |

| | | |
|---|---|---|
| $-CH_2-$ | 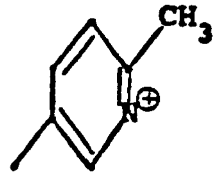 | $CH_3$ |
| " | 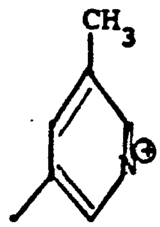 | " |
| " | 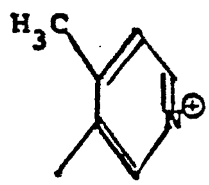 | " |
| " | 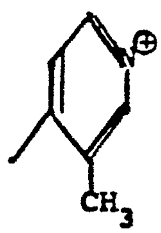 | " |
| " | 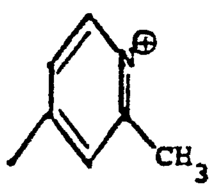 | " |
| $-CH_2CH_2-$ | 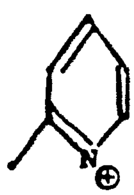 | " |

| | | |
|---|---|---|
| $-CH_2CH_2-$ | 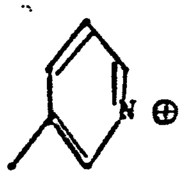 | $CH_3$ |
| " | 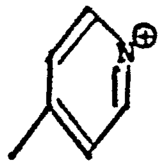 | " |
| -CH-<br>$CH_3$ | 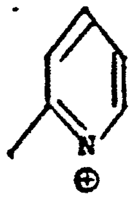 | " |
| " | 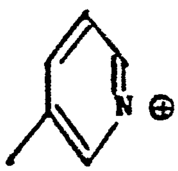 | " |
| " | 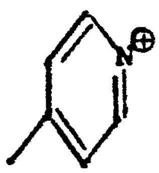 | " |
| $-CH_2-$ | 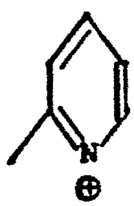 | $ØCH_2$ |

| | | |
|---|---|---|
| $-CH_2-$ | 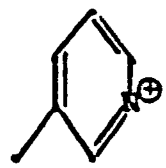 | $\emptyset CH_2$ |
| " | 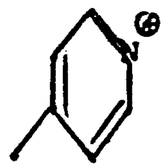 | " |
| " | 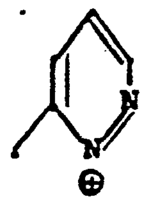 | $CH_3$ |
| " | 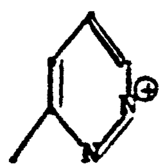 | " |
| " | 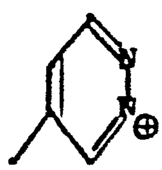 | " |
| " | 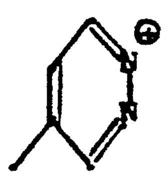 | " |

| | | |
|---|---|---|
| $-CH_2-$ | 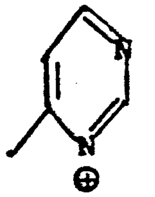 | $CH_3$ . |
| ″ | 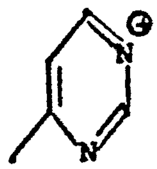 | ″ |
| ″ | 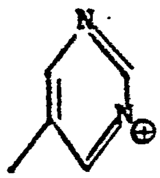 | ″ |
| ″ | 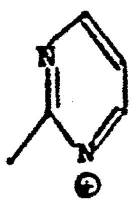 | ″ |
| ″ | 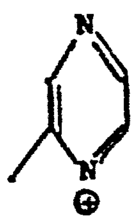 | ″ |
| ″ | 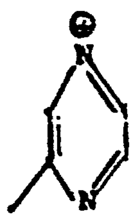 | ″ |

| | | |
|---|---|---|
| $-CH_2-$ | 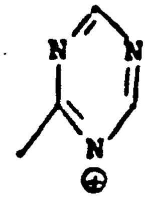 | $CH_3$ |
| " | 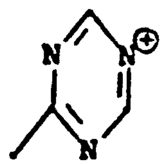 | " |
| " | 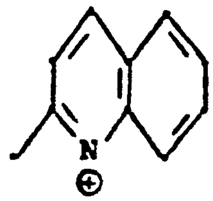 | " |
| " | 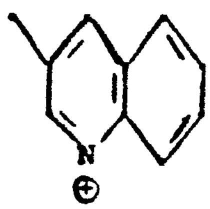 | " |
| " | 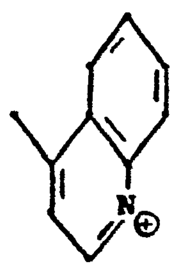 | " |
| " | 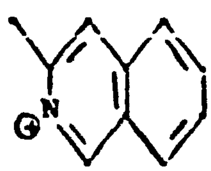 | " |

| | | |
|---|---|---|
| $-CH_2-$ | 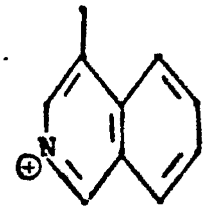 | $CH_3$ |
| " | 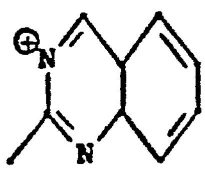 | " |
| " | 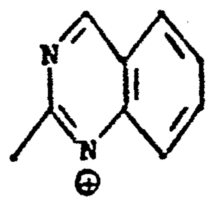 | " |
| " | 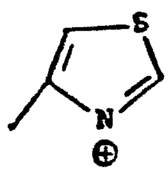 | $CH_2CH_2CH_3$ |
| " | " | $CH_2CH_3$ |
| " | 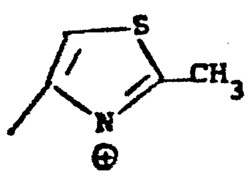 | $CH_3$ |
| " | 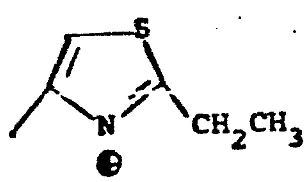 | " |

| | | |
|---|---|---|
| $-CH_2-$ | 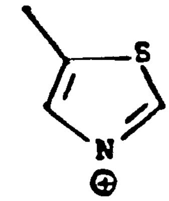 | $CH_3$ |
| " | 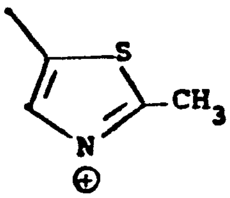 | " |
| $-\underset{CH_3}{CH}-$ | 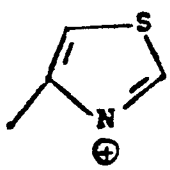 | " |
| " | 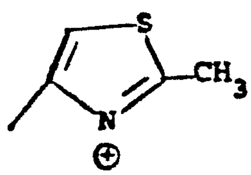 | " |
| $-CH_2-$ | 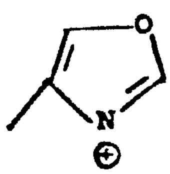 | " |
| " | " | $CH_2CH_3$ |
| " | 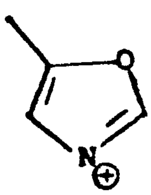 | $CH_3$ |

| | | |
|---|---|---|
| $-CH_2-$ | 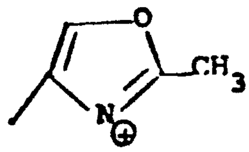 | $CH_3$ |
| " | 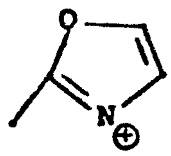 | " |
| " | 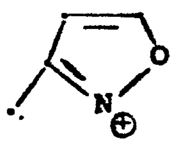 | " |
| " | 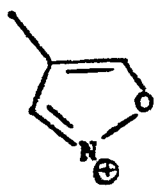 | " |
| " | 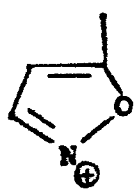 | " |
| " | 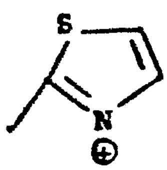 | " |
| $-CH_2CH_2-$ | 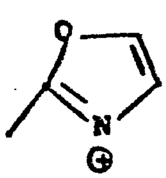 | " |

| | | |
|---|---|---|
| $-CH_2-$ | 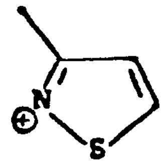 | $CH_3$ |
| " | 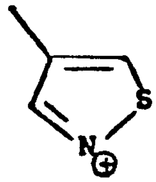 | " |
| " | 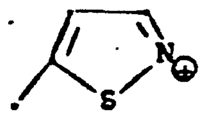 | " |
| " | 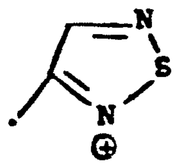 | " |
| " | 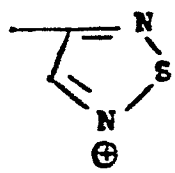 | " |
| " | 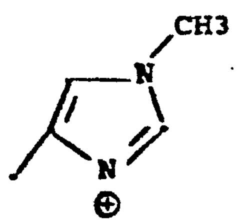 | $-CH_2CH_2CH_3$ |
| " | " | $-CH_2CH_3$ |

| | | |
|---|---|---|
| $-CH_2-$ | 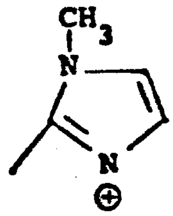 | $-CH_3$ |
| " | 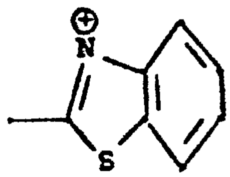 | " |
| " | 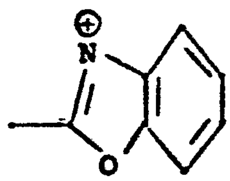 | " |
| " | 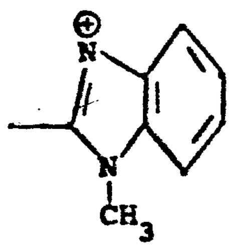 | " |
| " | 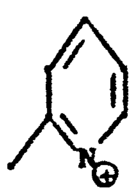 | $CH_2OCH_3$ |
| " | " | $CH_2CN$ |
| " | " | $CH_2CO_2H$ |
| " | " | $CH_2SO_2CH_3$ |

| | | |
|---|---|---|
| $-CH_2-$ | " | $CH_2\overset{O}{\uparrow}{P}(OH)OCH_3$ |
| " | " | $CH_2SO_3H$ |
| " | " | $CH_2CONMe_2$ |
| " | " | $CH_2SOCH_3$ |
| " | " | $CH_2NMe_2$ |
| " | " | $CH_2$ 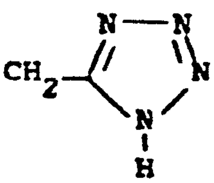 |
| " | 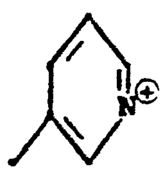 | $CH_2OCH_3$ |
| " | " | $CH_2SCH_3$ |
| " | " | $CH_2SOCH_3$ |
| " | " | $CH_2SO_2CH_3$ |
| " | " | $CH_2CO_2H$ |
| „ | " | $CH_2CONMe_2$ |

| | | |
|---|---|---|
| $-CH_2-$ | " | $CH_2\overset{O}{\overset{\uparrow}{P}}(OH)OCH_3$ |
| " | " | $CH_2SO_3H$ |
| " | " | $CH_2CN$ |
| " | " | $CH_2NMe_2$ |
| " | " | $CH_2CH_2NMe_2$ |
| " | 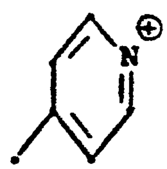 | $CH_2OCH_3$ |
| " | " | $CH_2NMe_2$ |
| " | " | $CH_2CH_2NMe_2$ |
| " | " | $CH_2CN$ |
| " | " | $CH_2SCH_3$ |
| " | " | $CH_2SOCH_3$ |
| " | " | $CH_2SO_2CH_3$ |
| " | " | $CH_2CO_2H$ |
| " | " | $CH_2CONMe_2$ |

| | | |
|---|---|---|
| $-CH_2-$ | " | $CH_2\overset{O}{\uparrow}{P}(OH)OCH_3$ |
| " | " | $CH_2SO_3H$ |
| " | 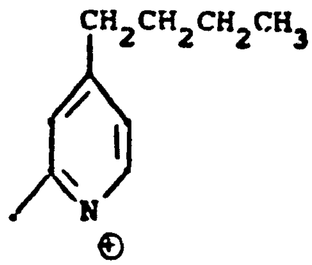 | $CH_3$ |
| $-CH_2CH_2CH_2-$ | 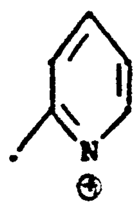 | " |
| $-CH_2\underset{CH_2OH}{CH}-$ | 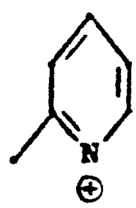 | " |
| $-CH_2-$ | 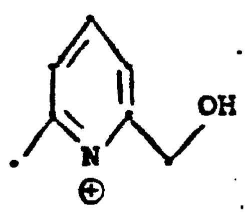 | " |

| | | |
|---|---|---|
| $-CH_2-$ | 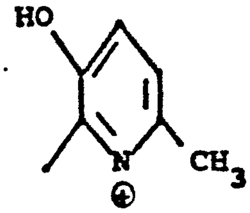 | $CH_3$ |
| " | 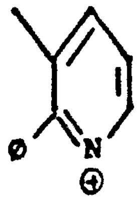 | " |
| $-CH_2CH_2CH_2-$ | 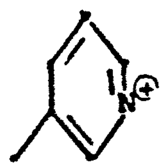 | " |
| $-CH_2CH_2-$ | 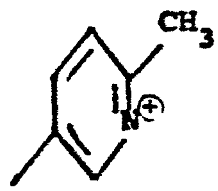 | " |
| $-CH_2-$ | 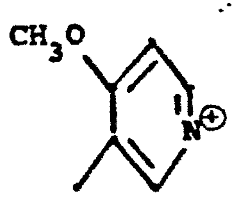 | " |
| " | 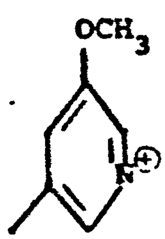 | " |

| | | |
|---|---|---|
| $-CH_2-$ | 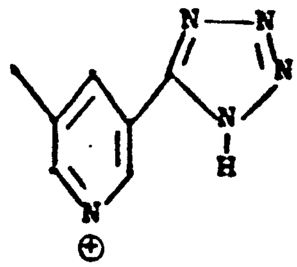 | $CH_3$ |
| $-CH_2CH_2CH_2-$ | 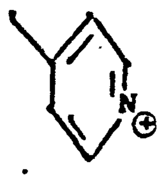 | " |
| $CH_2$ | 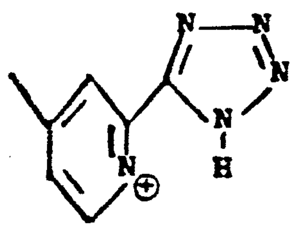 | " |
| " | 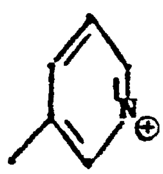 | " |
| " | 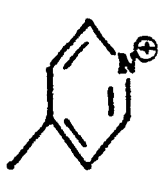 | $CH_2CH_3$ |

$-CH_2-$

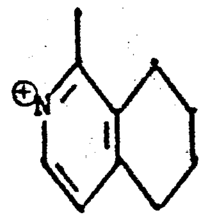

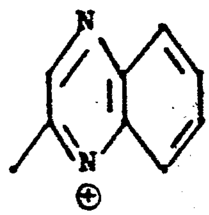

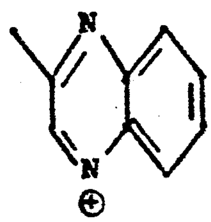

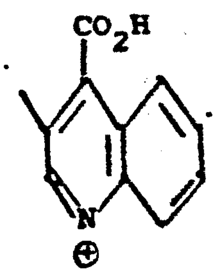

| | | |
|---|---|---|
| $-CH_2-$ | 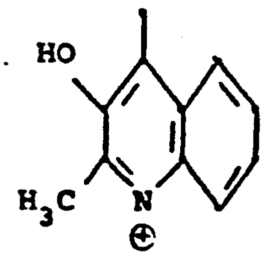 | $CH_3$ |
| $-CH_2CH_2-$ | 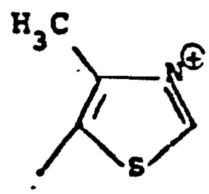 | " |
| $-CH(CH_3)-$ | 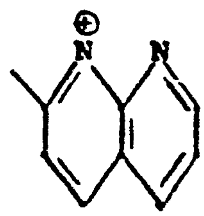 | " |

| | | |
|---|---|---|
| $-CH_2-$ | 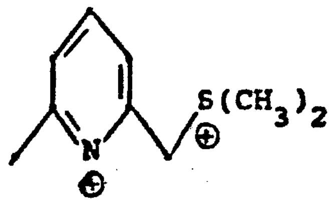 | $CH_3$ |
| " | 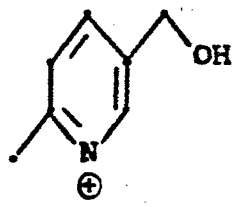 | " |
| " | 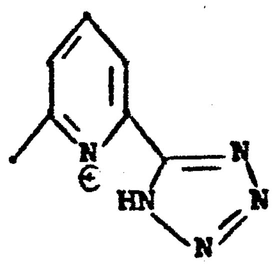 | " |
| " | 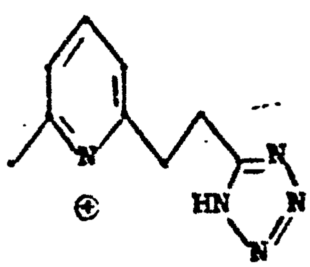 | " |
| " | 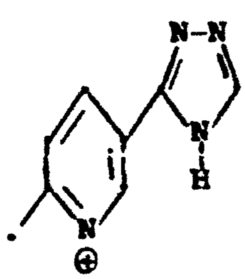 | " |

| | | |
|---|---|---|
| $CH_2$ | 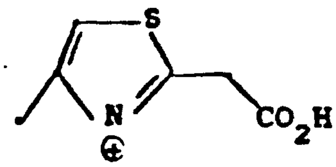 | $CH_3$ |
| " | 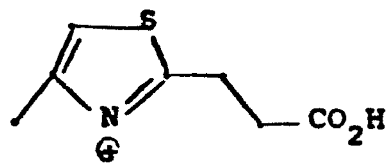 | " |
| " | 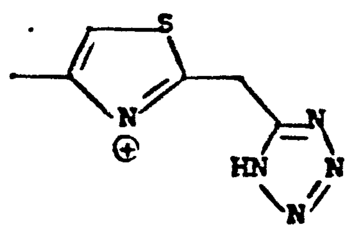 | " |
| " | 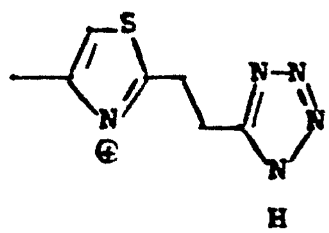 | " |
| " | 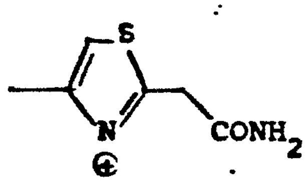 | " |
| " | 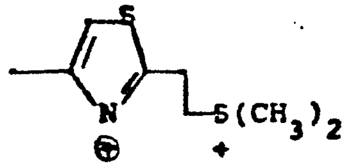 | " |

| | | |
|---|---|---|
| $-CH_2-$ | 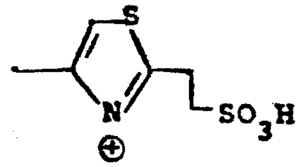 | $CH_3$ |
| " | 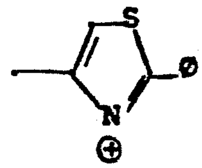 | " |
| " | 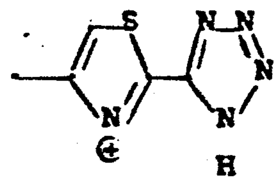 | " |
| " | 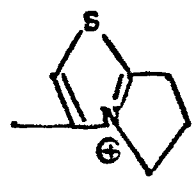 | -- |
| " | 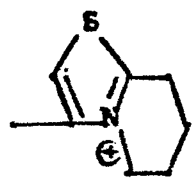 | -- |
| " | 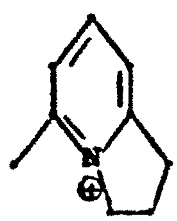 | -- |

| | | |
|---|---|---|
| $-CH_2-$ | 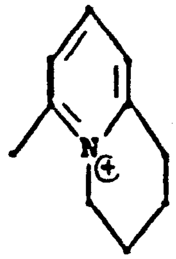 | -- |
| " | 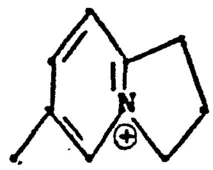 | - |
| " | 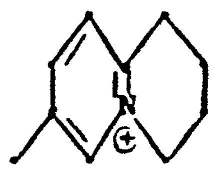 | -- |
| " | 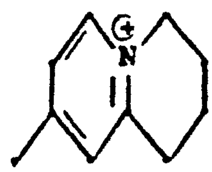 | -- |
| $CH_3$<br>$-CHCH_2-$ | 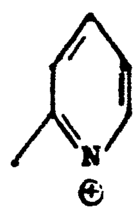 | $CH_3$ |
| $-CH_2-$ | 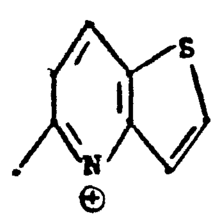 | " |

| | | |
|---|---|---|
| $-CH_2-$ | 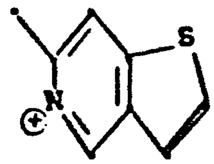 | $CH_3$ |
| " | 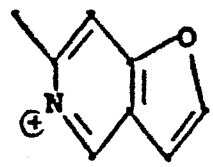 | " |
| " | 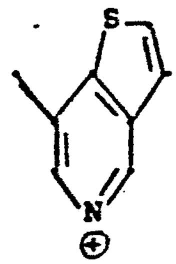 | " |
| " | 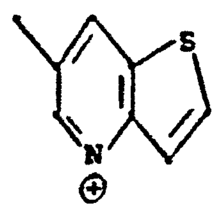 | " |
| " | 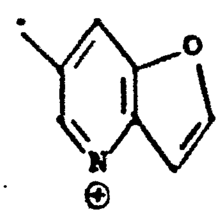 | " |
| " | 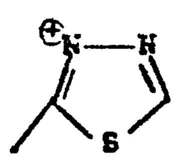 | " |

| | | |
|---|---|---|
| $-CH_2-$ | 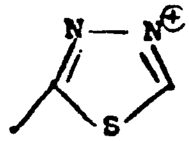 | $CH_3$ |
| " | 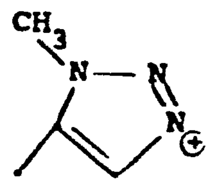 | " |
| " | 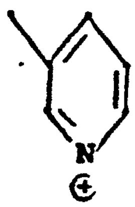 | $CH_2CONH_2$ |
| " | 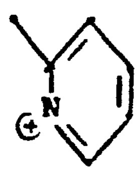 | " |
| " | 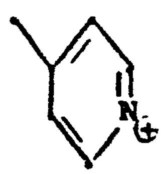 | " |
| " | 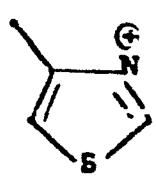 | " |

| | | |
|---|---|---|
| bond | 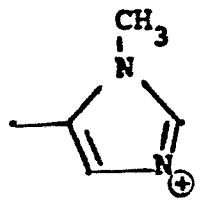 | $CH_3$ |
| " | 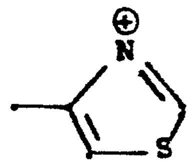 | " |
| " | 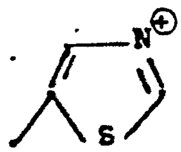 | " |
| " | 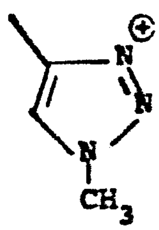 | " |
| " | 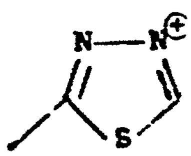 | " |
| " | 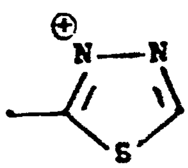 | " |
| " | 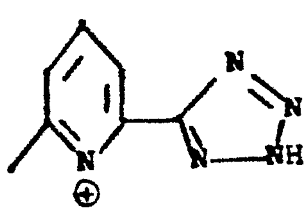 | " |

| | | |
|---|---|---|
| $-CH_2-$ | 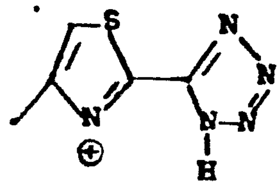 | $CH_3$ |
| " | 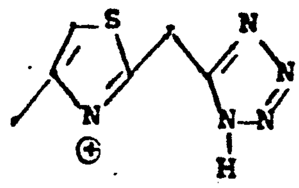 | " |
| " | 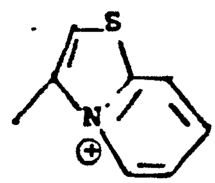 | -- |
| $CH_2$ | 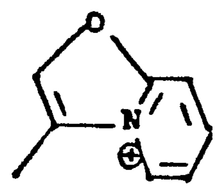 | -- |

| B | 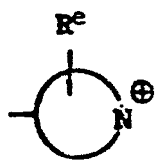 | $R^d$ | $R^e$ |
|---|---|---|---|
| $CH_2$ | 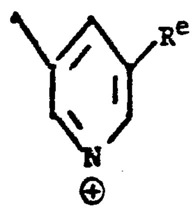 | $CH_3$ | $CO_2H$ |
| " | " | " | $CONH_2$ |
| " | " | " | CN |
| " | " | " | OH |
| " | " | " | $SO_2NH_2$ |
| " | " | " | $SO_3H$ |
| " | " | " | $NMe_2$ |
| " | " | " | $CONMe_2$ |

| | | | |
|---|---|---|---|
| * | * | * | $CH_2NMe_2$ |
| * | * | * | $CH_2CN$ |
| * | * | * | $CH_2CONH_2$ |
| * | * | * | $CH_2CO_2H$ |
| * | * | * | $CH_2SCH_3$ |
| * | * | * | $CH_2SOCH_3$ |
| * | * | * | $CH_2SO_2CH_3$ |
| * | * | * | $SO_2CH_3$ |
| * | * | * | $SOCH_3$ |
| * | * | * | 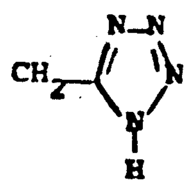 |
| * | * | * | $CH_2CH_2CO_2H$ |
| * | * | * | $CH_2SO_3H$ |
| * | * | * | $CH_2OCH_3$ |
| * | * | * | 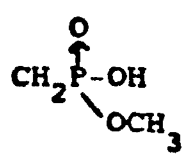 |

| | | | |
|---|---|---|---|
| • | • | • | $CH_2CH_2SO_3H$ |
| • | • | • | $CF_3$ |
| • | • | • | $CH_2OC(=O)NH_2$ |
| • | • | • | $CH_2SO_2NH_2$ |
| • | • | • | Br |
| • | • | • | Cl |
| • | • | • | F |
| • | 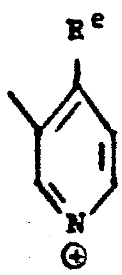 | • | $CO_2H$ |
| • | • | • | $CONH_2$ |
| • | • | • | CN |
| • | • | • | OH |
| • | • | • | $SONH_2$ |

| | | | |
|---|---|---|---|
| " | " | " | $SO_3H$ |
| " | " | " | $NMe_2$ |
| " | " | " | $CONMe_2$ |
| " | " | " | $CH_2NMe_2$ |
| " | " | " | $CH_2CN$ |
| " | " | " | $CH_2CONH_2$ |
| " | " | " | $CH_2CO_2H$ |
| " | " | " | $CH_2SCH_3$ |
| " | " | " | $CH_2SOCH_3$ |
| " | " | " | $CH_2SO_2CH_3$ |
| " | " | " | $SO_2CH_3$ |
| " | " | " | $SOCH_3$ |
| " | " | " | $CH_2$– 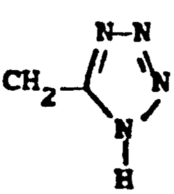 |
| " | " | " | $CH_2CH_2CO_2H$ |
| " | " | " | $CH_2SO_3H$ |
| " | " | " | $CH_2OCH_3$ |

| | | |
|---|---|---|
| ■ | ■ | ■ $CH_2\overset{O}{\overset{\uparrow}{P}}-OH$ ╲ $OCH_3$ |
| ■ | ■ | ■ $CH_2CH_2SO_3H$ |
| ■ | ■ | ■ $CF_3$ |
| ■ | ■ | ■ $CH_2O\overset{O}{\overset{\|}{C}}NH_2$ |
| ■ | ■ | ■ $CH_2SO_2NH_2$ |
| ■ | ■ | ■ $CH_2SO_2NMe_2$ |
| ■ | 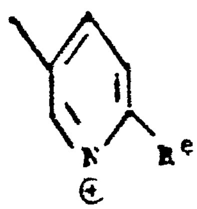 | ■ $CO_2H$ |
| ■ | ■ | ■ $CONH_2$ |
| ■ | ■ | ■ CN |
| ■ | ■ | ■ $OCH_3$ |
| ■ | ■ | ■ $SO_2NH_2$ |
| ■ | ■ | ■ $SO_3H$ |
| ■ | ■ | ■ $NMe_2$ |

| | | | |
|---|---|---|---|
| " | " | " | $CONMe_2$ |
| " | " | " | $CH_2NMe_2$ |
| " | " | " | $CH_2CN$ |
| " | " | " | $CH_2CONH_2$ |
| " | " | " | $CH_2CO_2H$ |
| " | " | " | $CH_2SCH_3$ |
| " | " | " | $CH_2SOCH_3$ |
| " | " | " | $CH_2SO_2CH_3$ |
| " | " | " | $SO_2CH_3$ |
| " | " | " | $SOCH_3$ |
| " | " | " | $CH_2$ - 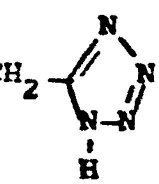 |
| " | " | " | $CH_2CH_2CO_2H$ |
| " | " | " | $CH_2SO_3H$ |
| " | " | " | $CH_2OCH_3$ |

| | | | |
|---|---|---|---|
| " | " | " | 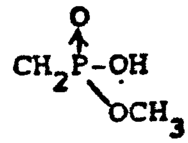 |
| " | " | " | $CH_2CH_2SO_3H$ |
| " | " | " | $CF_3$ |
| " | " | " | 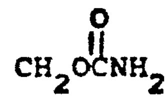 |
| " | " | " | $CH_2SO_2NH_2$ |
| " | " | " | $CH_2SO_2NMe_2$ |
| " | " | " | 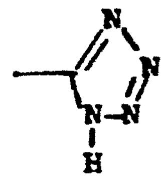 |
| " | 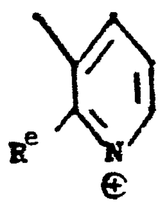 | " | $CO_2H$ |
| " | " | " | $CONH_2$ |
| " | " | " | CN |
| " | " | " | $OCH_3$ |

| | | | |
|---|---|---|---|
| ■ | ■ | ■ | $SO_2NH_2$ |
| ■ | ■ | ■ | $SO_3H$ |
| ■ | ■ | ■ | $NMe_2$ |
| ■ | ■ | ■ | $CONMe_2$ |
| ■ | ■ | ■ | $CH_2NMe_2$ |
| ■ | ■ | ■ | $CH_2CN$ |
| ■ | ■ | ■ | $CH_2CONH_2$ |
| ■ | ■ | ■ | $CH_2CO_2H$ |
| ■ | ■ | ■ | $CH_2SCH_3$ |
| ■ | ■ | ■ | $CH_2SOCH_3$ |
| $CH_3$ | ■ | ■ | $CH_2SO_2CH_3$ |
| ■ | ■ | ■ | $SO_2CH_3$ |
| ■ | ■ | ■ | $SOCH_3$ |
| ■ | ■ | ■ | 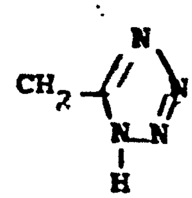 |
| ■ | ■ | ■ | $CH_2CH_2CO_2H$ |

| | | | |
|---|---|---|---|
| " | " | " | $CH_2SO_3H$ |
| " | " | " | $CH_2OCH_3$ |
| " | " | " | 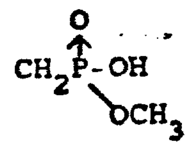 |
| " | " | " | $CH_2CH_2SO_3H$ |
| " | " | " | $CF_3$ |
| " | " | " | $CH_2OC(O)NH_2$ |
| " | " | " | $CH_2SO_2NH_2$ |
| " | " | " | $CH_2SO_2NMe_2$ |
| " | " | " | 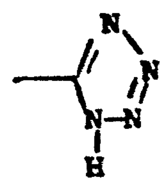 |
| $-CH_2-$ | 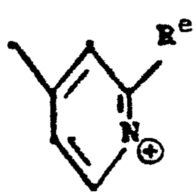 | " | $CO_2H$ |
| " | " | " | $CONH_2$ |
| " | " | " | CN |

| | | | |
|---|---|---|---|
| " | " | " | $OCH_3$ |
| " | " | " | $SO_2NH_2$ |
| " | " | " | $SO_3H$ |
| " | " | " | $NMe_2$ |
| " | " | " | $CONMe_2$ |
| " | " | " | $CH_2NMe_2$ |
| " | " | " | $CH_2CN$ |
| " | " | " | $CH_2CONH_2$ |
| " | " | " | $CH_2CO_2H$ |
| " | " | " | $CH_2SCH_3$ |
| " | " | " | $CH_2SOCH_3$ |
| " | " | " | $CH_2SO_2CH_3$ |
| " | " | " | $SO_2CH_3$ |
| " | " | " | $SOCH_3$ |
| " | " | " | $CH_2$– 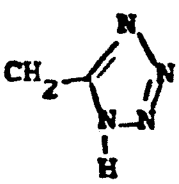 |

| | | | |
|---|---|---|---|
| " | " | " | $CH_2CH_2CO_2H$ |
| " | " | " | $CH_2SO_3H$ |
| " | " | " | $CH_2OCH_3$ |
| " | " | " | 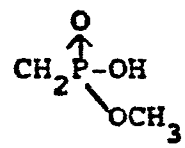 |
| " | " | " | $CH_2CH_2SO_3H$ |
| " | " | " | $CF_3$ |
| " | " | " | 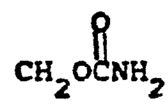 |
| " | " | " | $CH_2SO_2NH_2$ |
| " | " | " | $CH_2SO_2NMe_2$ |
| " | " | " | 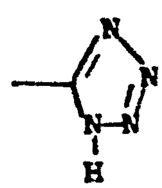 |
| " | 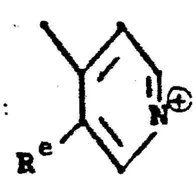 | " | $CO_2H$ |
| " | " | " | $CONH_2$ |

| | | | |
|---|---|---|---|
| " | " | " | CN |
| " | " | " | OH |
| " | " | " | $OCH_3$ |
| " | " | " | $SO_2NH_2$ |
| " | " | " | $SO_3H$ |
| " | " | " | $NMe_2$ |
| " | " | " | $CONMe_2$ |
| " | " | " | $CH_2NMe_2$ |
| " | " | " | $CH_2CN$ |
| " | " | " | $CH_2CONH_2$ |
| " | " | " | $CH_2CO_2H$ |
| " | " | " | $CH_2SCH_3$ |
| " | " | " | $CH_2SOCH_3$ |
| " | " | " | $CH_2SO_2CH_3$ |
| " | " | " | $SO_2CH_3$ |
| " | " | " | $SOCH_3$ |

| | | | |
|---|---|---|---|
| " | " | " | 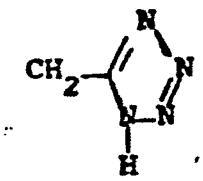 |
| " | " | " | $CH_2CH_2CO_2H$ |
| " | " | " | $CH_2SO_3H$ |
| " | " | " | $CH_2OCH_3$ |
| " | " | " | 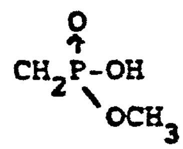 |
| " | " | " | $CH_2CH_2SO_3H$ |
| " | " | " | $CF_3$ |
| " | " | " | 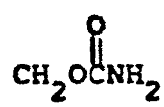 |
| " | " | " | $CH_2SO_2NH_2$ |
| " | " | " | $CH_2SO_2NMe_2$ |
| " | " | " | 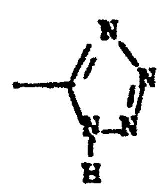 |

| | | | |
|---|---|---|---|
| " | " | " | F |
| " | " | " | Cl |
| " | " | " | Br |
| " | 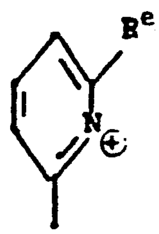 | " | $CO_2H$ |
| " | " | " | $CONH_2$ |
| " | " | " | CN |
| " | " | " | $SO_2NH_2$ |
| " | " | " | $SO_3H$ |
| " | " | " | $NMe_2$ |
| " | " | " | $CONMe_2$ |
| " | " | " | $CH_2NMe_2$ |
| " | " | " | $CH_2CN$ |
| " | " | " | $CH_2CONH_2$ |
| " | " | " | $CH_2CO_2H$ |

| | | | |
|---|---|---|---|
| " | " | " | $CH_2SCH_3$ |
| " | " | " | $CH_2SOCH_3$ |
| " | " | " | $CH_2SO_2CH_3$ |
| " | " | " | $SO_2CH_3$ |
| " | " | " | 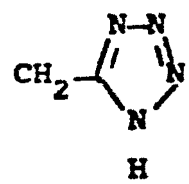 |
| " | " | " | $CH_2CH_2CO_2H$ |
| " | " | " | $CH_2SO_3H$ |
| " | " | " | $CH_2OCH_3$ |
| " | " | " | 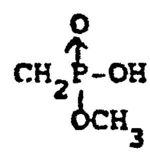 |
| " | " | " | $CH_2CH_2SO_3H$ |
| " | " | " | $CF_3$ |
| " | " | " | 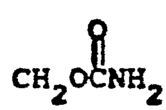 |
| " | " | " | $CH_2SO_2NH_2$ |

| | | | |
|---|---|---|---|
| " | 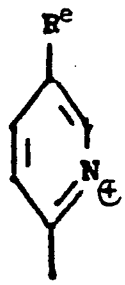 | " | $CO_2H$ |
| " | " | " | $CONH_2$ |
| " | " | " | CN |
| " | " | " | OH |
| " | " | " | $SO_2NH_2$ |
| " | " | " | $SO_3H$ |
| " | " | " | $NMe_2$ |
| " | " | " | $CONMe_2$ |
| " | " | " | $CH_2NMe_2$ |
| " | " | " | $CH_2CN$ |
| " | " | " | $CH_2CONH_2$ |
| " | " | " | $CH_2CO_2H$ |
| " | " | " | $CH_2SCH_3$ |
| " | " | " | $CH_2SOCH_3$ |

| | | |
|---|---|---|
| " | " | " $CH_2SO_2CH_3$ |
| " | " | " $SO_2CH_3$ |
| " | " | " $SOCH_3$ |
| " | " | " 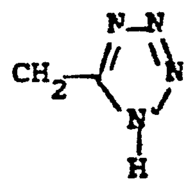 |
| " | " | " $CH_2CH_2CO_2H$ |
| " | " | " $CH_2SO_3H$ |
| " | " | " $CH_2OCH_3$ |
| " | " | " 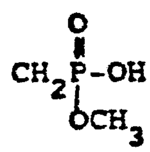 |
| " | " | " $CH_2CH_2SO_3H$ |
| " | " | " $CF_3$ |
| " | " | " 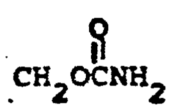 |
| " | " | " $CH_2SO_2NH_2$ |
| " | " | " Br |

| | | |
|---|---|---|
| ■ | ■ | ■ Cl |
| ■ | ■ | ■ F |
| ■ | 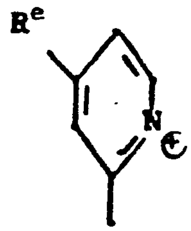 | ■ $CO_2H$ |
| ■ | ■ | ■ $CONH_2$ |
| ■ | ■ | ■ CN |
| ■ | ■ | ■ $SO_2NH_2$ |
| ■ | ■ | ■ $SO_3H$ |
| ■ | ■ | ■ $NMe_2$ |
| ■ | ■ | ■ $CONMe_2$ |
| ■ | ■ | ■ $CH_2NMe_2$ |
| ■ | ■ | ■ $CH_2CN$ |
| ■ | ■ | ■ $CH_2CONH_2$ |
| ■ | ■ | ■ $CH_2CO_2H$ |
| ■ | ■ | ■ $CH_2SCH_3$ |

| | | | |
|---|---|---|---|
| * | * | * | $CH_2SOCH_3$ |
| * | * | * | $CH_2SO_2CH_3$ |
| * | * | * | $SO_2CH_3$ |
| * | * | * | $SOCH_3$ |
| * | * | * | 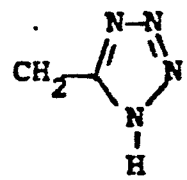 |
| * | * | * | $CH_2CH_2CO_2H$ |
| * | * | * | $CH_2SO_3H$ |
| * | * | * | $CH_2OCH_3$ |
| * | * | * | 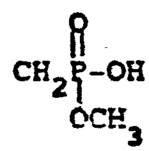 |
| * | * | * | $CH_2CH_2SO_3H$ |
| * | * | * | $CF_3$ |
| * | * | * | 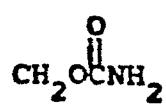 |
| * | * | * | $CH_2SO_2NH_2$ |

| | | | |
|---|---|---|---|
| " | 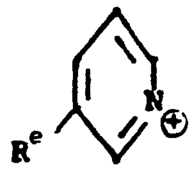 | " | $CO_2H$ |
| " | " | " | $CONH_2$ |
| " | " | " | CN |
| " | " | " | OH |
| " | " | " | $SO_2NH_2$ |
| " | " | " | $SO_3H$ |
| " | " | " | $NMe_2$ |
| " | " | " | $CONMe_2$ |
| " | " | " | $CH_2NMe_2$ |
| " | " | " | $CH_2CN$ |
| " | " | " | $CH_2CONH_2$ |
| " | " | " | $CH_2CO_2H$ |
| " | " | " | $CH_2SCH_3$ |
| " | " | " | $CH_2SOCH_3$ |

| | | | |
|---|---|---|---|
| ■ | ■ | ■ | $CH_2SO_2CH_3$ |
| ■ | ■ | ■ | $SO_2CH_3$ |
| ■ | ■ | ■ | $SOCH_3$ |
| ■ | ■ | ■ | 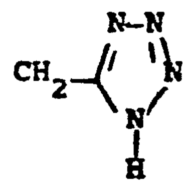 |
| ■ | ■ | ■ | $CH_2CH_2CO_2H$ |
| ■ | ■ | ■ | $CH_2SO_3H$ |
| ■ | ■ | ■ | $CH_2OCH_3$ |
| ■ | ■ | ■ | 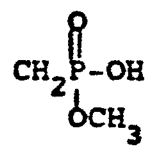 |
| ■ | ■ | ■ | $CH_2CH_2SO_3H$ |
| ■ | ■ | ■ | $CF_3$ |
| ■ | ■ | ■ | 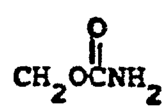 |
| ■ | ■ | ■ | $CH_2SO_2NH_2$ |
| ■ | ■ | ■ | Br |
| ■ | ■ | ■ | Cl |
| ■ | ■ | ■ | F |

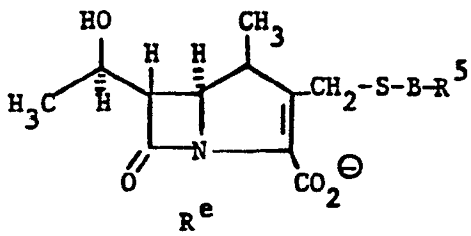

$R^5$ = 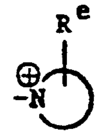

| B | 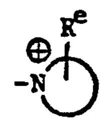 |
| --- | --- |
| $-CH_2CH_2-$ | 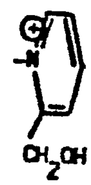 |
| $-CH_2CH_2-$ | 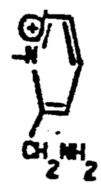 |

$-CH_2CH_2-$ 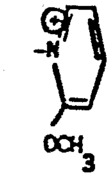

$-CH_2CH_2-$ 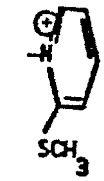

$-CH_2CH_2-$ 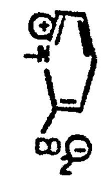

$-CH_2CH_2-$ 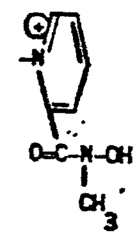

$-CH_2CH_2-$ 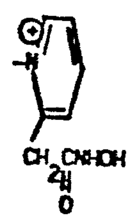

$-CH_2CH_2-$ 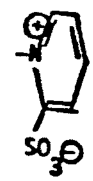

$-CH_2CH_2-$ 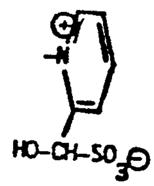

$-CH_2CH_2-$ 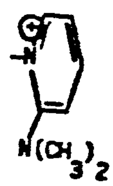

$-CH_2CH_2-$ 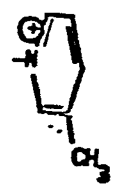

$-CH_2CH_2-$ 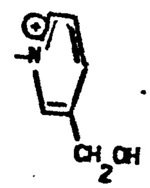

$-CH_2CH_2-$ 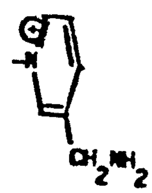

$-CH_2CH_2-$ 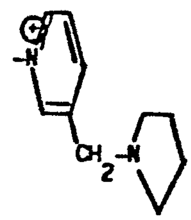

$-CH_2CH_2-$ 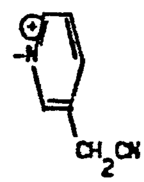

$-CH_2CH_2-$ 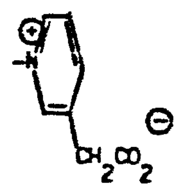

$-CH_2CH_2-$ 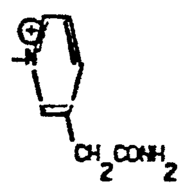

$-CH_2CH_2-$ 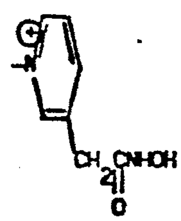

$-CH_2CH_2-$ 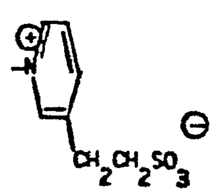

$-CH_2CH_2-$ 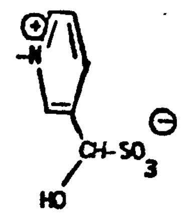

$-CH_2CH_2-$ 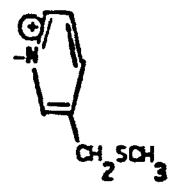

$-CH_2CH_2-$ 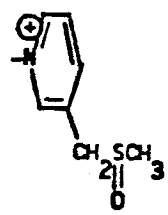

$-CH_2CH_2-$ 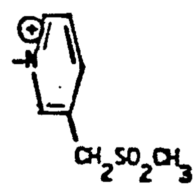

$-CH_2CH_2-$ 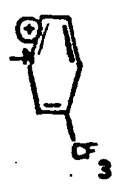

$-CH_2CH_2-$ 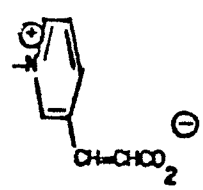

$-CH_2CH_2-$

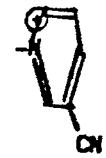

$-CH_2CH_2-$

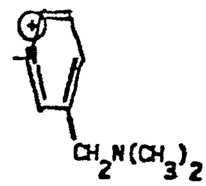

$-CH_2CH_2-$

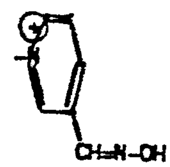

$-CH_2CH_2-$

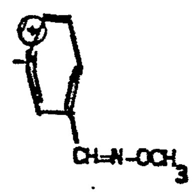

$-CH_2CH_2-$

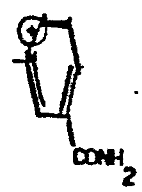

$-CH_2CH_2-$

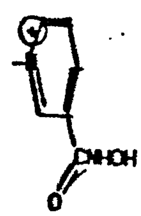

| | |
|---|---|
| $-CH_2CH_2-$ | 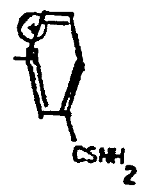 |
| $-CH_2CH_2-$ | 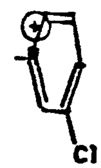 |
| $-CH_2CH_2-$ | 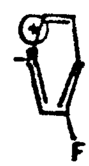 |
| $-CH_2CH_2-$ | 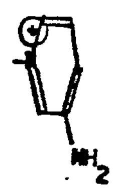 |
| $-CH_2CH_2-$ | 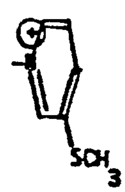 |
| $-CH_2CH_2-$ | 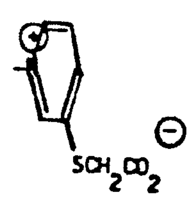 |

| | |
|---|---|
| $-CH_2CH_2-$ | 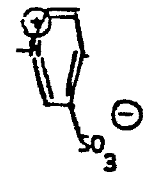 |
| $-CH_2CH_2-$ | 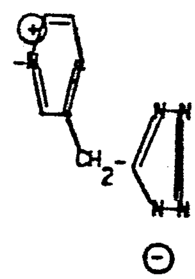 |
| $-CH_2CH_2-$ | 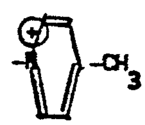 |
| $-CH_2CH_2-$ | 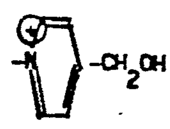 |
| $-CH_2CH_2-$ | 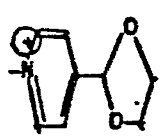 |
| $-CH_2CH_2-$ | 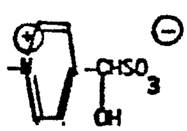 |

| | |
|---|---|
| $-CH_2CH_2-$ | 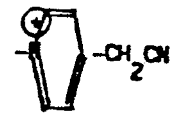 |
| $-CH_2CH_2-$ | 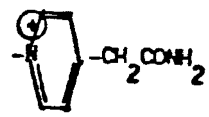 |
| $-CH_2CH_2-$ | 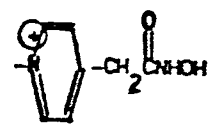 |
| $-CH_2CH_2-$ | 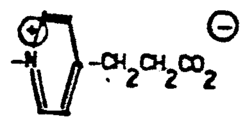 |
| $-CH_2CH_2-$ | 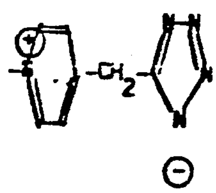 |
| $-CH_2CH_2-$ | 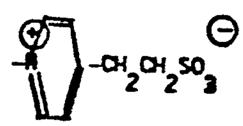 |

$-CH_2CH_2-$ 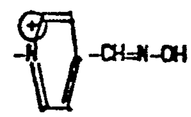

$-CH_2CH_2-$ 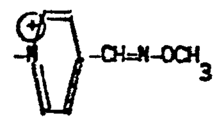

$-CH_2CH_2-$ 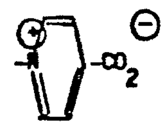

$-CH_2CH_2-$ 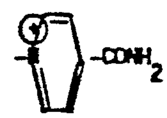

$-CH_2CH_2-$ 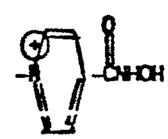

$-CH_2CH_2-$ 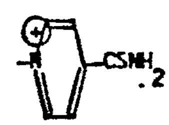

| | |
|---|---|
| $-CH_2CH_2-$ | 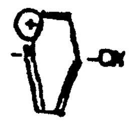 |
| $-CH_2CH_2-$ | 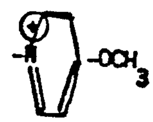 |
| $-CH_2CH_2-$ | 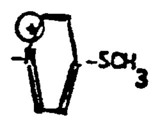 |
| $-CH_2CH_2-$ | 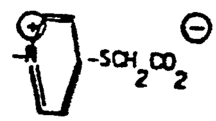 |
| $-CH_2CH_2-$ | 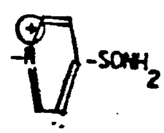 |
| $-CH_2CH_2-$ | 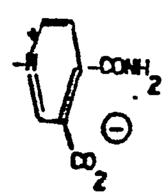 |

$-CH_2CH_2-$ 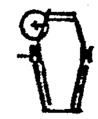

$-CH_2CH_2-$ 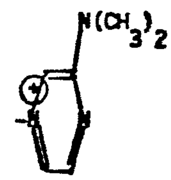

$-CH_2CH_2-$ 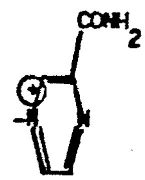

$-CH_2CH_2-$ 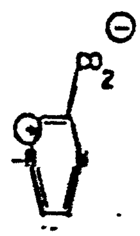

$-CH_2CH_2-$ 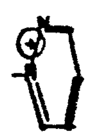

$-CH_2CH_2-$ 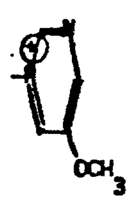

$-CH_2CH_2-$ 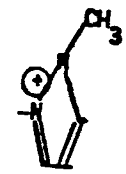

$-CH_2CH_2-$ 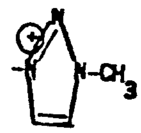

$-CH_2CH_2-$ 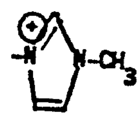

$-CH_2CH_2-$ 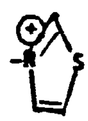

$-CH(CH_3)CH_2-$ 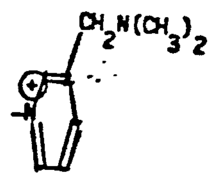

$-CH(CH_3)CH_2-$ 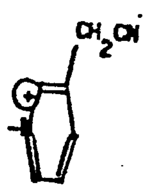

$-CH(CH_3)CH_2-$ 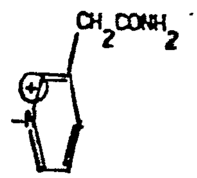

$-CH(CH_3)CH_2-$ 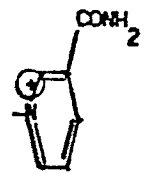

$-CH(CH_3)CH_2-$ 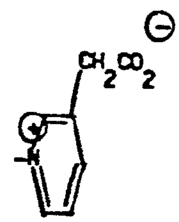

$-CH(CH_3)CH_2-$ 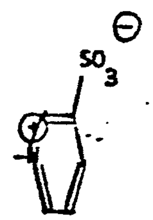

$-CH(CH_3)CH_2-$ 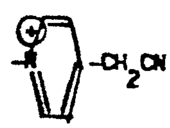

$-CH(CH_3)CH_2-$ 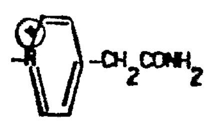

| | |
|---|---|
| $-CH(CH_3)CH_2-$ | 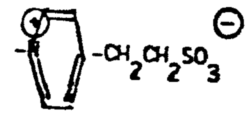 |
| $-CH(CH_3)CH_2-$ | 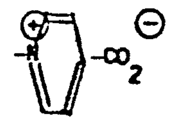 |
| $-CH(CH_3)CH_2-$ | 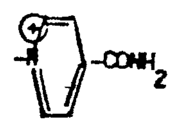 |
| $-CH_2$ | 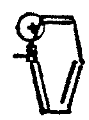 |
| $-CH_2$ | 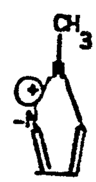 |
| $-CH_2$ | 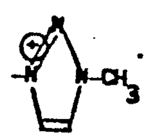 |

| | |
|---|---|
| $-CH(CH_3)-$ | 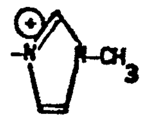 |
| $-CH_2CH_2CH_2-$ | 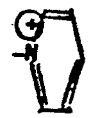 |
| $-CH_2CH_2-$ | 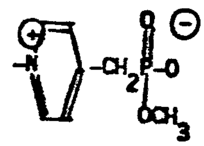 |
| $-CH_2CH_2-$ | 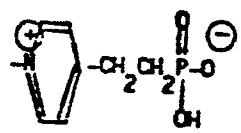 |
| $-CH_2CH_2-$ | 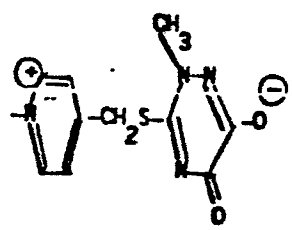 |
| $-CH_2$ | 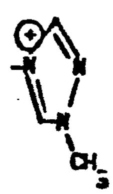 |

$-CH_2-$ 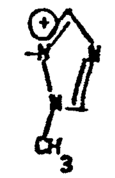

$-CH_2-$ 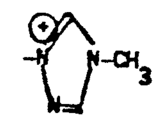

$-CH_2-$ 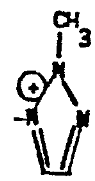

$-CH_2-$ 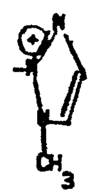

$-CH_2-$ 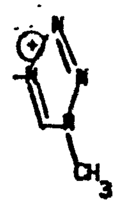

$-CH_2-$ 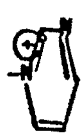

$-CH_2-$ 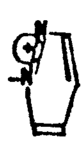

$-CH_2-$ 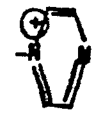

$-CH_2-$ 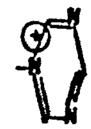

$-CH_2-$ 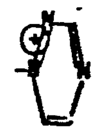

$-CH_2-$ 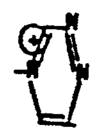

$-CH_2-$ 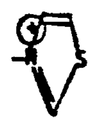

$-CH_2-$ 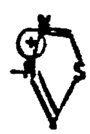

$-CH_2-$ 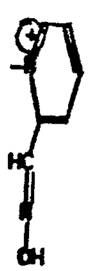

$-CH_2CH_2-$

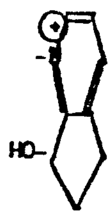

$-CH_2CH_2-$

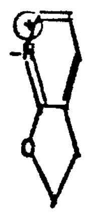

$-CH_2CH_2-$

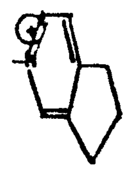

$-CH_2CH_2-$

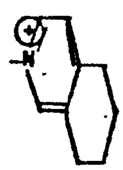

$-CH_2CH_2-$

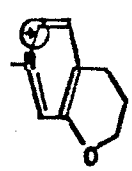

$-CH_2CH_2-$ 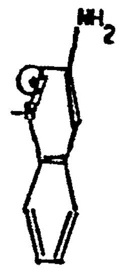

$-CH_2CH_2-$ 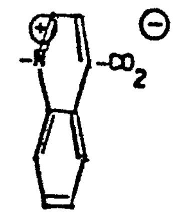

$-CH_2CH_2-$ 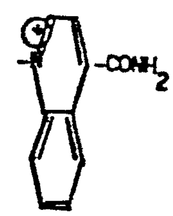

$-CH_2CH_2-$ 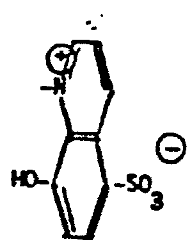

$-CH_2CH_2-$ 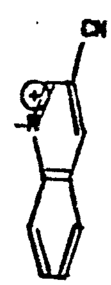

$-CH_2CH_2-$ 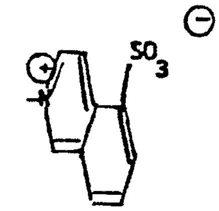

$-CH_2CH_2-$ 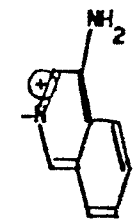

$-CH_2CH_2-$ 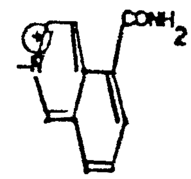

$-CH_2CH_2-$ 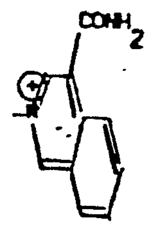

$-CH_2CH_2-$ 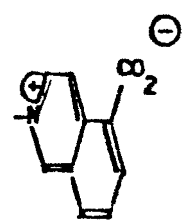

$-CH_2CH_2-$

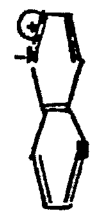

$-CH_2CH_2-$

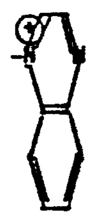

$-CH_2CH_2-$

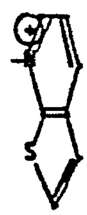

$-CH_2CH_2-$

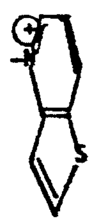

$-CH_2CH_2-$

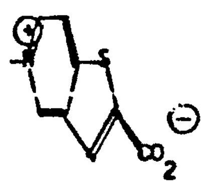

$-CH_2CH_2-$

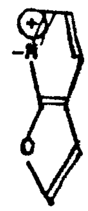

$-CH_2CH_2-$

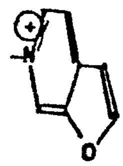

$-CH_2CH_2-$

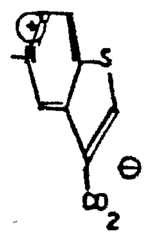

$-CH_2CH_2-$

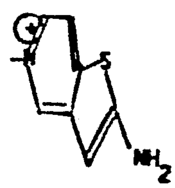

$-CH_2CH_2-$

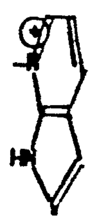

$-CH_2CH_2-$ 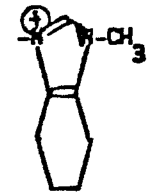

$-CH_2CH_2-$ 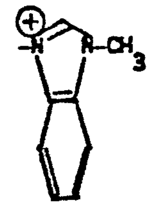

$-CH_2CH_2-$ 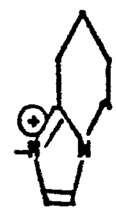

$-CH_2CH_2-$ 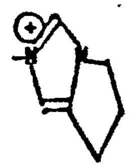

$-CH_2CH_2-$ 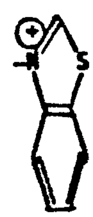

$-CH_2CH_2-$

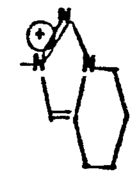

$-CH_2CH_2-$

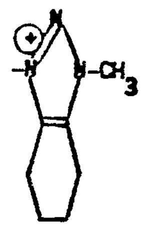

$-CH_2CH_2-$

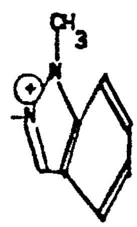

$-CH_2CH_2-$

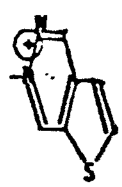

$-CH_2CH_2-$

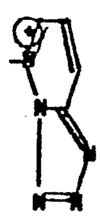

| | |
|---|---|
| $-CH_2CH_2-$ | 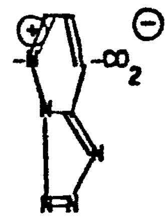 |
| $-CH_2CH_2-$ | 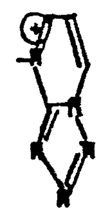 |
| $-CH_2CH_2-$ | 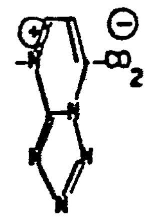 |
| $-CH_2CH_2-$ | 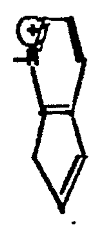 |
| $-CH_2CH_2-$ | 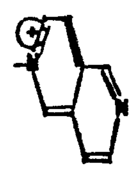 |

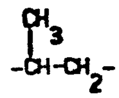 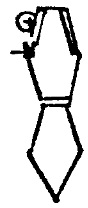

$-CH_2-$ 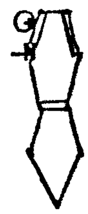

$-CH_2-$ 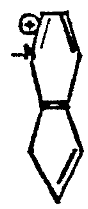

$-CH_2-$ 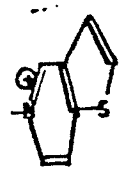

$-CH_2-$ 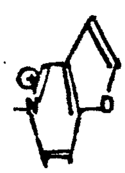

$-CH_2-$ 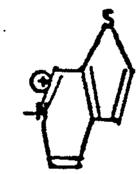

$-CH_2-$ 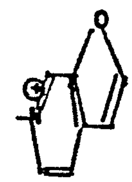

$-CH_2-$ 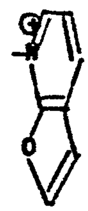

$-CH_2-$ 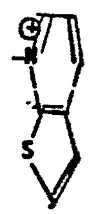

$-CH_2-$ 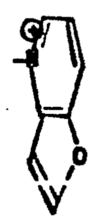

$-CH_2-$ 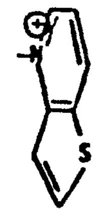

$-CH_2-$ 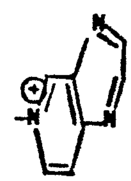

$-CH_2-$ 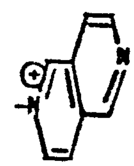

$-CH_2-$ 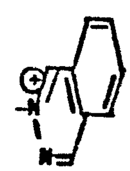

$-CH_2CH_2-$ 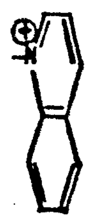

$-CH_2CH_2-$ 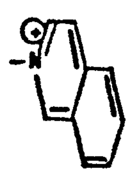

$-CH_2CH_2-$

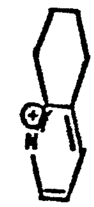

$-CH_2CH_2-$

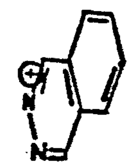

$-CH_2CH_2-$

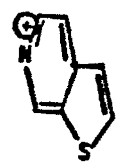

$-CH_2CH_2-$

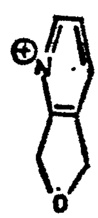

$-CH_2CH_2-$

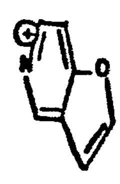

| | |
|---|---|
| $-CH_2CH_2-$ | 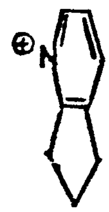 |
| $-CH_2CH_2-$ | 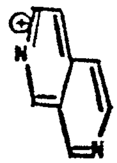 |
| $-CH_2CH_2-$ | 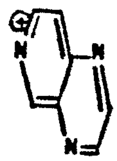 |
| $-CH_2CH_2-$ | 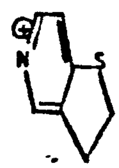 |
| $-CH_2CH_2-$ | 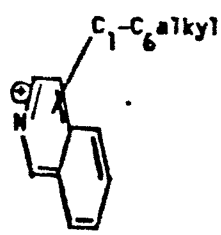 |
| $-CH_2CH_2-$ | 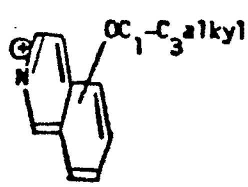 |

$-CH_2CH_2-$

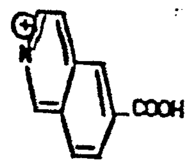

$-CH_2CH_2-$

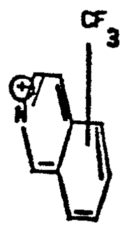

$-CH_2CH_2-$

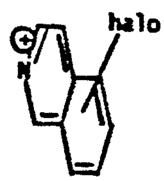

$-CH(CH_3)CH_2-$

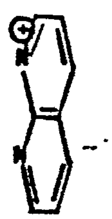

$-CH(CH_3)CH_2-$

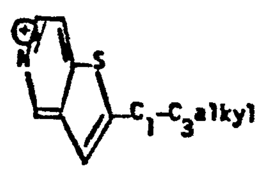

$-CH(CH_3)CH_2-$ 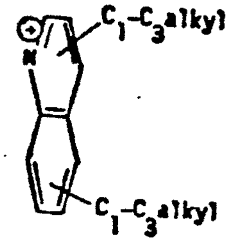

$-CH(CH_3)CH_2-$ 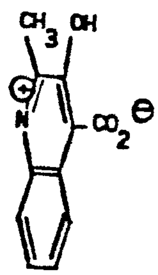

$-CH(CH_3)CH_2-$ 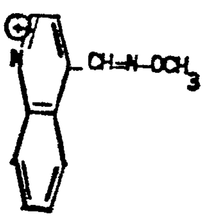

$-CH(CH_3)CH_2-$ 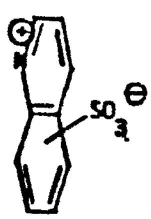

$-CH(CH_3)CH_2-$ 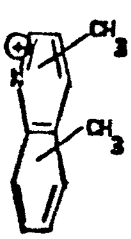

10. <u>Amidino and Amidinium</u>

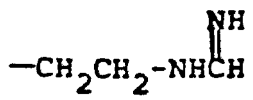

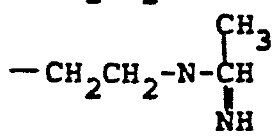

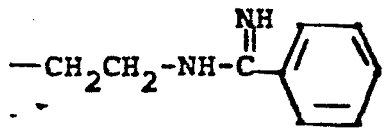

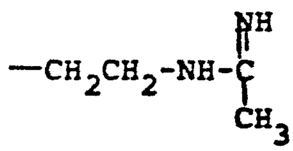

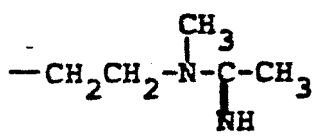

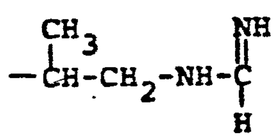

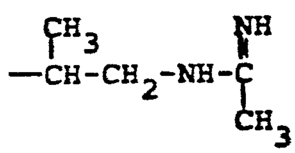

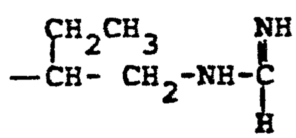

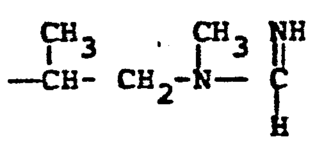

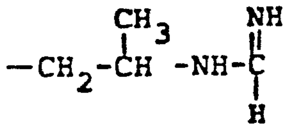

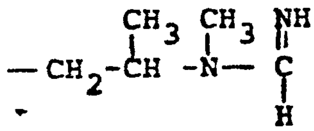

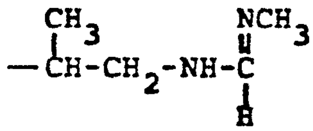

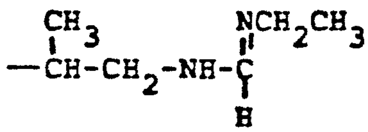

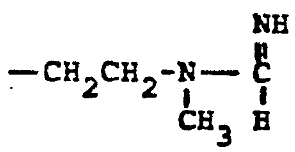

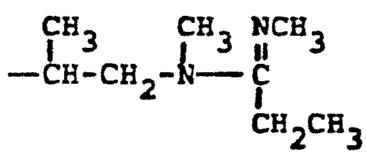

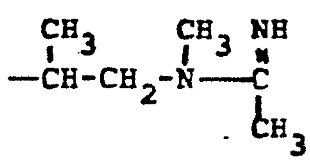

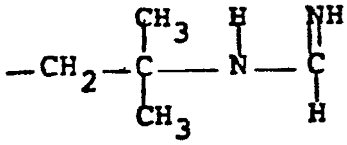

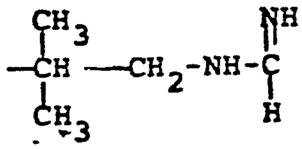

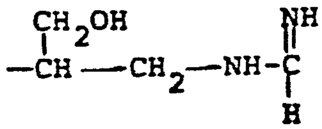

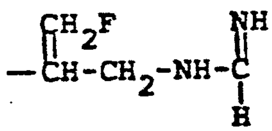

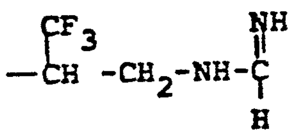

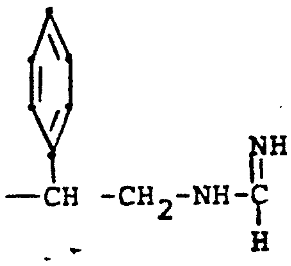

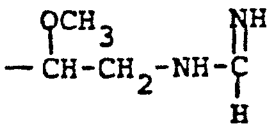

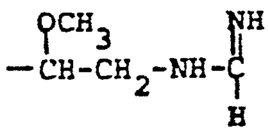

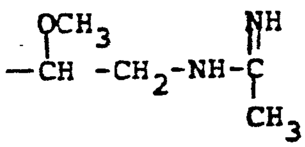

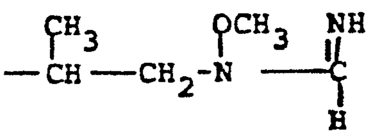

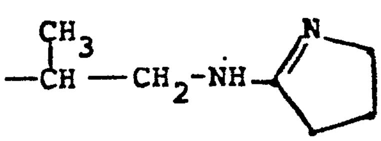

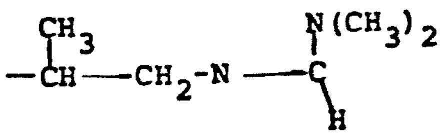

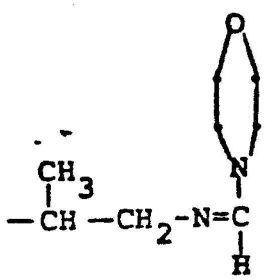

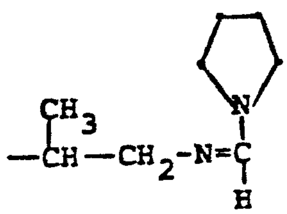

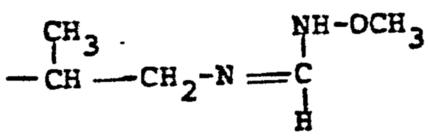

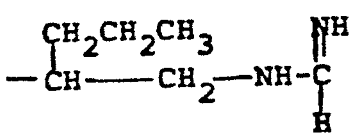

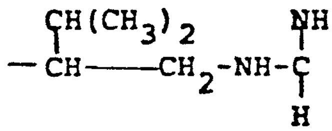

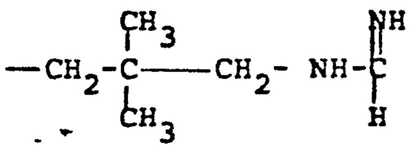

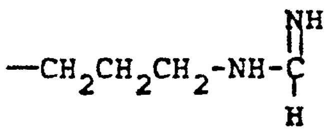

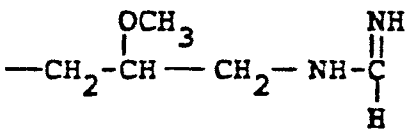

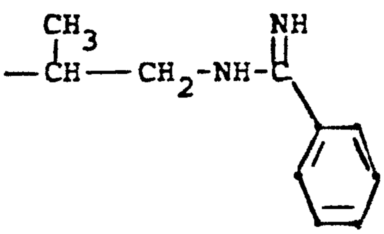

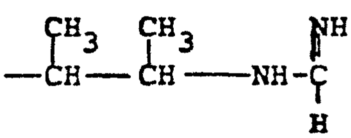

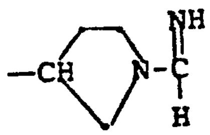

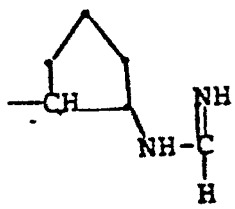

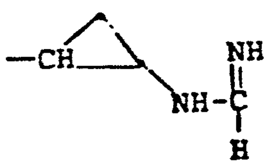

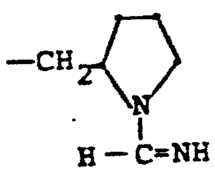

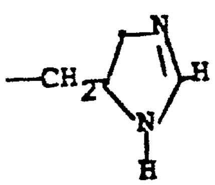

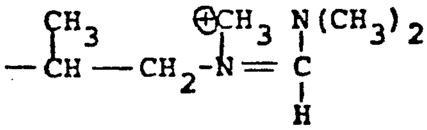

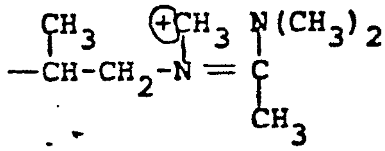

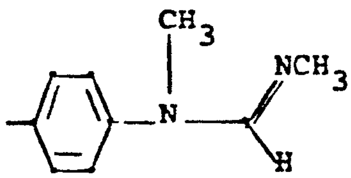

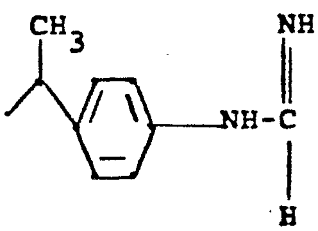

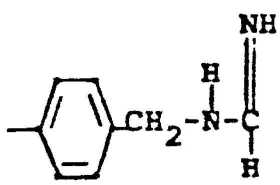

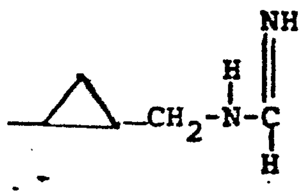

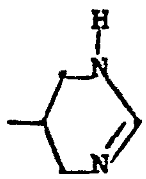

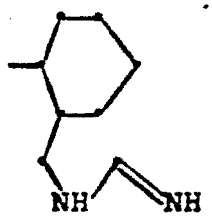

11. Guanidino and Guanidinium

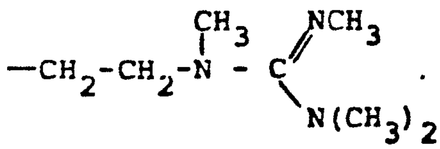

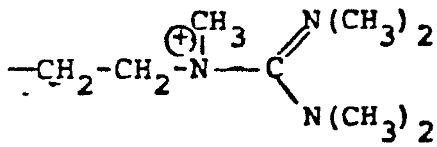

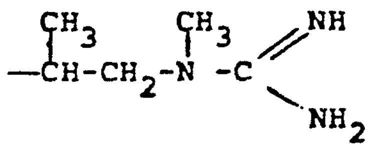

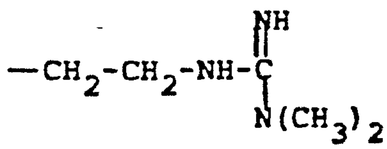

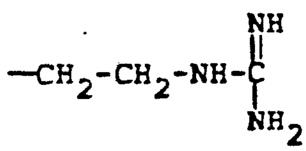

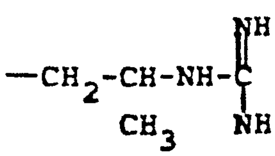

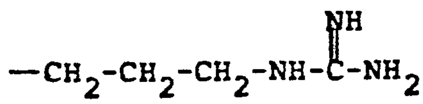

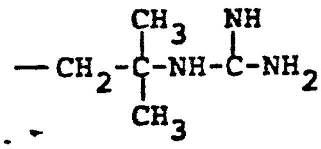

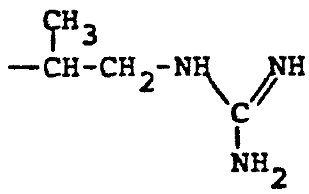

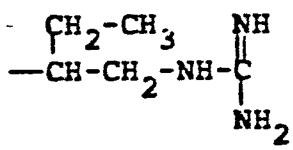

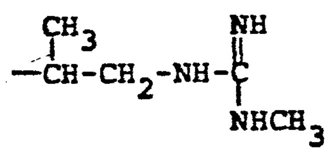

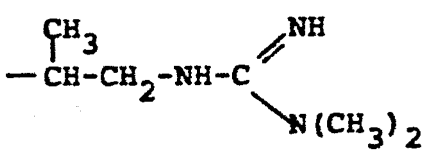

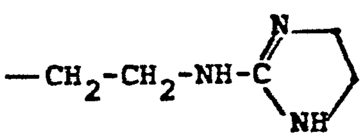

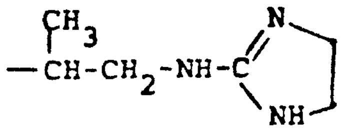

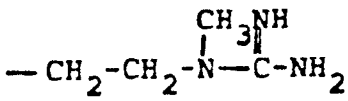

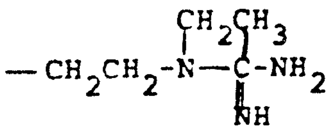

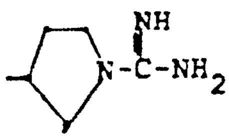

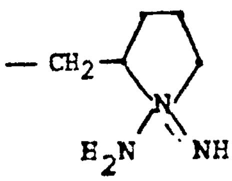

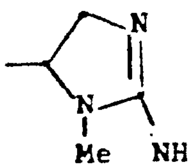

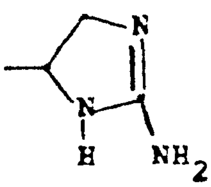

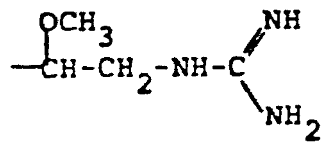

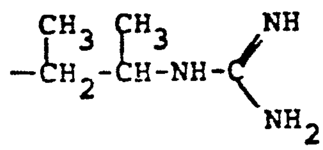

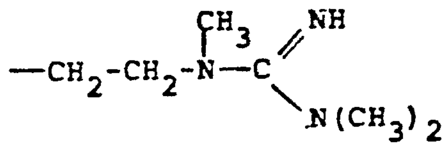

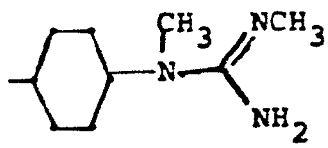

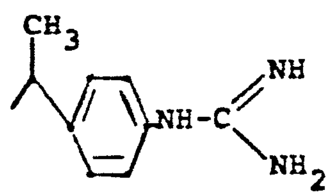

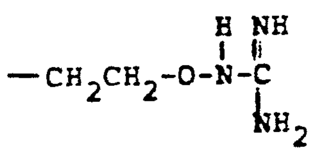

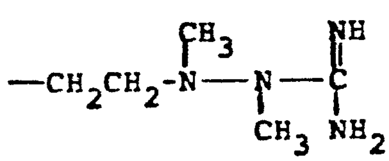

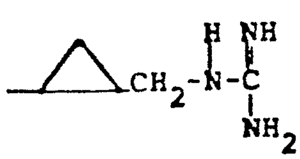

12. <u>Carbamimidoyl</u>

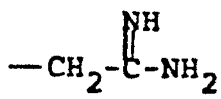

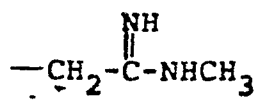

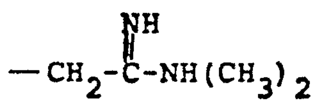

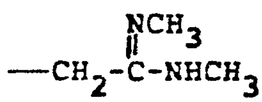

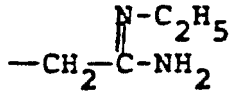

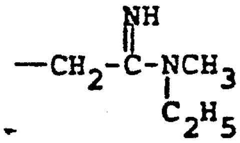

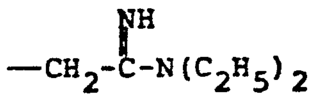

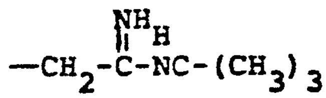

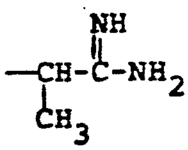

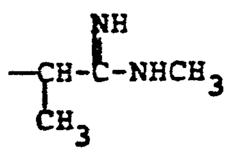

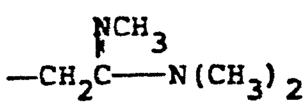

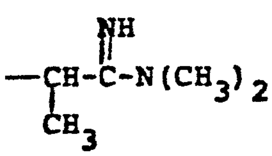

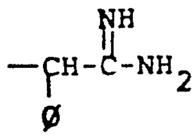

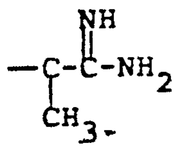

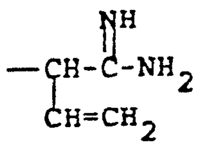

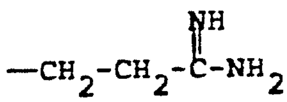

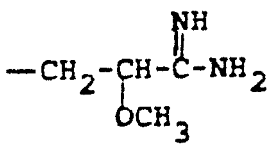

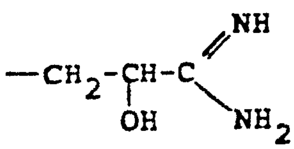

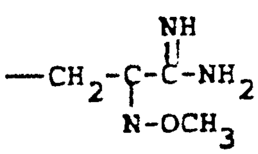

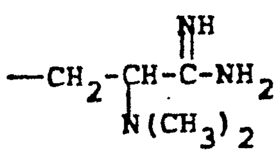

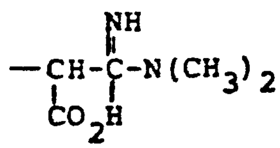

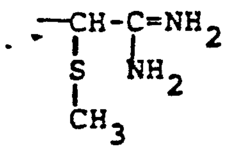

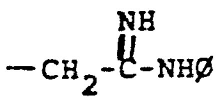

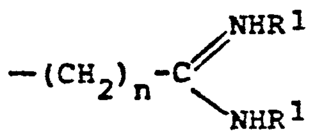

n = 2-5, $R^1$ = H, $CH_3$
$R^1$ = H, $CH_3$

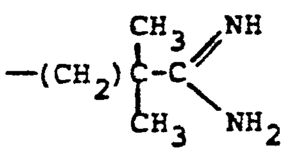

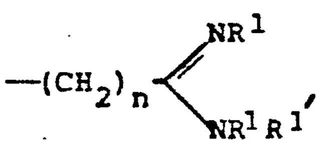

n = 2-5, $R^1$, $R^{1'}$ = H, $CH_3$

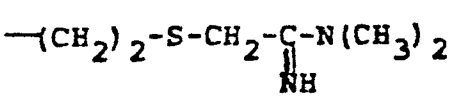

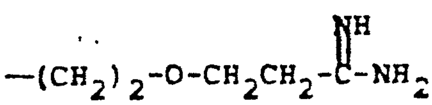

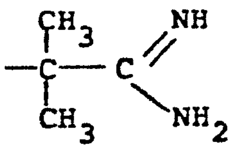

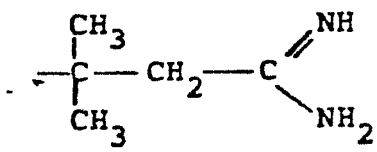

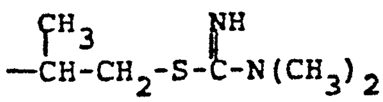

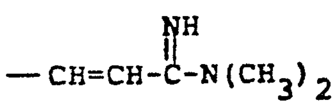

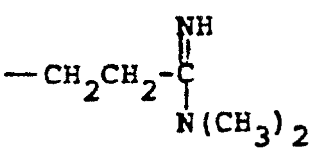

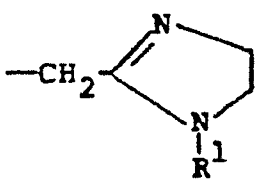

$R^1$ = H, $CH_3$

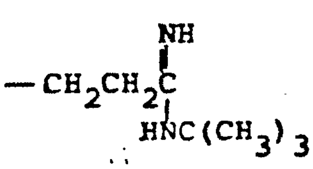

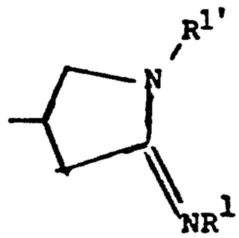

$R^1$ = H, $CH_3$
$R^{1'}$ = H, $CH_3$

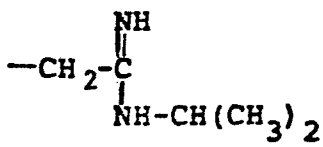

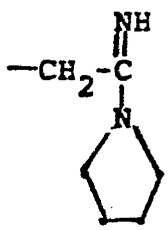

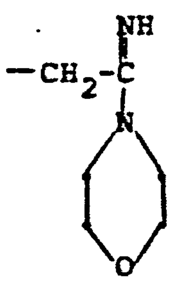

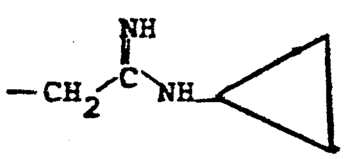

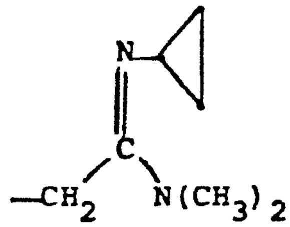

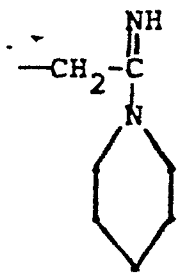

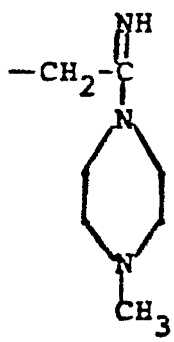

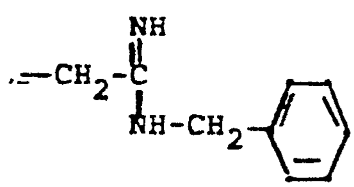

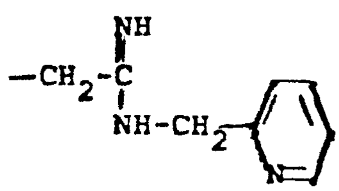

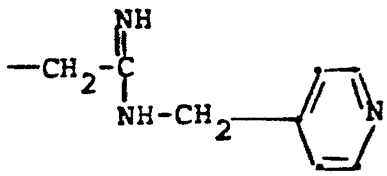

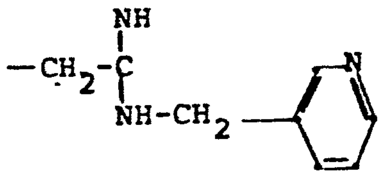

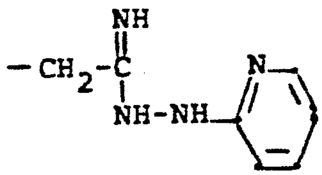

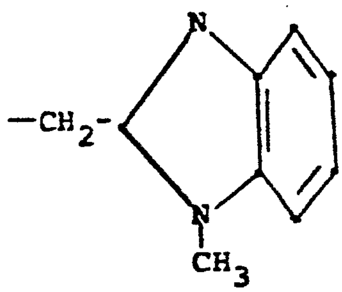

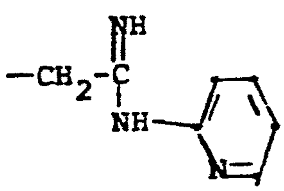

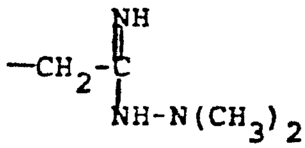

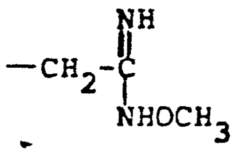

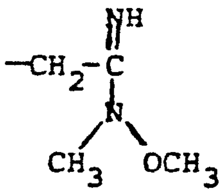

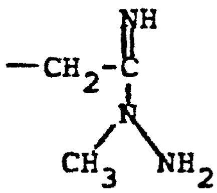

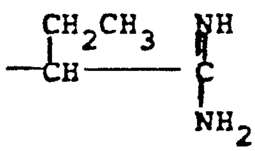

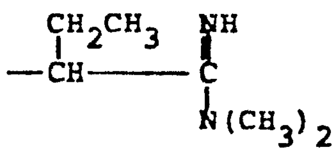

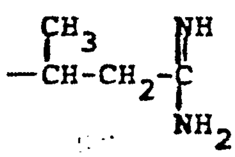

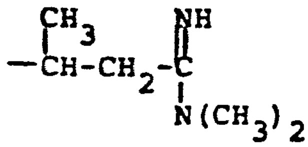

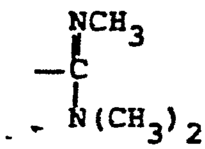

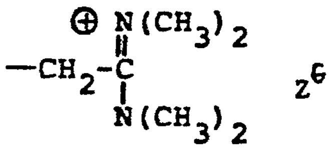

$Z^{\ominus}$ = any compatible anion

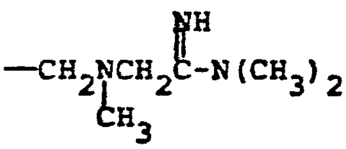

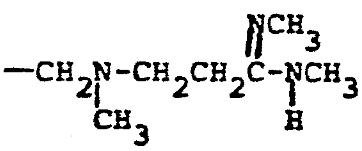

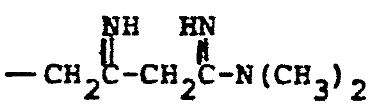

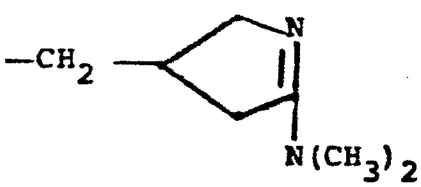

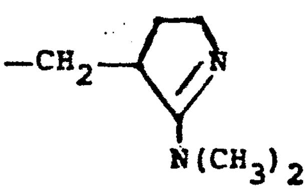

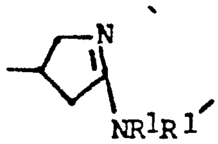

$R^1$ = H, $CH_3$
$R^{1'}$ = H, $CH_3$

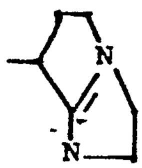

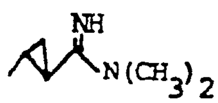

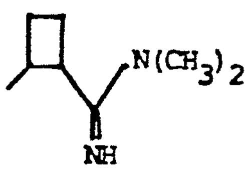

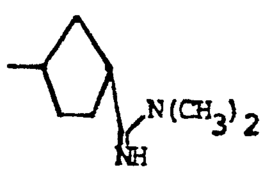

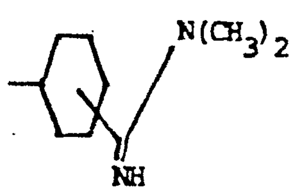

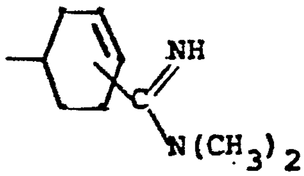

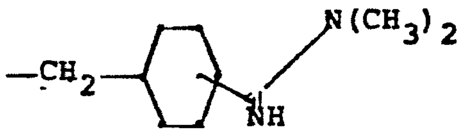

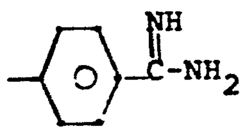

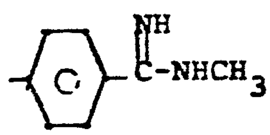

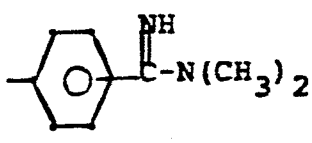

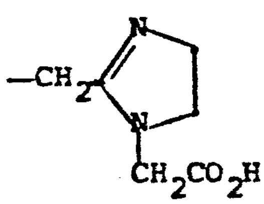

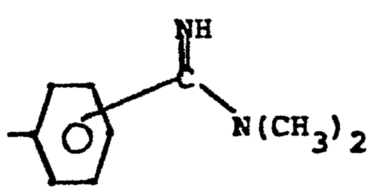

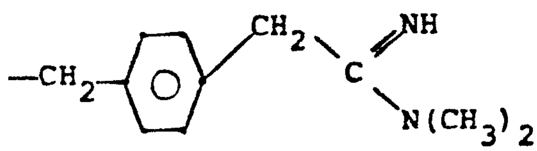

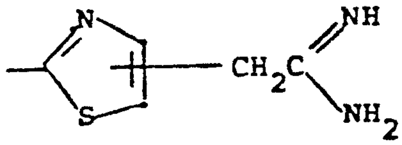

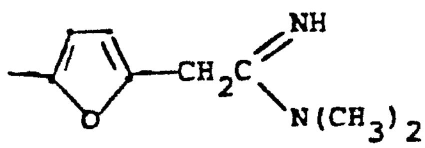

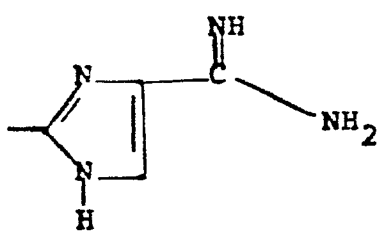

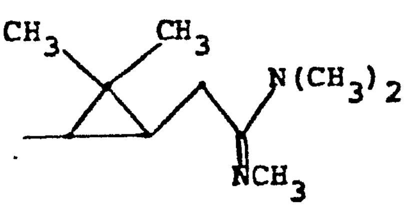

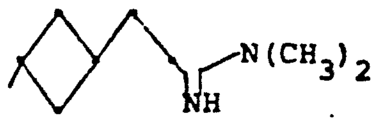

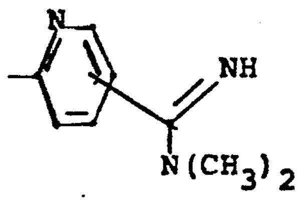

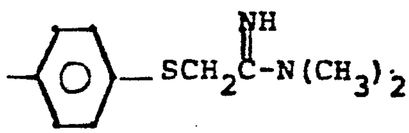

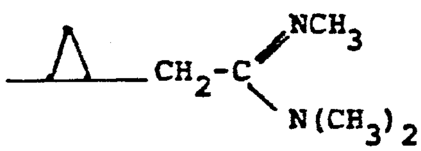

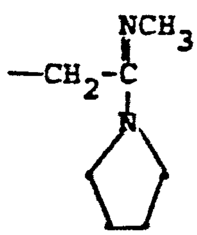

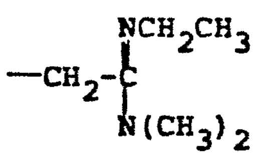

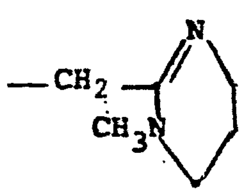

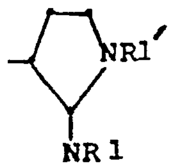

R1 = H, $CH_3$
R1′ = H, $CH_3$

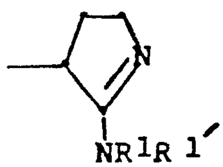

R1 = H, $CH_3$
R1′ = H, $CH_3$

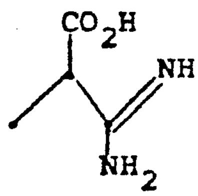

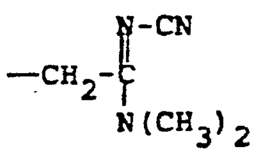

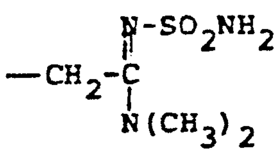

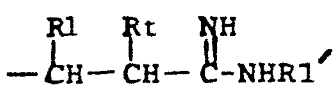

R1 = $CH_3$
Rt = $CH_3$, $N(CH_3)_2$, $OCH_3$
R1′ = H, $CH_3$

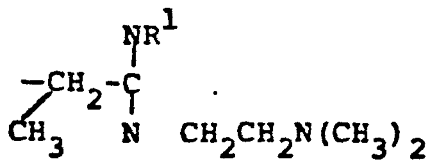

$R^1$ = H, $CH_3$

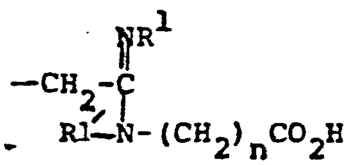

$R^1$ = H, $CH_3$

Rl' = H, $CH_3$

n = 1 or 2

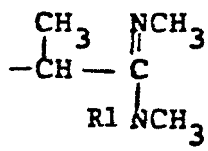

Rl = H, $CH_3$

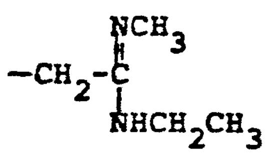

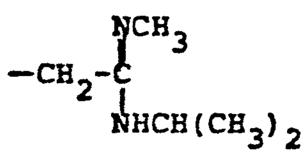

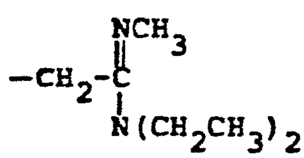

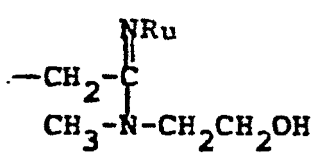

Ru = $CH_2CH_3$, $CH_3$, H

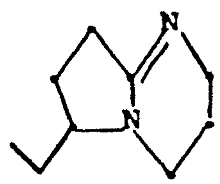

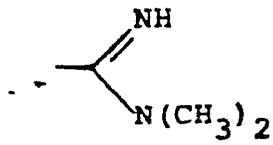

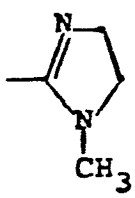

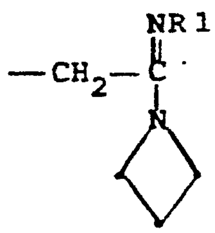

$R1 = H, CH_3$

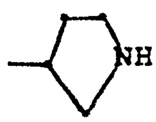

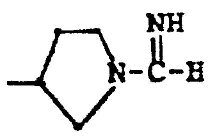

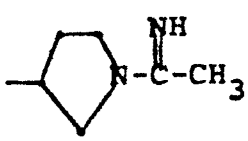

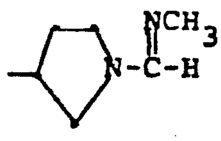

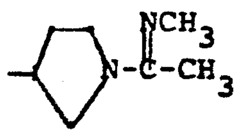

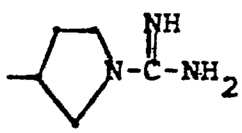

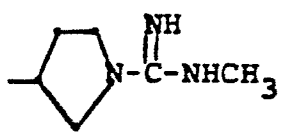

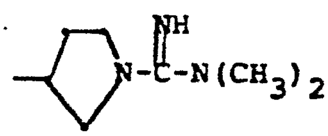

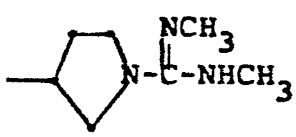

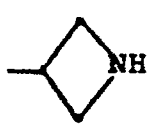

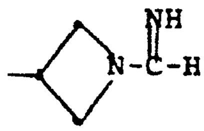

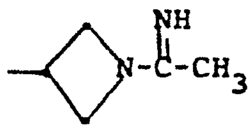

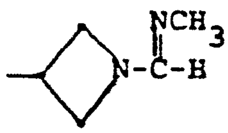

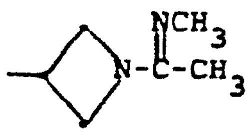

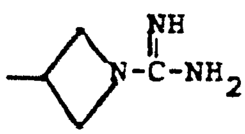

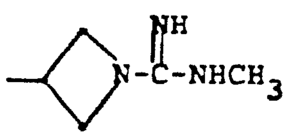

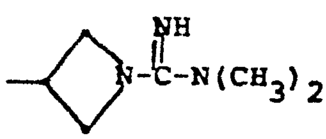

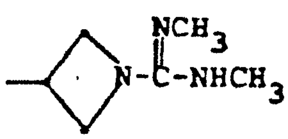

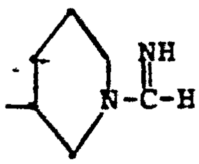

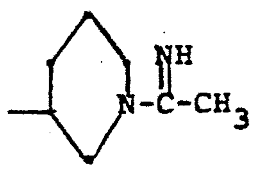

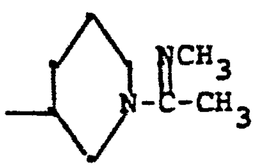

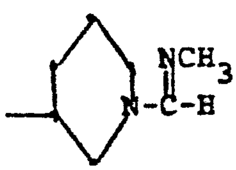

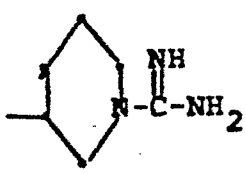

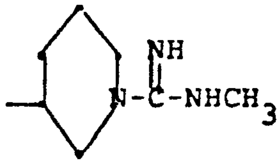

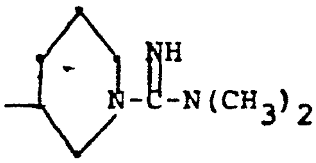

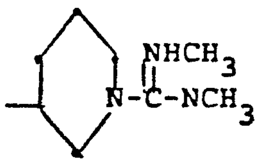

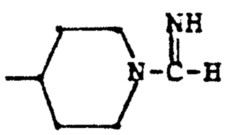

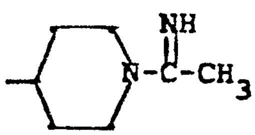

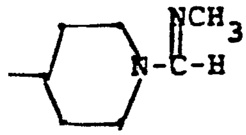

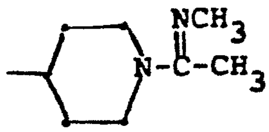

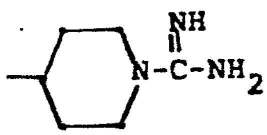

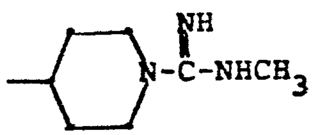

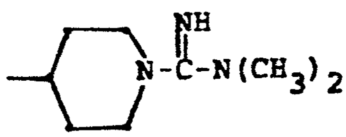

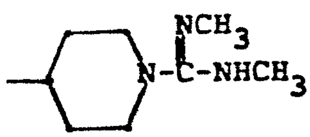

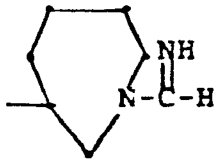

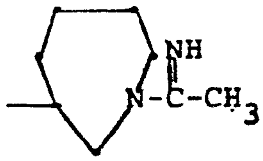

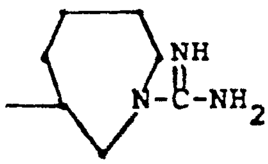

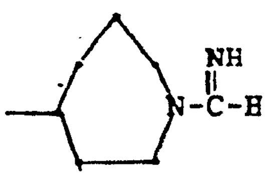

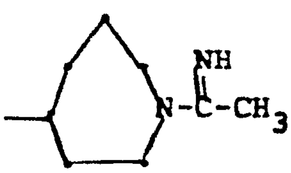

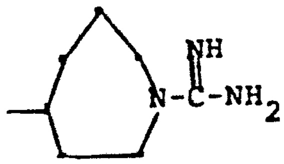

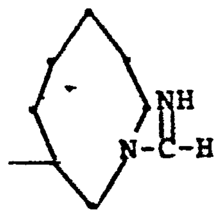

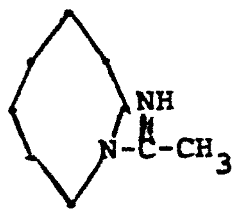

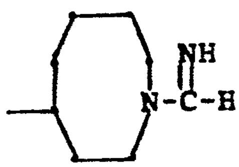

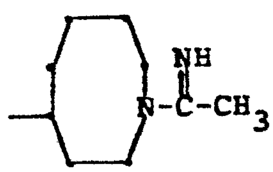

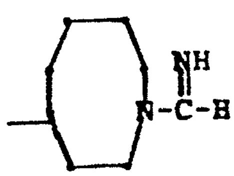

The compounds of Formula I include inner (Zwitterion) salts when W is $COO^-$ e.g.

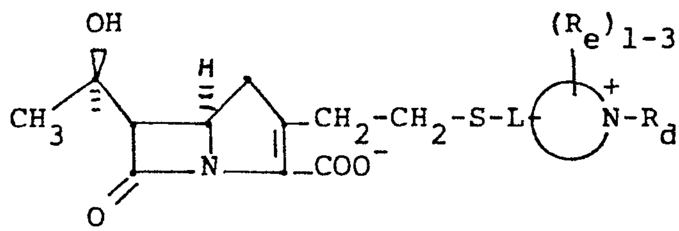

I

or, when W is other than $COO^-$, salts with an external, physiologically acceptable counterion $Z^-$ such as $Cl^-$, $Br^-$, $I^-$, $OCH_3^-$, $OSO_2CF_3^-$, $ØP(O)(O\ phenyl)_2^-$ and the like.

The inner salts are preferred.

Again, the compounds of Formula I include the stereoisomers as mixtures and as separate isomers.

The compounds of the present invention (I) are valuable antibiotics active against various Gram-positive and Gram-negative bacteria and accordingly find utility in human and veterinary medicine. Representative pathogens which are sensitive to antibiotics I include: Staphylococcus aureus, Escherichia coli, Klebsiella Pneumoniae, Bacillus subtilis, Salmonella typhosa, Pseudomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of

antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The compounds of this invention may be used in any of a variety of pharmaceutical preparations. They may be employed in capsule, powder form, in liquid solution, or in suspension. They may be administered by a variety of means; those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic

art. In general, a daily dosage consists of from about 5 to about 600 mg of active ingredient per kg of body weight of the subject in one or more treatments per day. A preferred daily dosage for adult humans lies in the range of from about 10 to 240 mg of active ingredient per kg of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg to about 1500 mg of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution.

The preferred method of administration of the formula I antibiotic is parenteral by i.v. infusion, i.v. bolus, or i.m. injection.

For adults, 5-50 mg of Formula I antibiotic per kg of body weight given 2, 3, or 4 times per day is preferred. Preferred dosage is 250 mg to 1000 mg of the Formula I antibiotic given two (b.i.d.) three (t.i.d.) or four (q.i.d.) times per day. More specifically, for mild infections, and particularly urinary tract infections, a dose of 250 mg t.i.d. or q.i.d. is recommended. For moderate infections

against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibiotic compounds of Formula I are of the broad class known as carbapenems or 1-carbade-thiapenems. Certain of these carbapenems are susceptible to attack by a renal enzyme known as dehydropeptidase (DHP). This attack or degradation may reduce the efficacy of the carbapenem antibiotic. Inhibitors of DHP and their use with carbapenem antibiotics are disclosed in the prior art [see published European Patent Applications No. 79102616.4 filed July 24, 1979 (Patent Number 10573); 79102615.6, filed July 24, 1979 (application no. 15573); and No. 82107174.3, filed August 9, 1980 (application no. 72014)].

The present I compounds may, where DHP inhibition is desired or necessary, be combined or used with the appropriate DHP inhibitor as described in the aforesaid published applications. Thus, to the extent that the cited European patent applications 1.) define the procedure for determining DHP susceptibility on the present carbapenems and 2.) disclose suitable inhibitors, combination compositions and methods of treatment, they are incorporated herein by reference. A preferred weight ratio of I

compound:DHP inhibitor in the combination compositions is about 1:1. A preferred DHP inhibitor is 7-(L-2-amino-2-carboxyethylthio)-2-(2,2-dimethylcyclopropanecarboxamide)-2-heptenoic acid or a useful salt thereof.

These combination compositions and their use is another embodiment of the present invention.

The compounds of Formula I may be prepared by any convenient process.

Preparation of Compounds of Formula I

Due to the varied nature of $R^4$ and $R^5$, several different synthetic approaches are used to prepare compounds of type I. These are summarized below. Implicit in these synthetic routes is the fact that when $R^5$ contains a functional group which might interfere with the intended course of the reaction, the offending group is blocked. For example, when a basic nitrogen group is present as $-NHR$ or $-NH_2$, it is usually protected by acylation ($-CO_2$-allyl or $-CO_2PNB$) or silylation. In the case of a carboxyl group, protection is achieved *via* use of a suitable ester, such as allyl or PNB.

In many instances, the actual order of carrying out the individual synthetic transformations in a synthetic scheme can be permuted for reasons of synthetic expediency. Thus, many of the examples listed below could proceed with equal facility by having the order of operations changed.

## Synthetic Methodology

A. Key Intermediate II:

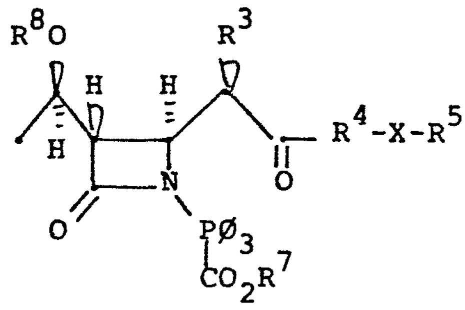

II

X, $R^3$, $R^4$ and $R^5$ are as defined above;

$R^7$ is a suitable ester protecting group such as p-nitrobenzyl, allyl, or the like;

$R^8$ is a suitable alcohol protecting group such as $(CH_3)_3Si-$, $t-BuMe_2Si$, p-nitrobenzyloxycarbonyl, allyloxycarbonyl, or the like.

Method IIa

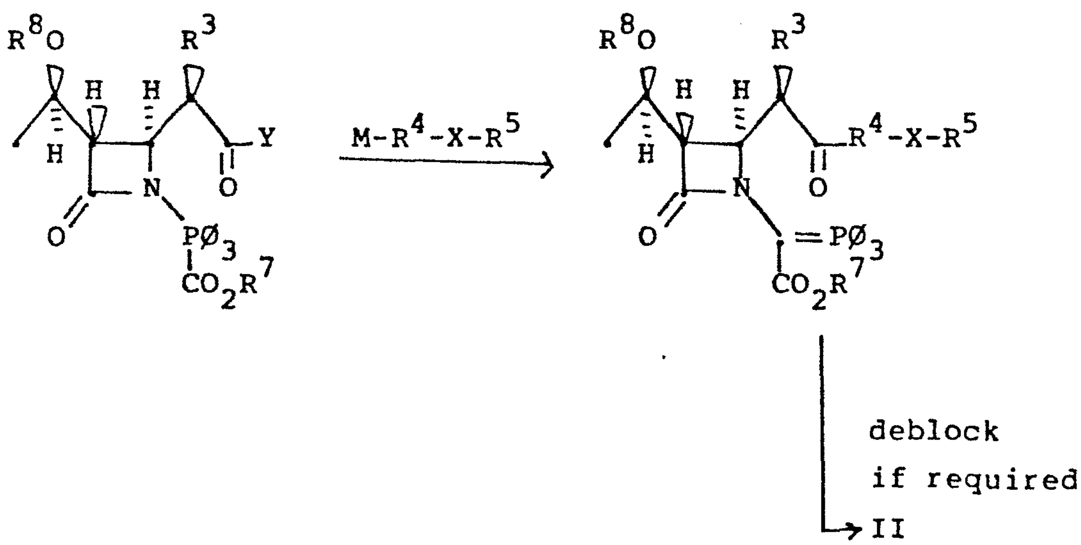

Y = 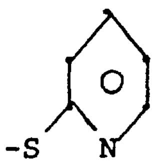 , 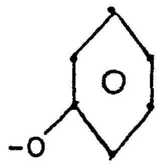 , 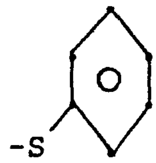 , -Cl, -Br, 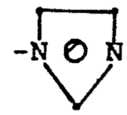 ,

or the like;

$R^5$, $R^3$, $R^7$, and $R^8$ are as defined above;

$R^4$ = $-\underset{CH_3}{CH}-$, $-CH_2CH_2-CH_2$, $-(CH_2)_4-$, $-\overset{CH_3}{C}-CH_2CH_2$, $-CH_2-\overset{CH_3}{CH}-CH_2-$;

M = Li, MgX, Cu or the like.

Method IIc

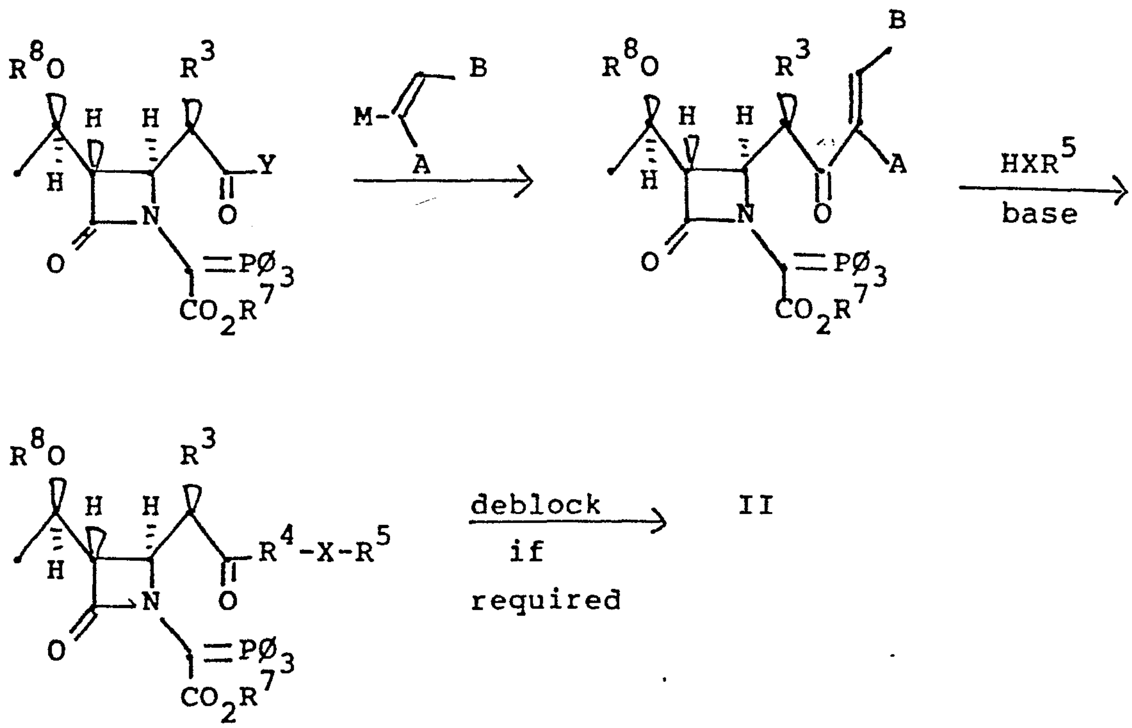


X, M, Y, $R^3$, and $R^5$ are as defined above;

$R^8$ = triorganosilyl, allyloxycarbonyl or the like;

$R^7$ = allyl, benzyl or the like;

A, B = H or $CH_3$ (both not $CH_3$);

$R^4$ = $-CH_2CH_2-$, $-CH(CH_3)-CH_2-$, $-CH_2-CH(CH_3)-$;

base = $Et_3N$, $iPr_2NEt$, NaH, n-BuLi, $LiNiPr_2$ or the like.

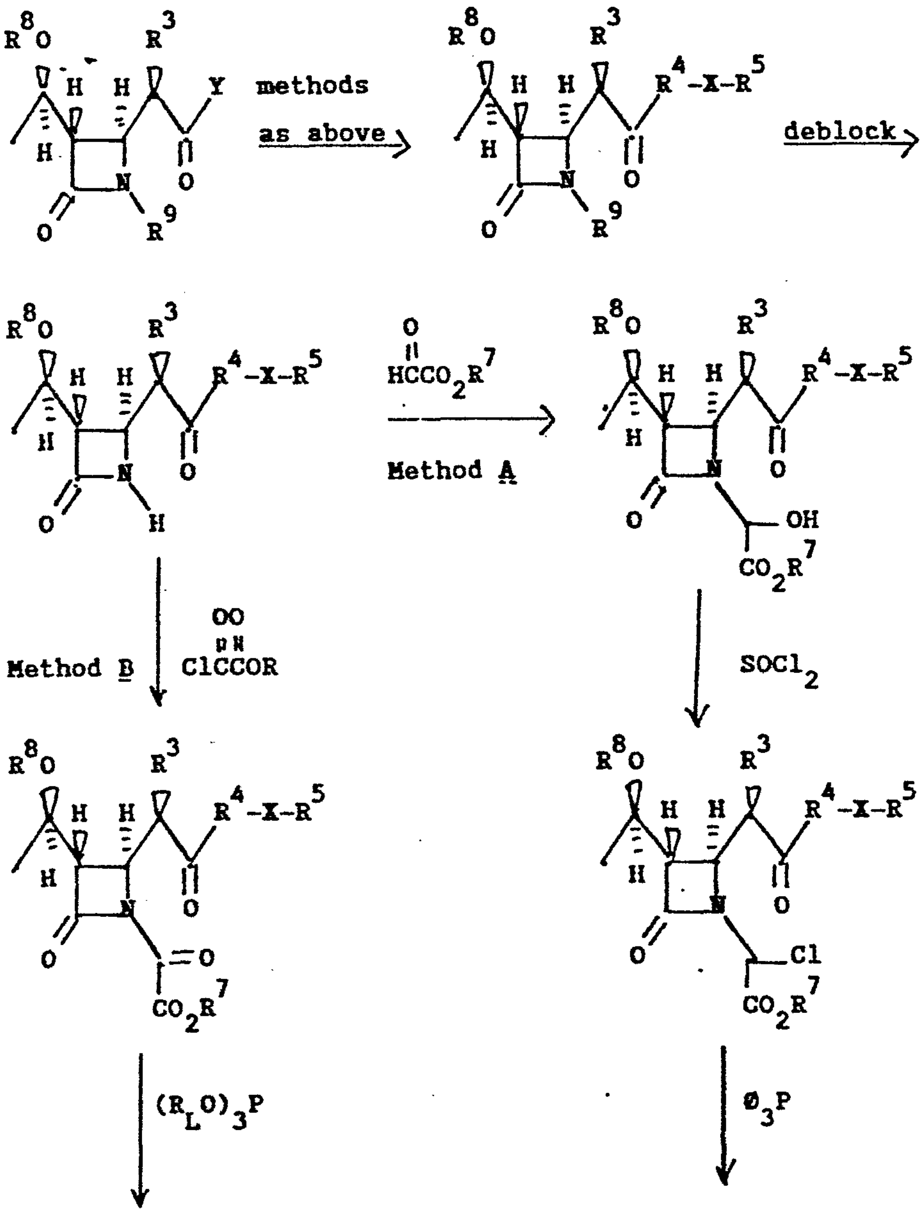

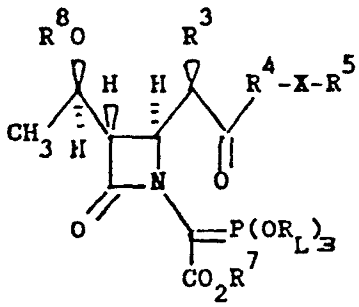

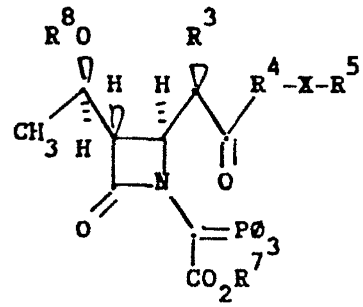

$L = CH_3$, $CH_2CH_3$, $-CH(CH_3)_2$, and the like.

One example of such a route is shown in Method IIf.

Method IIf

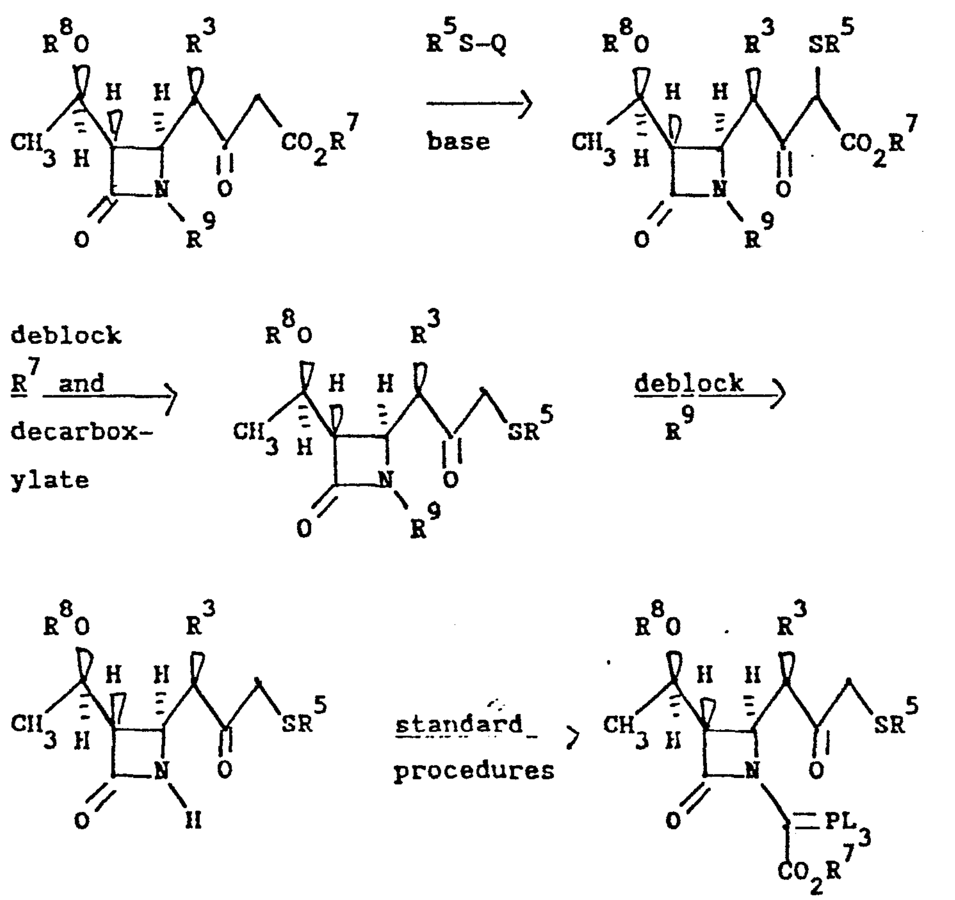


L=Ø, OEt, etc.

Method IIh

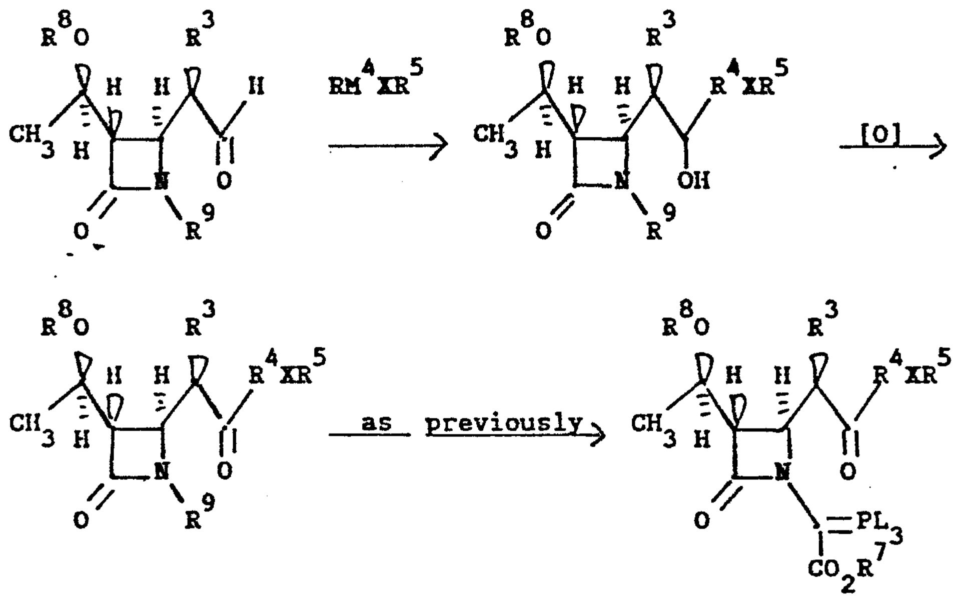


- all substituents as previously defined

Method IIi

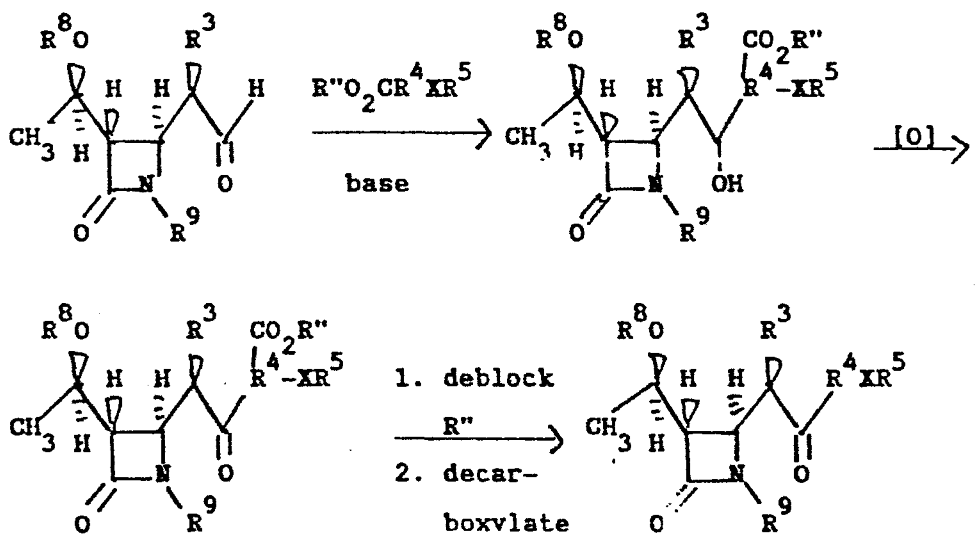

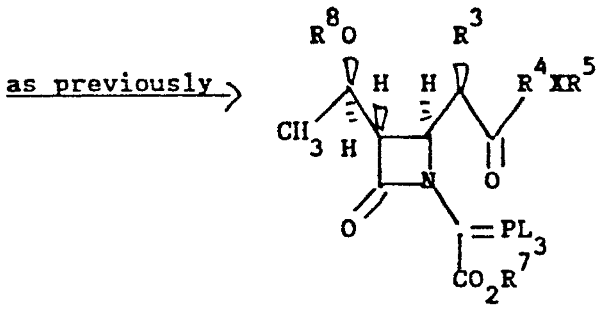


Completion of the Synthesis

from Intermediate II - Methods a-i

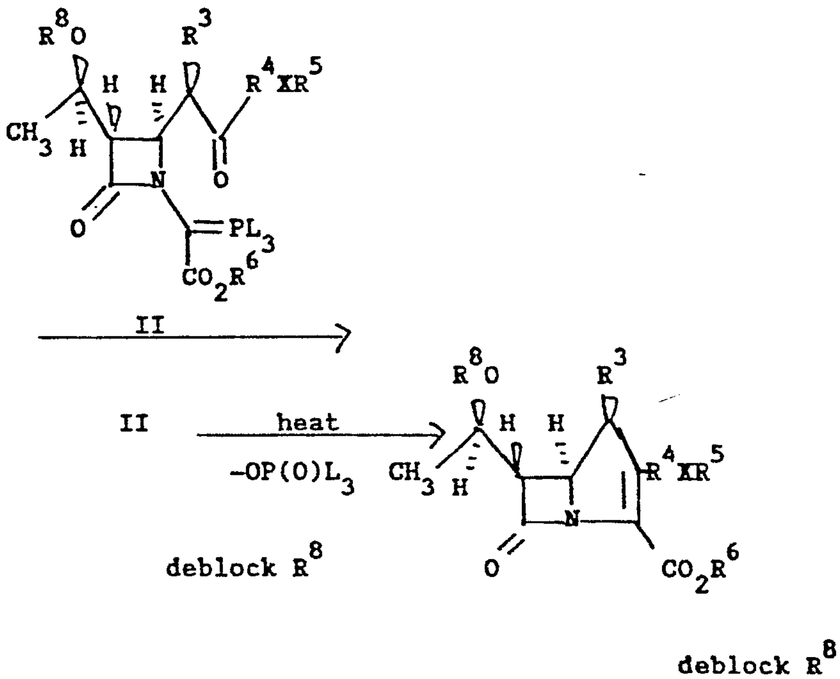

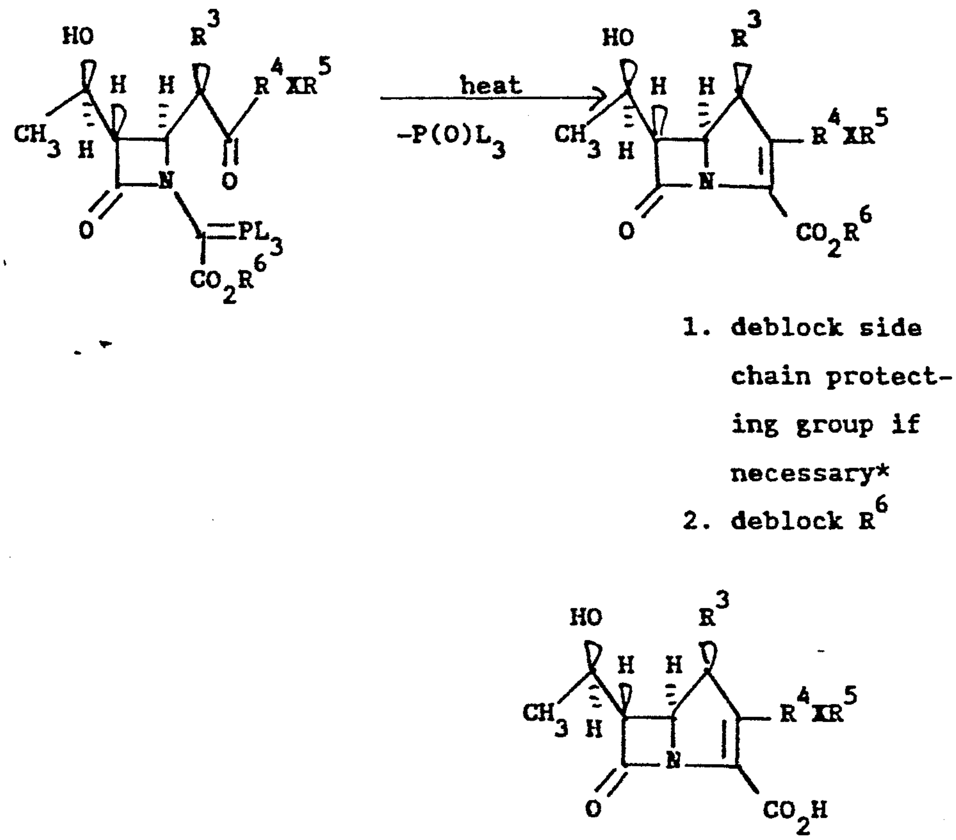


* In those cases where the final product will contain a quaternized heterocyclic group, an additional alkylation step would be added.

The following examples illustrate the preparation of compounds of Formula I. The temperature is in degrees Celsius unless otherwise indicated.

EXAMPLE 1

Preparation of:

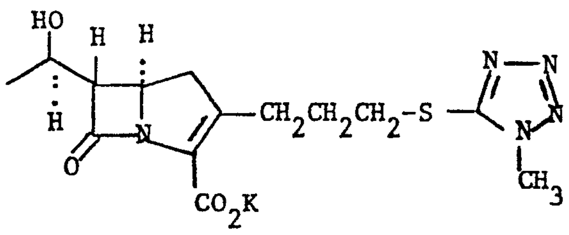

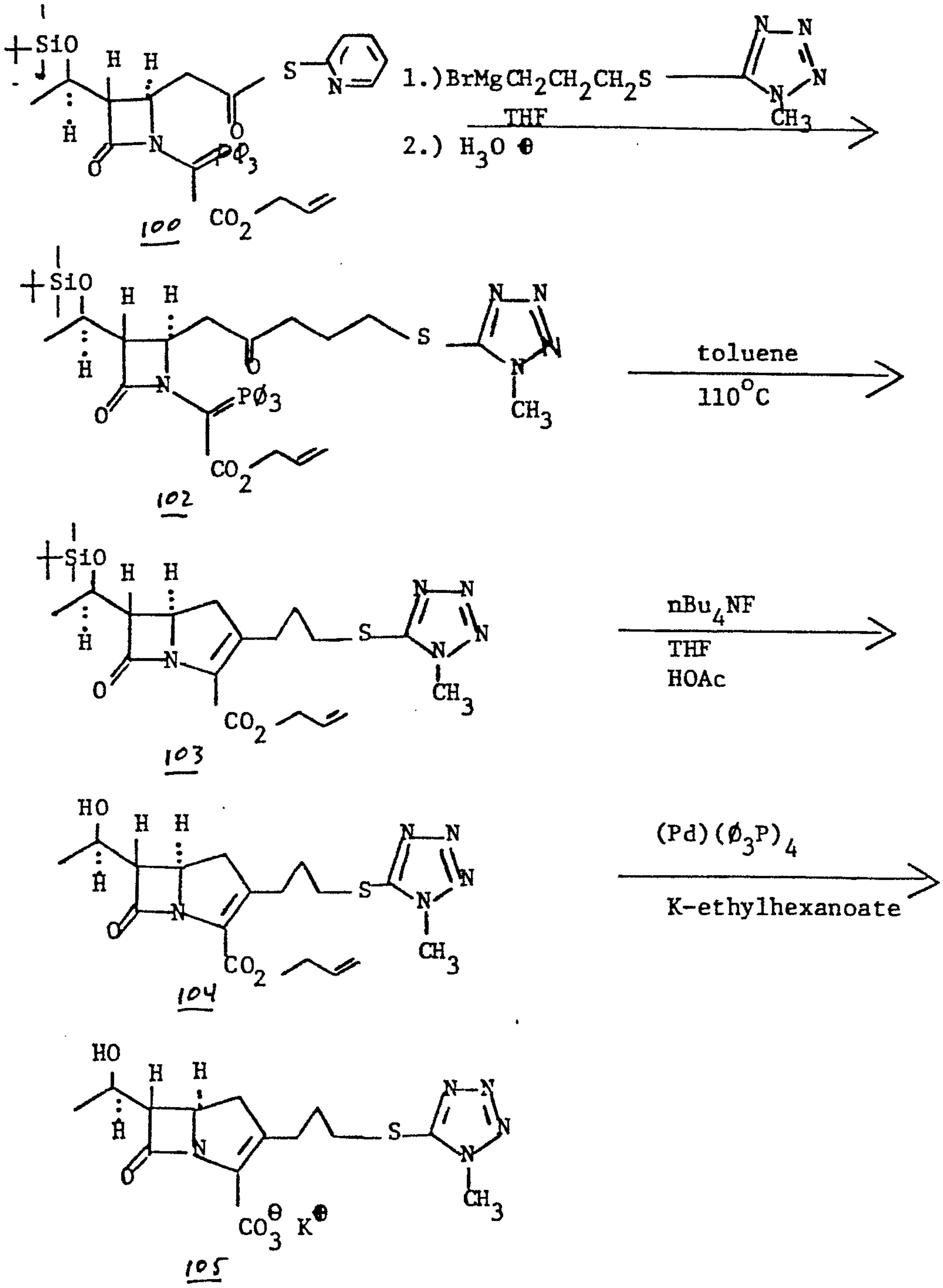


100, 102, 103, 104, 105

Preparation of (5R, 6S, 8R)-2-[[[1-methyl-1H-tetrazol-5-yl]thio]-propyl]-6-(1-hydroxyethyl)carbapen-2-em-3-carboxylic acid, potassium salt.

Step A

A solution of grignard reagent 101 in ether (2.0 mmol) is added slowly by syringe to a stirred solution of pyridylthio ester 100 (1.11 g, 1.50 mmol) in anhydrous tetrahydrofuran (25 ml) at -78°C under dry nitrogen. After 30 min. at -78°C the reaction is quenched by the rapid addition of saturated aqueous ammonium chloride solution (10 ml). The resulting mixture is poured into ethyl acetate (50 ml) and the phases are separated. The aqueous phase is extracted with an additional portion of ethyl acetate (15 ml) and the combined organic phases are washed with cold 1N hydrochloric acid solution (35 ml), cold 10% aqueous sodium bicarbonate solution (35 ml), and brine. After drying over anhydrous magnesium sulfate, the organic layer is concentrated in vacuo to yield a yellow gum. Chromatography on silica gel (eluting with ethyl acetate/methylene chloride) gives pure ketone 102.

Step B

A solution of ketone 102 (785 mg, 1.0 mmol) in deoxygenated toluene is heated at reflux for 16 hr. The resulting solution is cooled and concentrated in vacuo to give an oil. Preparative thin layer chromatography on silica gel (eluting with ethyl acetate/methylene chloride) gives carbapenem 103.

Step C

A solution of carbapenem 103 (253 mg, 0.50 mmol), tetrabutylammonium fluoride (2.5 ml of 1N solution in THF, 2.5 mmol), the glacial acetic acid (450 mg, 7.5 mmol) in anhydrous tetrahydrofuran is stirred at ambient temperature for 28 hr. The reaction mixture is then poured into 0.5M pH 7 phosphate buffer (50 ml), water (50 ml) and ethyl acetate (100 ml). The layers are separated and the aqueous layer is extracted with an additional portion of ethyl acetate (25 ml). The combined organic layers are washed with brine then dried over anhydrous magnesium sulfate. The dried solution is concentrated in vacuo and purified by preparative thin layer chromatography (eluting with ethyl acetate) to provide alcohol 104.

Step D

Triphenyl phosphine (1.8 mg, 0.007 mmol), tetrakis(triphenyl phosphine) palladium (0.3 mg, 0.00026 mmol) and 0.5M potassium 2-ethylhexanoate in ethyl acetate (220 microl, 0.11 mmol) are added to a stirred solution of allyl ester 104 (39 mg, 0.10 mmol) in 1:1 ethyl acetate-methylene chloride (__ ml) at ambient temperature under dry nitrogen. After 45 min. of stirring at room temperature the reaction mixture is concentrated in vacuo and the resulting solid is washed with anhydrous ether (3x3 ml) to yield an off-white solid. This material is dissolved in a minimum amount of water and is chromatographed in the cold room on two 500 RPS-F plates (Analtech) using ethanol in water as the eluting system. The major UV active band is extracted with 4:1 acetonitrile:water. The resulting solution is

concentrated to about __ ml then is washed four times with hexane. The aqueous layer is then lyophilized to give (5R, 6S, 8R)-2-[[[1-methyl-1H-tetrazol-5-yl]thio]propyl]-6-(1-hydroxyethyl)-carbapen-2-em-3-carboxylic acid, potassium salt.

EXAMPLE 2

Preparation of:

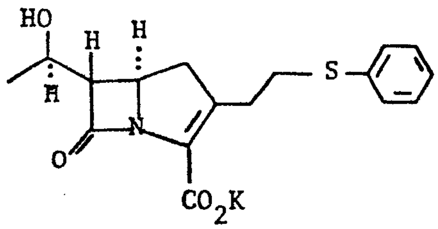

100

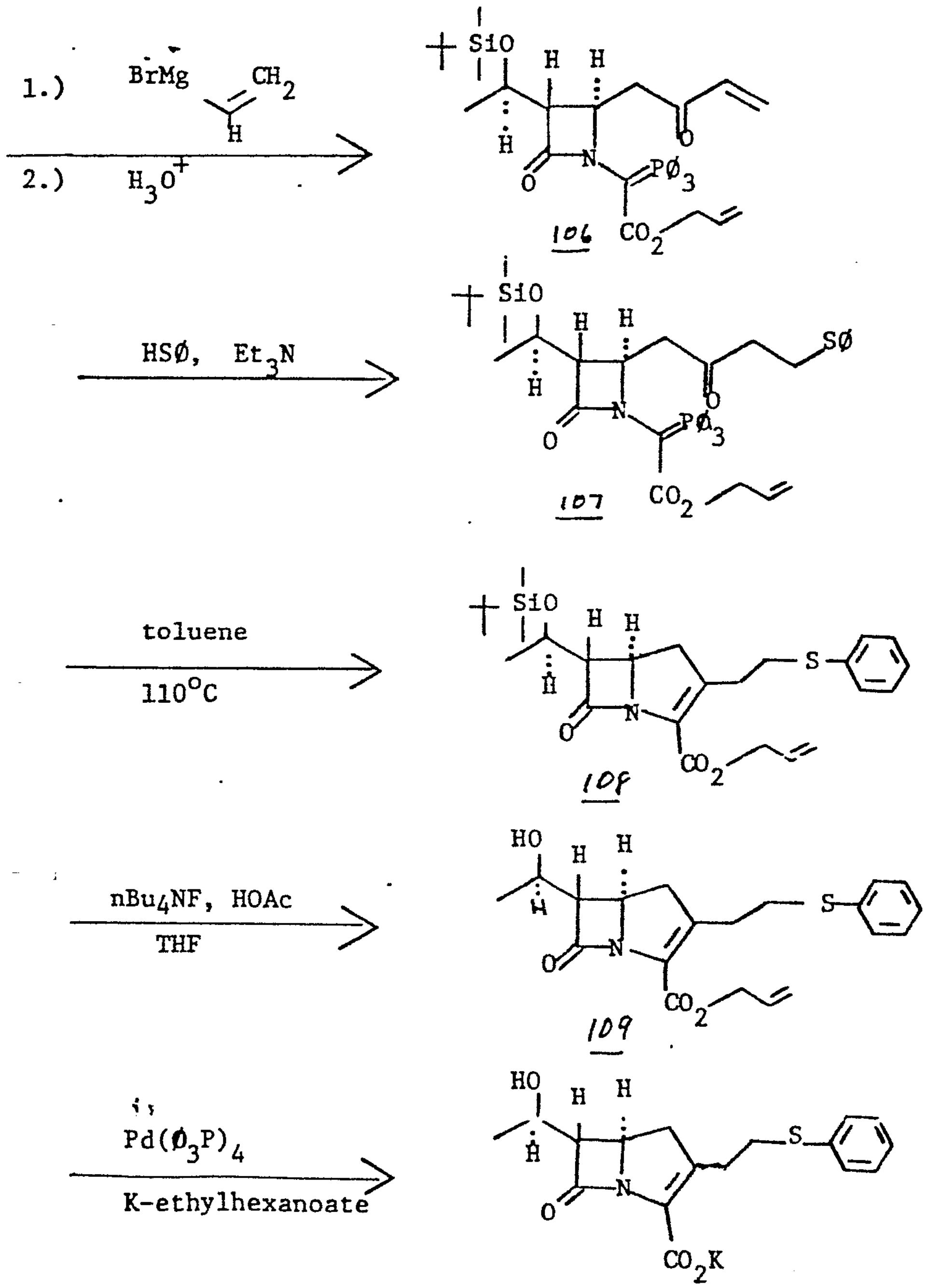

Preparation of (5R, 6S, 8R)-2-[[2-phenylthio]ethyl)-6-(1-hydroxyethyl)carbapen-2-em-3-carboxylic acid, potassium salt.

Step A

A solution of vinyl magnesium bromide in ether (2.0 mmol) is added slowly by syringe to a stirred solution of pyridylthio ester 100 (1.11 g, 1.50 mmol) in anhydrous tetrahydrofuran (25 ml) at -78°C under at atmosphere of dry nitrogen. After 30 min. at -78°C the reaction is quenched by the rapid addition of saturated aqueous amonium chloride solution (25 ml). The resulting mixture is poured into ethyl acetate (50 ml) and the phases are separated. The aqueous phase is extracted with an additional portion of ethyl acetate (15 ml) and the combined organic phases are washed with cold 1N hydrochloric acid solution (35 ml), cold 10% aqueous sodium bicarbonate solution (35 ml) and brine. After drying over anhydrous magnesium sulfate, the organic layer is concentrated in vacuo to yield a yellow gum. Chromatography on silica gel (eluting with ethyl acetate/methylene chloride) gives pure enone 106.

Step B

A solution of enone 106 (327 mg, 0.5 mmol) in dry dimethylformamide (5 ml) at 0°C is treated successively with thiophenol (55 mg, 0.50 mmol) and triethyl amine (10 mg, 0.10 mmol). The resulting solution is stirred under dry nitrogen, for 1 hr, then concentrated under high vacuum. The residue is dissolved in ethyl acetate (20 ml) and the resulting solution is washed with waterr (3x10 ml) and brine.

The organic phase is dried over anhydrous magnesium sulfate and concentrated in vacuo to given an oil. Chromatography on silica gel (eluting with ethyl acetate/methylene chloride) provides more ketone 107.

Step C

A solution of ketone 107 (306 mg, 0.4 mmol) in deoxygenated toluene (10 ml) is heated at reflux for 14 hr. The resulting solution is then cooled and concentrated in vacuo to give an oil. Preparative thin layer chromatography on silica gel (eluting with ethyl acetate/methylene chloride) gives carbapenem 108.

Step D

A solution of carbapenem 108 (97 mg, 0.2 mmol), tetrabutyl ammonium fluoride (1.0 ml of 1N solution in tetrahydrofuran, 1.0 mmol), and glacial acetic acid (180 mg, 3.0 mmol) in anhydrous tetrahydrofuran (5 ml) is stirred at room temperature for 29 hr. The reaction mixture is then poured into 0.5M pH 7 phosphate buffer (20 ml), water (20 ml) and ethyl acetate (40 ml). The layers are separated and the aqueous layer is extracted with an additional portion of ethyl acetate (10 ml). The combined organic layers are washed with brine then dried over anhydrous magnesium sulfate. The dried solution is concentrated in vacuo and purified by preparative thin layer chromatography (eluting with ethyl acetate) to provide alcohol 109.

Step E

Triphenyl phosphine (1.8 mg, 0.007 mmol), tetrakis(triphenyl phosphine) palladium (0.3 mg, 0.00026 mmol) and 0.5M potassium 2-ethyl hexanoate in ethyl acetate (0.22 ml, 0.11 mmol) are added to a stirred solution of allyl ester 109 (37 mg, 0.1 mmol) in 1:1 ethyl acetate-methylene chloride (__ ml) under dry nitrogen at room temperature. After 30 min., of stirring at room temperature, the reaction mixture is concentrated in vacuo and the resulting residue is washed with ether (3x3 ml) to yield a solid. This material is dissolved in a minimum amount of water and is chromatographed at 0°C on two 500 RPS-F plates (Analtech) using ethanol/water as the eluting system. The major UV visible band is extracted with 4:1 acetonitrile-water. The resulting solution is concentrated to about 10 ml, then is washed four times with hexane. The aqueous layer is lyophilized to give (5R, 6S, 8R)-2-]]2-phenylthio]-ethyl)-6-(1-hydroxyethyl)carbapen-2-em-3-carboxylic acid, potassium salt.

EXAMPLE 3

Preparation of:

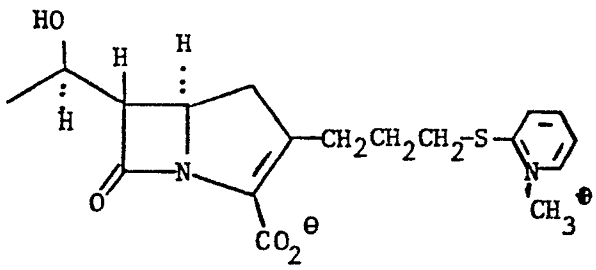

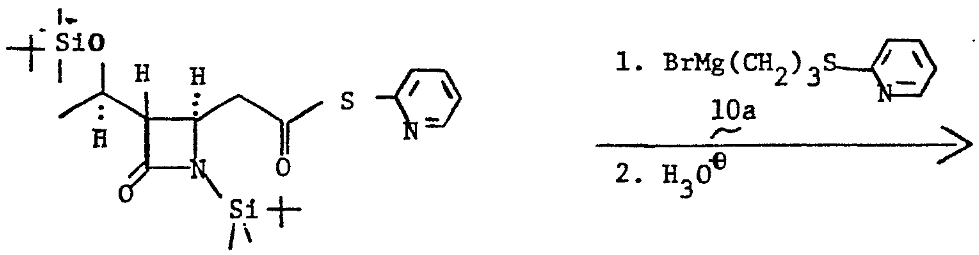


110

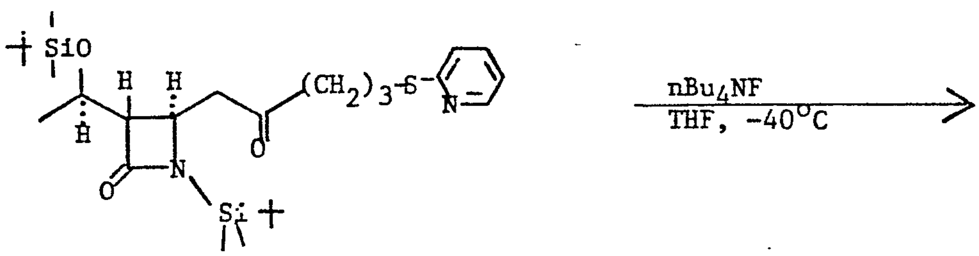


111

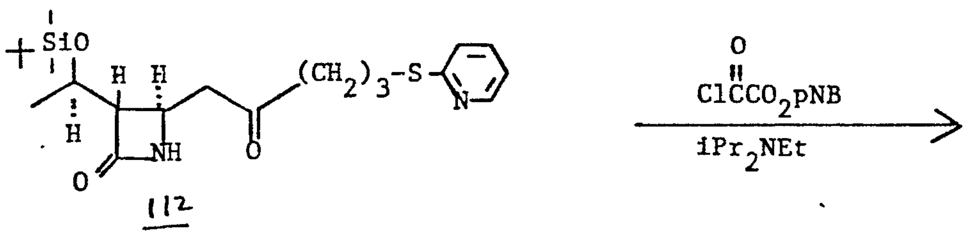


112

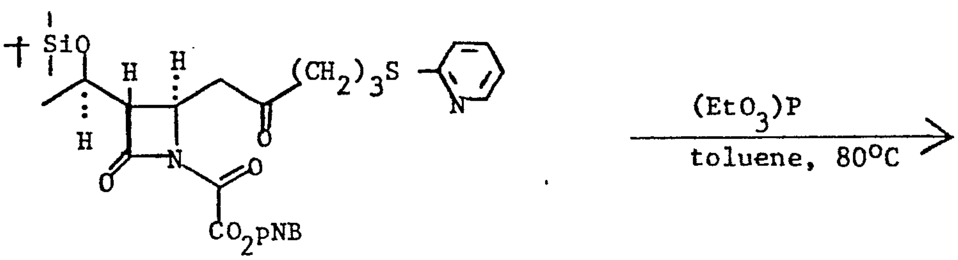


113

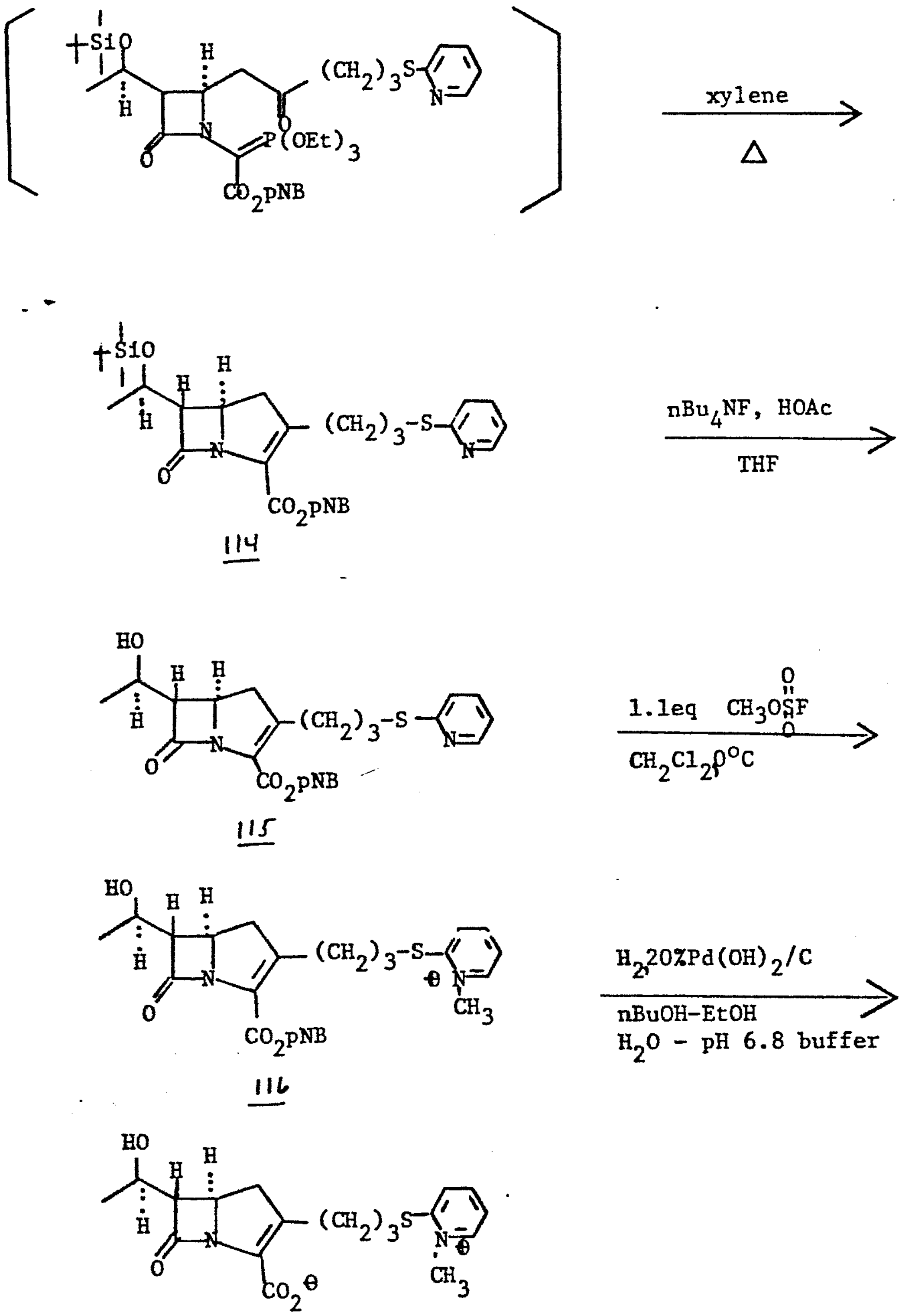
xylene
Δ
nBu4NF, HOAc
THF
1.1eq CH3OSF
CH2Cl2,0°C
H2,20%Pd(OH)2/C
nBuOH-EtOH
H2O - pH 6.8 buffer

114

115

116

Preparation of (5R, 6S, 8R)-2-[3-(2-N-methylpyridiniumthio)propyl]-6-(1-hydroxyethyl)carbapen-2-em-3-carboxylate.

Step A

A solution of grignard reagent 109 in ether (2.0 mmol) is added slowly by syringe to a stirred solution of pyridylthio ester 110 (641 mg, 1.50 mmol) in anhydrous tetrahydrofuran (25 ml) at -78°C under dry nitrogen. After 30 min. at -78°C the reaction is quenched by the rapid addition of saturated aqueous ammonium chloride solution (10 ml). The resulting mixture is poured into ethyl acetate (50 ml) and the phases are separated. The aqueous phase is extracted with an additional portion of ethyl acetate (15 ml) and the combined organic phases are washed with cold 1N hydrochloric acid solution (35 ml), cold 10% aqueous sodium bicarbonate solution (35 ml), and brine. After drying over anhydrous magnesium sulfate, the organic layer is concentrated in vacuo. Chromatography of the resulting residue on silica gel (eluting with ethyl acetate/methylene chloride) gives pure ketone 111.

Step B

Tetrabutyl ammonium fluoride (1.05 ml of 1N solution in tetrahydrofuran, 1.05 mmol) is added by syringe to a solution of ketone 111 (536 mg, 1.0 mmol) in anhydrous tetrahydrofuran (20 ml) at -40°C. The resulting solution is stirred at -40°C for 15 min., then is quenched by pouring into ethyl acetate (50 ml)/0.5M pH 7 phosphate buffer. The layers are separated and the aqueous phase is extracted with

additional portion of ethyl acetate (25 ml). The combined organic layers are washed with water (10 ml) and brine and dried over magnesium sulfate. The dried solution is concentrated in vacuo and the residue is chromatographed on silica gel (eluting with ethyl acetate/hexanes) to provide ketone 112.

Step C

A solution of ketone 112 (350 mg, 0.83 mmol) and p-nitrobenzyloxy oxalyl chloride (609 mg, 2.5 mmol) in dry methylene chloride (15 ml) is cooled to 0°C and triethyl amine (253 mg, 2.5 mmol) is added by syringe. The resulting mixture is stirred at 0°C for 2 hr, then is poured into ethyl acetate (25 ml) and ice cold 0.5N pH 7 phosphate buffer. The layers are separated and the aqeous layer extracted with an additional portion of ethyl acetate (10 ml). The combined organic layers are washed with brine and dried over anhydrous magnesium sulfate. The dried solution is concentrated in vacuo and the residue is rapidly chromatographed in the cold on silica gel (eluting with ethyl acetate/hexanes) to provide oxalimide 113.

Step D

A solution of oxalimide 113 (0.50 mmol) and triethyl phosphite (515 mg, 2.5 mmol) in dry, deoxygenated toluene (10 ml) is heated to 85°C for 3 hr. The reaction is then cooled to room temperature and toluene and excess triethyl phosphite are removed in vacuo. The residue is dissolved in dry, deoxygenated xylenes (10 ml) containing a trace of hydroquinone and the resulting solution is heated at

reflux for 24 hr. The reaction mixture is then cooled to room temperature and the solvent is removed in vacuo. The residue is chromatographed on silica gel (eluting with ethyl acetate/hexanes) to yield carbapenem 114.

Step E

A solution of carbapenem 114 (180 mg, 0.30 mmol), tetrabutyl ammonium fluoride (1.5 ml of 1N solution in tetrahydrofuran, 1.5 mmol), and glacial acetic acid (270 mg, 4.5 mmol) in anhydrous tetrahydrofuran (6 ml) is stirred at room temperature for 32 hr. The reaction mixture is then poured into 0.5M pH 7 phosphate buffer (25 ml), water (25 ml) and ethyl acetate (50 ml). The layers are separated and the aqueous layer is extracted with an additional portion of ethyl acetate (50 ml). The layers are separated and the aqueous layer is extracted with an additional portion of ethyl acetate (25 ml). The combined organic layers are washed with brine and dried over anhydrous magnesium sulfate. The dried solutionis concentrated in vacuo and purified by preparative thin layer chromatography (eluting with ethyl acetate) to provide alcohol 115.

Step F

Methylfluorosulfonate (13 mg, 0.02 mmol) is added via syringe to a solution of carbapenem 115 (97 mg, 0.20 mmol) in dry methylene chloride at 0°C. The resulting solution is stirred at 0°C for 1 hr, then is warmed to room temperature. Solvents are removed in vacuo and the residue containing compound 116 is carried on in crude form to the next reaction.

Step G

Crude compound 116 is dissolved in a mixture of n-butanol, ethanol, water, and pH 6.8 buffer containing 20% palladium hydroxide on charcoal and the resulting mixture is hydrogenated at 40 p.s.i. for 1 hr. The resulting mixture is then filtered to remove the catalyst and the filtrate is washed with ethyl acetate (2x10 ml). The aqueous phase is concentrated in vacuo and the resulting solution is chromatographed on three 500 RPS plates (eluting with ethanol-water) to yield (5R, 6S, 8R)-2-[3-(2-N-methylpyridiniumthio)propyl]-6-(1-hydroxyethyl)carbapen-2-em-3-carboxylate.

WHAT IS CLAIMED IS:

1. A compound having the formula:

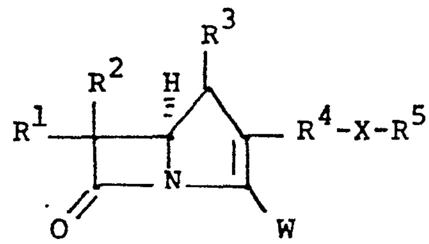

(I.)

wherein:

$R^1$ and $R^2$ are independently H, $CH_3$-, $CH_3CH_2$-, $(CH_3)_2CH$-, $HOCH_2$-, $CH_3CH(OH)$-, $(CH_3)_2C(OH)$-, $FCH_2$-, $F_2CH$-, $F_3C$-, $CH_3CH(F)$-, $(CH_3)_2C(F)$-, or $CH_3CF_2$-;

$R^3$ is H;

$R^4$ is a bridging group comprising substituted or unsubstituted $C_2$-$C_4$ straight, $C_2$-$C_6$ branched or $C_3$-$C_7$ cycloalkyl groups wherein the substituents are selected from $C_1$-$C_6$ alkyl, O-$C_1$-$C_6$ alkyl, S-$C_1$-$C_6$ alkyl, $CF_3$, $N(C_1$-$C_6$ alkyl$)_2$;

X is -S-, -SO-, $-SO_2-$, -O-, or -NH-;

$R^5$ is selected from the group consisting of:

1. Aliphatic

-$C_1$-$C_{10}$ straight or branched alkyl; $C_3$-$C_8$ cycloalkyl; $C_2$-$C_{10}$ straight or branched alkenyl; $C_3$-$C_8$ cycloalkenyl; or $C_2$-$C_{10}$ straight or branched alkynyl;

2. <u>Substituted Aliphatic</u>

$-C_1-C_{10}$ branched or unbranched alkyl; $C_2-C_{10}$ branched or unbranched alkenyl, or akynyl; or $C_3-C_8$ cycloalkyl or cycloalkenyl; wherein all of the above groups are substituted by one or more halo, $OR_a$, $O\overset{O}{\overset{\parallel}{C}}R_a$, $O\overset{O}{\overset{\parallel}{C}}NR_aR_b$, $NHR_a$, $NR_aR_b$, $\overset{O}{\overset{\parallel}{C}}R_a$, $CO_2R_a$, $CONHR_a$, $CONR_aR_b$, CN, $SR_a$, $\underset{O}{S}R_a$, $SO_2R_a$, $SO_2NHR_a$, $SO_2NR_aR_b$, $NH\overset{O}{\overset{\parallel}{C}}R_a$, $NH\overset{O}{\overset{\parallel}{C}}NHR_a$, $NH\overset{O}{\overset{\parallel}{C}}NR_aR_b$, or $NH\overset{O}{\overset{\parallel}{C}}OR_a$, wherein $R_a$ and Rb are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heteroalkyl, heteroaralkyl, heterocyclo and heterocycloalkyl and wherein the heteroatom or atoms in the above-named heterocyclic moieties are selected from the group consisting of 1-4 oxygen, nitrogen or sulfur atoms and wherein the alkyl moieties associated with said heterocyclic moieties have 1-6 carbon atoms;

3. <u>Aryl</u>

-phenyl; or phenyl substituted with 1-3 substituents selected from the group consisting of those substituents set forth above under "Substituted Aliphatic";

4. Heterocyclic

-mono- or bicyclic heterocyclic group containing from 5 to 11 total atoms of which 1 to 5 are heteroatoms selected from nitrogen, oxygen, and sulfur; or substituted heterocyclic where heterocyclic is as defined immediately above and the substituents are 1 to 3 in number and are selected from the group consisting of those substituents set forth above under "Substituted Aliphatic";

5. Arylaliphatic

-the group: -Y-Aryl, where "Aryl" is as defined above, including substituted aryl; and Y is $C_1$-$C_6$ straight or branched alkyl, $C_3$-$C_8$ cycloalkyl, or $C_2$-$C_6$ alkenyl or alkynyl;

6. Heterocycloaliphatic

-the group: -Y-Heterocyclic, where "Heterocyclic" is as defined above, including substituted heterocyclic; and Y is as defined above;

7. Alkyl-Heteroatom-Alkyl

-the group: $-(CH_2)_n-X-(CH_2)_{n'}-R_c$, where n and n' are independently 2-4; X is NR, O, or S where R is H, $CH_3$, $CH_2CH_3$, $CH_2CH_2OH$, or $CH_2CH_2NH_2$; and $R_c$ is OH, $NH_2$, $NHCH_3$, $N(CH_3)_2$, $OC(=O)CH_3$, or $NHC(=O)CH_3$;

provided that the methylene carbons of the group formula may additionally be substituted with $C_1$-$C_3$ alkyl to form branched alkyl groups;

8. <u>Heteroarylium</u>

-the group: 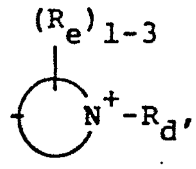, which is a mono- or bicyclic heteroarylium group containing from 5-11 ring atoms of which up to 5 are heteroatoms wherein $R_d$ is:

1) an unsubstituted or substituted $C_1$-$C_6$ alkyl radical;
2) an unsubstituted or substituted $C_2$-$C_6$ alkenyl radical;
3) an unsubstituted or substituted $C_2$-$C_6$ alkynyl radical;
4) a $C_3$-$C_7$ cycloalkyl radical in which the ring is substituted or unsubstituted and one or more atoms may be replaced by a heteroatom;
5) a $C_3$-$C_7$ cycloalkyl methyl radical in which the ring may be substituted and one or more atoms may be replaced by a heteroatom;
6) an unsubstituted or substituted $C_5$-$C_7$ cycloalkenyl radical;
7) an unsubstituted or substituted bivalent $C_2$-$C_6$ alkylidene radical, optionally interrupted by a heteroatom, and joined to the heteroarylium group to form a ring which is carbocyclic or in which one or more atoms is replaced by a heteroatom. The new ring may contain one or more double bonds;

8) an unsubstituted or substituted phenyl or heteroaryl radical;
9) an unsubstituted or substituted phenyl ($C_1$-$C_4$ alkyl) or heteroaryl ($C_1$-$C_4$ alkyl) radical;
10) a cyano ($C_1$-$C_4$ alkyl) radical;
11) a carboxy ($C_1$-$C_4$ alkyl) radical;
12) a sulfo ($C_1$-$C_4$ alkyl) radical;
13) a carbamoyl ($C_1$-$C_4$ alkyl) radical;
14) a phosphonyl ($C_1$-$C_4$ alkyl) radical;
15) a hydroxy ($C_1$-$C_4$ alkyl) radical;
16) an amino ($C_1$-$C_4$ alkyl) radical in which the nitrogen atom is unsubstituted or substituted with one to three $C_1$-$C_4$ alkyl groups;

wherein the substituents in the above definitions of $R_d$ are independently selected from the group consisting of the definitions of $R_e$ set out below;

$R_e$ is independently selected from:
a) a trifluoromethyl group;
b) a halogen atom;
c) an unsubstituted or substituted $C_1$-$C_4$ alkoxyl radical;
d) a hydroxy group;
e) an unsubstituted or substituted ($C_1$-$C_6$ alkyl) carbonyloxy radical;
f) a carbamoyloxy radical which is unsubstituted or substituted on nitrogen with one or two $C_1$-$C_4$ alkyl groups;

g) a $C_1$-$C_6$ alkylthio radical, $C_1$-$C_6$ alkylsulfinyl radical or $C_1$-$C_6$ alkylsulfonyl radical, each of which is unsubstituted or substituted on the alkyl group;

h) a sulfo group;

i) a sulfamoyl group which is unsubstituted or substituted on nitrogen by one or two $C_1$-$C_4$ alkyl groups;

j) an amino group;

k) a mono ($C_1$-$C_4$ alkyl) amino or di($C_1$-$C_4$ alkyl)amino group, each of which is unsubstituted or substituted on the alkyl group;

l) a formylamino group;

m) an unsubstituted or substituted ($C_1$-$C_6$ alkyl)carbonylamino radical;

n) a ($C_1$-$C_4$ alkoxyl) carbonylamino radical;

o) a ureido group in which the terminal nitrogen is unsubstituted or substituted with one or two $C_1$-$C_4$ alkyl groups;

p) an arylsulfonamido or a ($C_1$-$C_6$ alkyl)sulfonamido group;

q) a cyano group;

r) a formyl or acetalized formyl radical;

s) an unsubstituted or substituted ($C_1$-$C_6$ alkyl)carbonyl radical wherein the carbonyl is free or acetalized;

t) an unsubstituted or substituted phenylcarbonyl or heteroarylcarbonyl radical;

u) a hydroximinomethyl radical in which the oxygen or carbon atom is optionally substituted by a $C_1$-$C_4$ alkyl group;

v) a carboxyl group;

w) a ($C_1$-$C_6$ alkoxy)carbonyl radical;

x) a carbamoyl radical which is unsubstituted or substituted on nitrogen by one or two $C_1$-$C_4$ alkyl groups;

y) an N-hydroxycarbamoyl or N($C_1$-$C_4$ alkoxy)carbamoyl radical in which the nitrogen atom may be additionally substituted by a $C_1$-$C_4$ alkyl group;

z) a thiocarbamoyl group;

aa) a 5-(1H)-tetrazolyl group;

ab) an amidino group 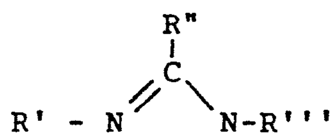

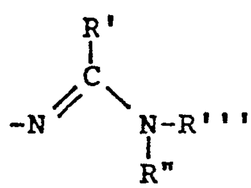

where R', R" and R''' are independently hydrogen, $C_1$-$C_4$alkyl or wherein two of the alkyl groups together form a $C_2$-$C_6$alkylidene radical optionally interrupted by a heteroatom and joined together to form a ring;

ac) a carboxamidino group 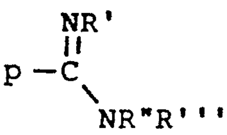, where R', R" and R''' are as defined above;

ad) a guanidinyl group where R" in ab) above is $NR^{iv}R^{v}$ and $R^{iv}$ and $R^{v}$ are as defined for R' through R''' above;

ae) a phosphonate group $-P(O)(O^{-})OR^{vi}$ where $R^{vi}$ is $C_1$-$C_3$alkyl;

af) an alkyl phosphonate group $-(CH_2)_nP(O)(O^{-})(OR^{vi})$ where n = 1 to 3 and $R^{vi}$ is $C_1$-$C_3$alkyl;

ag) hydrogen;

ah) an unsubstituted or substituted $C_1$-$C_6$ alkyl radical;

ai) an unsubstituted or substituted $C_2$-$C_6$ alkenyl radical;

aj) an unsubstituted or substituted $C_2$-$C_6$ alkynyl radical;

ak) a $C_3$-$C_7$ cycloalkyl radical in which the ring is substituted or unsubstituted and one or more atoms may be replaced by a heteroatom;

al) a $C_3$-$C_7$ cycloalkyl methyl radical in which the ring may be substituted and one or more atoms may be replaced by a heteroatom;

am) an unsubstituted or substituted $C_5$-$C_7$ cycloalkenyl radical;

an) an unsubstituted or substituted phenyl or heteroaryl radical;

ao) an unsubstituted or substituted phenyl ($C_1$-$C_4$ alkyl) or heteroaryl ($C_1$-$C_4$ alkyl) radical; and

9. Heteroaryliumaliphatic

-the group: -Y-Heteroarylium, where Y is as defined above; and "Heteroarylium" is as defined above and additionally includes the group:

$(R_e)_{1-3}$

$-\overset{+}{N}$ , which is a mono- or bicyclic heteroarylium group containing from 5-11 ring atoms of which up to 5 are heteroatoms, where $R_e$ is as defined above;

10. Amidino and Amidinium

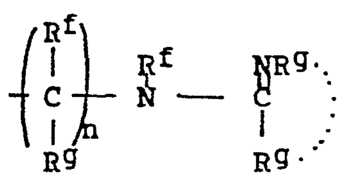

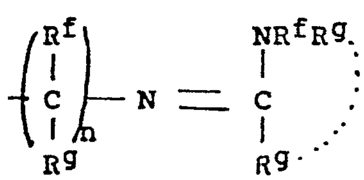

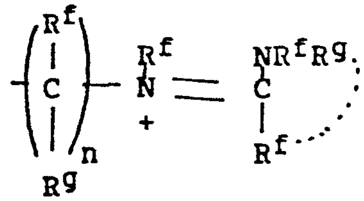

0186057

and wherein n = 2-6; $R^f$ and $R^g$ are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heterocyclic groups as defined above, including heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms; and the dotted line indicates provision for the ring formed by the joinder of substituents carried by the imino carbon atoms;

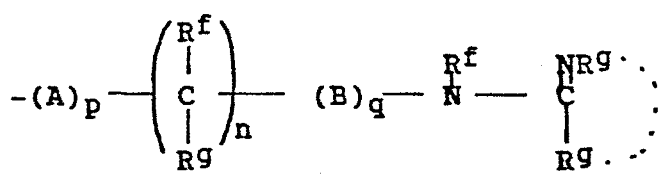

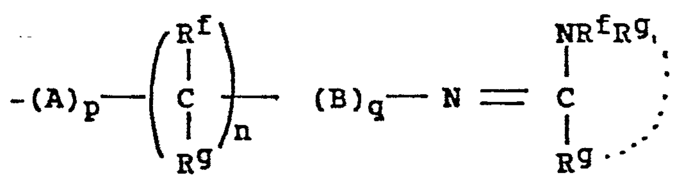

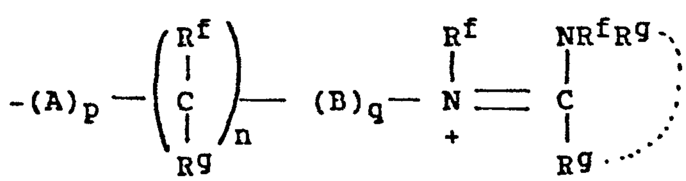

wherein $R^f$, $R^g$ and n are as defined immediately above; p and q are 0 or 1; A and B are in bivalent form and are selected from the following definitions above of $R^5$: cycloalkyl, alkenyl, cycloalkenyl, alkynyl; substituted: cycloalkyl, alkenyl, cycloalkenyl, alkynyl; phenyl and substituted phenyl; substituted and unsubstituted heteroaryl; arylaliphatic; heteroarylaliphatic, heterocyclyloaliphatic, and heterocyclic; and alkyl-heteroatom-alkyl; and B can also be selected from -O- and $-NR^f-$;

11. Guanidino and Guanidinium

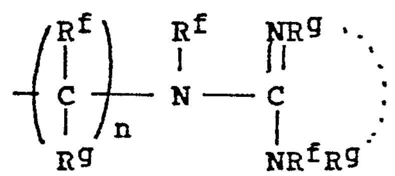

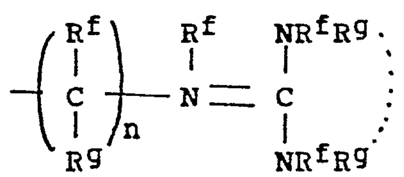

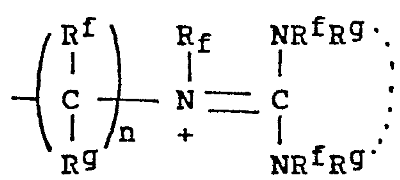

and wherein n = 2-6; $R^f$ and $R^g$ are as initially defined above under "Amidino and Amidinium"; and the dotted line indicates provision for the ring formed by the joinder of substituents carried by the imino carbon atoms;

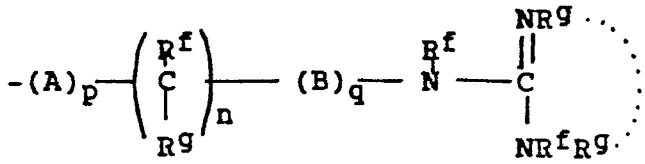

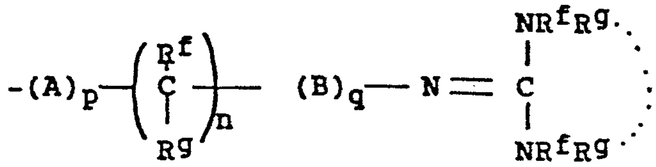

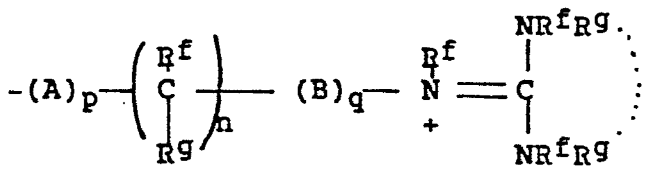

wherein $R^f$ and $R^g$ and n are as defined immediately above; p and q are 0 or 1; A and B are as defined above under "Amidino and Amidinium";

12. <u>Carbamimidoyl and Carbamimidinium</u>

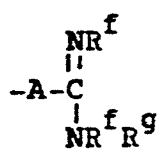 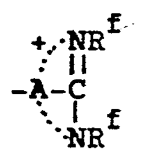 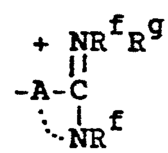

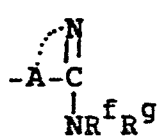 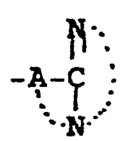 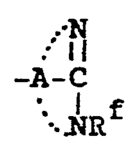

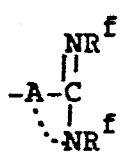 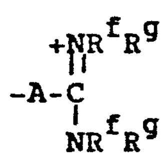 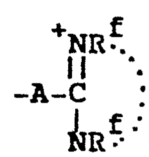

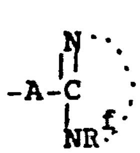 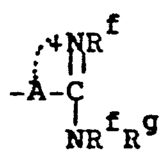 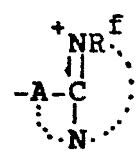

and wherein $R^f$ and $R^g$ are as defined above under "Amidino and Amidinium"; the two nitrogen atoms demonstrated in the above structure may participate in cyclic structures which are indicated by the dotted lines; and A is a connecting group between the sulfur atom and carbamimidoyl function which is bivalent, cyclic or acyclic, is to be read both left to right and right to left, and is selected from: substituted and unsubstituted: loweralkyl having from 1-6 carbon atoms; cycloalkyl having from 3-10 atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; loweralkenyl having from 2-10 carbon atoms, cycloalkenyl having from 3 to 10 carbon atoms, cycloalkenylalkyl wherein the cycloalkenyl moiety comprises 3 to 10 carbon atoms, and the alkyl moiety comprises 1 to 6 carbon atoms; alkynyl having from 2 to 10 carbon atoms; aryl, which is defined as phenyl and naphthyl; arylalkyl and alkylaryl; heteroalkyl, alkylheteroalkyl, arylheteroalkyl and alkylheteroaryl wherein the heteroatoms are selected from the group of sulfur, oxygen and nitrogen, and the alkyl moiety has 1 to 6 carbon atoms, and the aryl moiety is phenyl; heterocyclyo comprising mono- and bicyclic structures having 5 to 10 ring atoms wherein one or more of the heteroatoms is selected from oxygen, nitrogen, or sulphur; heterocycloalkyl wherein the heterocyclyo moiety comprises from 3 to 10 atoms and the alkyl moiety comprises from 1 to 6 atoms; the substituent (or substituents) relative to the

above-named radicals are selected from the group consisting of amino, hydroxyl, cyano, carboxyl, nitro, chloro, bromo, fluoro, alkoxy having from 1 to 6 carbon atoms, mercapto, haloloweralkyl, and alkylthio having from 1-6 carbon atoms;

W is selected from:

i) COOH or a pharmaceutically acceptable ester or salt thereof,

ii) COOR wherein R is a removable carboxy protecting group,

iii) COOM wherein M is an alkali metal, or

iv) $COO^-$; provided that when W is other than iv) a counterion $Z^-$ is provided.

2. A compound of Claim 1 having the configuration and formula:

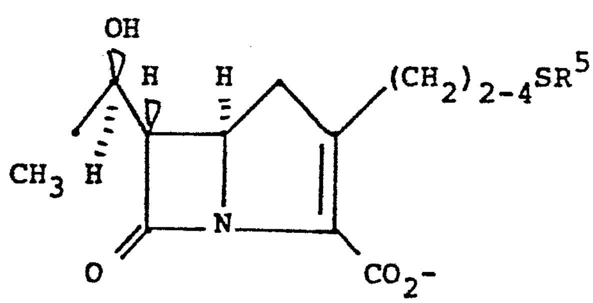

**3.** A compound of Claim 2 wherein said $R^5$ is

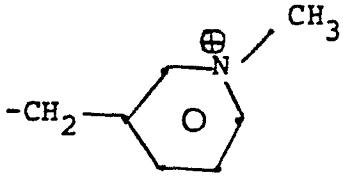 ,

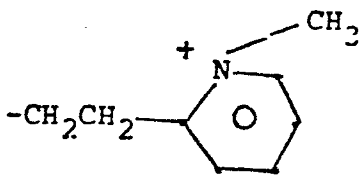 ,

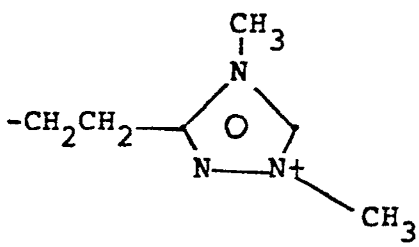 ,

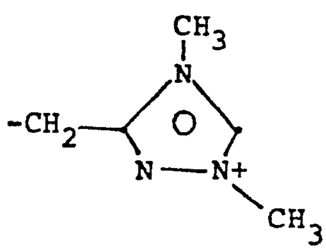 ,

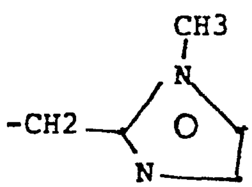 ,

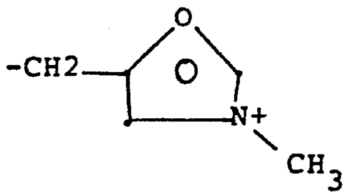
,

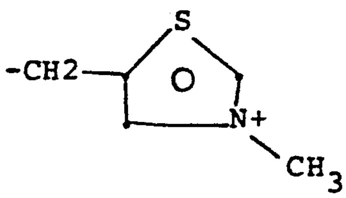
,

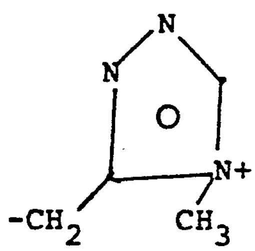
,

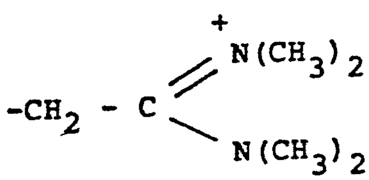
,

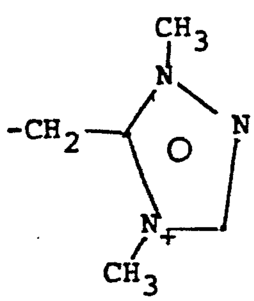
,

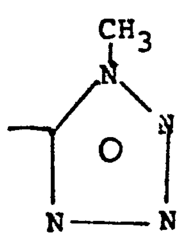

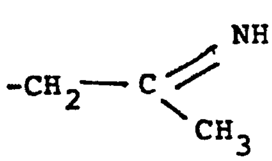
.

4. A compound of Claim 2 wherein the compound is a member selected from the group consisting of:

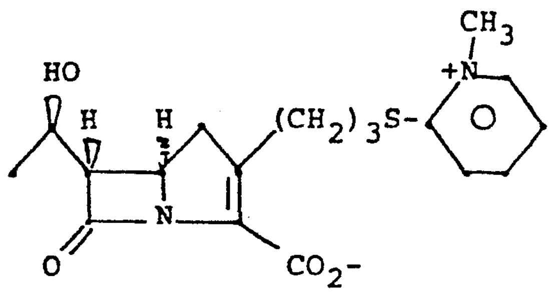

,

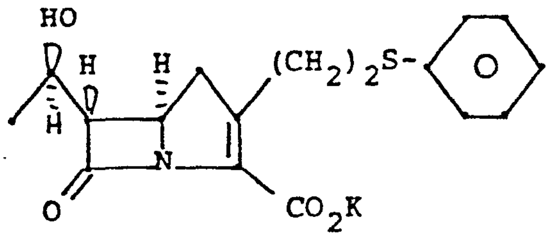

, or

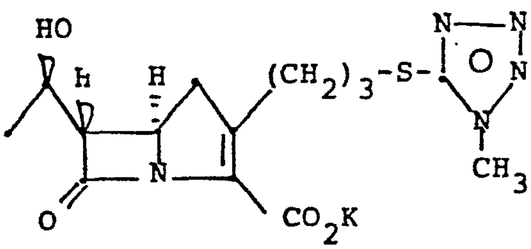

.

5. The combination of a compound of Claim 1 and a DHP inhibitor.

6. A combination of Claim 5 wherein the DHP inhibitor is 7-(L-2-amino-2-carboxyethylthio)-2-(2,2-dimethylcyclopropanecarboxamido)-2-heptenoic acid.

7. A pharmaceutical composition for antibiotic use comprising an antibacterially effective amount of a compound of Claim 1, an inhibitorily effective amount of a DHP inhibitor, and optionally, a pharmaceutically acceptable carrier.

8. A pharmaceutical composition for antibiotic use comprising an antibacterially effective amount of a compound of Claim 1, and, optionally, a pharmaceutically acceptable carrier.

European Patent Office

# EUROPEAN SEARCH REPORT

0186057

Application number

EP 85 11 5847

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| X | CHEMICAL ABSTRACTS, vol. 101, no. 5, 30th July 1984, page 492, no. 38274j, Columbus, Ohio, US; & JP - A - 59 36 677 (SHIONOGI AND CO., LTD.) 28-02-1984 * Abstract * | 1,7 | C 07 D 487/04<br>A 61 K 31/40 //<br>C 07 F 9/65<br>C 07 F 7/18<br>(C 07 D 487/04<br>C 07 D 209:00<br>C 07 D 205:00 ) |
| A | EP-A-0 112 283 (CIBA-GEIGY) * Claims 1,18 * | 1,7 | |
| | | | **TECHNICAL FIELDS SEARCHED (Int. Cl.4)** |
| | | | C 07 D 487/00<br>C 07 D 499/00<br>C 07 D 519/00<br>A 61 K 31/00 |

The present search report has been drawn up for all claims

| Place of search | Date of completion of the search | Examiner |
|---|---|---|
| THE HAGUE | 18-03-1986 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82